(19) Europäisches Patentamt
European Patent Office
Office européen des brevets

(11) **EP 2 683 440 B1**

(12) **EUROPÄISCHE PATENTSCHRIFT**

(45) Veröffentlichungstag und Bekanntmachung des Hinweises auf die Patenterteilung:
**04.11.2015 Patentblatt 2015/45**

(51) Int Cl.:
*A61N 2/00* (2006.01)     *A61K 41/00* (2006.01)

(21) Anmeldenummer: **12708249.3**

(22) Anmeldetag: **08.03.2012**

(86) Internationale Anmeldenummer:
**PCT/EP2012/001034**

(87) Internationale Veröffentlichungsnummer:
**WO 2012/119775 (13.09.2012 Gazette 2012/37)**

(54) **COMPUTERGESTÜTZTES SIMULATIONSWERKZEUG ZUR UNTERSTÜTZUNG BEI DER PLANUNG EINER THERMOTHERAPIE**

COMPUTER-AIDED SIMULATION TOOL FOR PROVIDING ASSISTANCE IN THE PLANNING OF THERMOTHERAPY

OUTIL DE SIMULATION ASSISTÉ PAR ORDINATEUR DESTINÉ À AIDER UN UTILISATEUR LORSQU'IL PLANIFIE UNE THERMOTHÉRAPIE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **10.03.2011 EP 11001993**

(43) Veröffentlichungstag der Anmeldung:
**15.01.2014 Patentblatt 2014/03**

(73) Patentinhaber: **MagForce AG**
**12489 Berlin (DE)**

(72) Erfinder:
• **NADOBNY, Jacek**
**12623 Berlin (DE)**
• **LIU, Peng**
**13583 Berlin (DE)**
• **OLLEK, Jens-Thorsten**
**13407 Berlin (DE)**
• **BENDER, Heike, C.**
**84405 Dorfen (DE)**

(74) Vertreter: **Lahrtz, Fritz**
**Isenbruck Bösl Hörschler LLP**
**Patentanwälte**
**Prinzregentenstrasse 68**
**81675 München (DE)**

(56) Entgegenhaltungen:
**WO-A1-2007/113572     WO-A2-2006/102307**

## Beschreibung

Die Erfindung betrifft ein computergestütztes Simulationswerkzeug zur Unterstützung bei der Planung einer Thermotherapie, und genauer gesagt ein computergestütztes Simulationsverfahren zur Unterstützung einer Thermotherapie-Planung sowie entsprechend ausgebildete Computereinrichtungen.

STAND DER TECHNIK

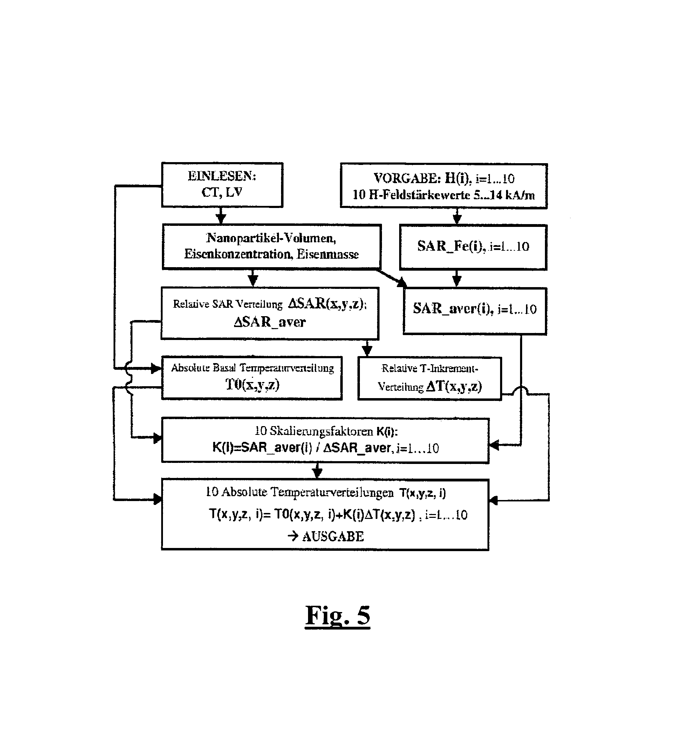

Gneveckow et al., "Description and characterization of the novel hyperthermia- and thermoablation-system MFH® 300F for clinical magnetic fluid hyperthermia", Med. Phys. 31 (6), June 2004, 1444 ff. beschreiben unter anderem die messtechnische Bestimmung von Kennlinien, welche den Zusammenhang zwischen den Werten der magnetischen Feldstärke ("H-Feldstärkewerten" in Kiloampere pro Metern, kA/m) und der auf die Eisenmasse bezogenen Leistungsabsorptionsraten $SAR_{fe}$ ("Specific Absorption Rate of Iron", in Watt pro Gramm, W/g) für bestimmte Magnetflüssigkeiten angeben. Weitere Aspekte einer Hyperthermie-Behandlung basierend auf Magnetflüssigkeiten werden beschrieben (Abstract; S. 1445-1446, Abschnitt "II. Methods"; S. 1447, Abschnitt "IIIB. Power absorption" iVm. Fig. 5; S. 1448, Abschnitt "IIID. Thermal distribution in the quasi-steady state"; S. 1449-1450, Abschnitt "IV. Discussion").

Wust et al., "Magnetic nanoparticles for interstitial thermotherapy - feasibility, tolerance and achieved temperatures", Int. J. Hyperthermia, Dezember 2006, 22(8), 673-685, beschreiben ein Konzept für eine Hyperthermie-Behandlung basierend auf Magnetflüssigkeiten. Die Magnetflüssigkeit enthält Eisenoxid-Nanopartikel, dispergiert in Wasser. Die Magnetflüssigkeit muss im Tumor verteilt werden und wird nachfolgend durch Applikation eines magnetischen Wechselfeldes über einen Applikator aufgeheizt. Resultierende Temperaturverteilungen werden analysiert. Resultierende Werte der Spezifischen Absorptionsrate im Gewebe (SAR) werden aus der mittels Computertomographie (CT) bestimmten Partikelverteilung in Zusammenhang mit konkreten H-Feldstärkewerten in kA/m ermittelt. Die Temperaturverteilung im Tumorbereich wird unter Verwendung einer Bioheat-Transfer-Gleichung (nachfolgend BHTE für "Bio-Heat Transfer-Equation") numerisch berechnet. Eine Anpassung der berechneten Temperaturverteilung an direkt gemessene Temperaturwerte an Referenzpunkten in oder nahe bei dem Zielbereich wird mittels einer geeignet gewählten, mittleren Perfusionsrate vorgenommen. (Abstract; S. 675, Abschnitt "Magnetic fluid"; S. 677, Abschnitt "Post-implantation analysis (PIA) and Thermotherapy").

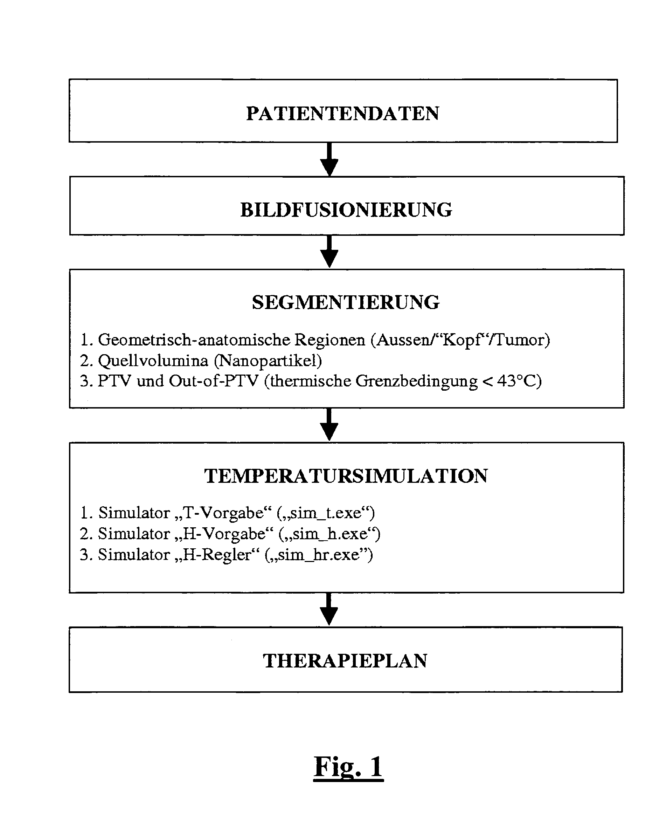

Maier-Hauff et al., "Efficacy and safety of intratumoral thermotherapy using magnetic iron-oxide nanoparticles combined with external beam radiotherapy on patients with recurrent glioblastoma multiforme", J. Neurooncol. DOI 10/2007/s11060-010-0389-0, veröffentlicht online am 16. September 2010, beschreiben intratumorale Thermotherapieverfahren unter Verwendung magnetischer Nanopartikel. Eine Dichte der Nanopartikel nach dem Einbringen wird mittels CT-Verfahren ermittelt. Auf Basis der Dichteverteilung der Nanopartikel, ihrer SAR und einer abgeschätzten Perfusionsrate innerhalb des Tumorgebietes wird die Wärmeerzeugung innerhalb des Zielgewebes als Funktion einer magnetischen Feldstärke unter Verwendung einer BHTE bestimmt. Die magnetische Feldstärke (nachfolgend auch als "H-Feldstärke" oder nur "Feldstärke" bezeichnet) sollte so gewählt werden, dass eine Temperatur von 43°C außerhalb eines Saumes von 2 cm um den Tumor herum nicht überschritten wird. Während der Behandlungen wurden unmittelbare Temperaturmessungen in einem vorab platzierten Thermometrie-Katheter vorgenommen. Hierdurch wird die Einhaltung der Grenztemperatur überwacht (Abstract; S. 3, Fig. 1 und linke Spalte und rechte Spalte, 1. Absatz).

Nadobny et al., "Evaluation of MR-Induced Hot Spots for Different Temporal SAR Modes Using a Time-Dependent Finite Difference Method With Explicit Temperature Gradient Treatment", IEEE Transactions on Biomedical Engineering, Vol. 54, No. 10, Oktober 2007, S. 1837 ff. beschreibt die numerische Lösung einer nichtlinearen BHTE im Zeitbereich, die eine temperaturabhängige Perfusion enthält, und auf deren Basis numerische Simulationen zur Temperaturverteilung im menschlichen Körper durchgeführt werden (Abstract; S. 1837, Abschnitt "I. Introduction"; S. 1838, Abschnitt "II. Time-Dependent BHTE"; S. 1840-1841, Abschnitt IIIC: "Thermal Simulation Procedures"; S. 1845-1846, Abschnitte "V. Discussion", "VI. Conclusion"). Unter anderem wurde in Nadobny et al. ein Verfahren (sog. "decomposition way") beschrieben, wonach zur numerischen Lösung der BHTE mit Hilfe der Finite-Differenzen (FD)-Methode eine Aufteilung der Temperaturverteilung in einen basalen und einen SAR-abhängigen Teil erfolgt (S. 1841, linke Spalte, Gleichungen 5a, 5b und 6).

ALLGEMEINE DARSTELLUNG DER ERFINDUNG

Eine Aufgabe der vorliegenden Erfindung besteht darin, ein Werkzeug für die Vorbereitung einer Thermotherapie vorzuschlagen, das einem Arzt oder sonstigem medizinischen Personal einen umfassenden Überblick über unterschiedliche Therapieoptionen bereitstellt, um eine Entscheidung für eine bestimmte Option zu erleichtern.

Diese Aufgabe wird durch ein erfindungsgemäßes computergestütztes Simulationswerkzeug zur Unterstützung bei der Planung einer Thermotherapie, und genauer gesagt ein erfindungsgemäßes computergestütztes Simulationsverfahren zur Unterstützung einer Thermotherapie-Planung sowie entsprechend ausgebildete Computereinrichtungen gelöst.

Erfindungsgemäß wird ein computergestütztes Simulationsverfahren zur Unterstützung einer Thermotherapie-Planung vorgeschlagen. Die Thermotherapie umfasst eine (lokale/ regionale) Hyperthermie-Behandlung eines Tumorvolumens in einem Körpervolumen eines menschlichen Körpers. Die Hyperthermie-Behandlung umfasst das Applizieren eines Magnetfeldes in einem Behandlungsvolumen mittels eines Magnetfeld-Applikators. Dabei ist - mittels zuvor im Körper deponierter magnetischer, paramagnetischer und/oder superparamagnetischer Nanopartikel - durch Leistungsabsorption im applizierten Magnetfeld Wärmeenergie in mindestens ein "Depotvolumen", d.h. in ein Volumen, welches Nanopartikel beinhaltet, einbringbar.

Entsprechend wird unter Hyperthermie-Behandlung im Rahmen der vorliegenden Erfindung allgemein eine Therapie mittels erhöhter Temperatur bezeichnet. Bei geringeren Temperaturerhöhungen bis ca. 45 °C führt dies zur Steigerung der Wirksamkeit von Chemotherapie und/oder Radiotherapie, der Hyperthermie im engeres Sinn (Hildebrandt, B., et al. (2002). "The cellular and molecular basis of hyperthermia." Critical Reviews in Oncology Hematology 43(1): 33-56). Bei Temperaturerhöhungen auf mehr als 45 °C erfolgt das direkte Absterben von (Tumor-)Zellen, der sogenannten Thermoablation (Jordan, A., et al. (2006). "The effect of thermotherapy using magnetic nanoparticles on rat malignant glioma." J Neurooncol. 78(1): 7-14. Epub 2005 Nov 29).

Anzustreben ist, dass sich das Depotvolumen oder die Depotvolumina ganz oder zumindest teilweise innerhalb des Behandlungsvolumens, vorzugsweise des Tumorvolumens, befinden. Dies sollte durch die vorausgegangene Instillation der Nanopartikel sichergestellt worden sein. Das Tumorvolumen oder die Tumorvolumina sollten ganz oder teilweise im Behandlungsvolumen liegen, je nach Therapieziel. Damit ist das Behandlungsvolumen das Volumen, das durch die Hyperthermie primär erwärmt werden soll. Ob und inwieweit dieses Ziel voraussichtlich erreicht werden kann, wird durch das erfindungsgemäße Simulationsverfahren vorhergesagt.

Das erfindungsgemäße Verfahren wird als ein Simulationsverfahren zur Unterstützung der Planung einer Thermotherapie angesehen, weil es die bei einer Behandlung mit dem Magnetfeld-Applikator auftretenden Temperaturverteilungen im Körper simuliert, ohne dass tatsächlich eine derartige Behandlung vorgenommen wird. Das Verfahren benötigt also bspw. keine Eichung durch Messwerte, die bei einer momentan ablaufenden Behandlung gemessen werden. Aus den Ergebnissen, die vom erfindungsgemäßen Simulationswerkzeug geliefert werden, können Rückschlüsse auf Behandlungsparameter einer nachfolgenden tatsächlichen Behandlung gezogen werden, z.B. in Bezug auf den zu wählenden Feldstärkewert bzw. H-Feldstärkewert.

Der Anwender des erfindungsgemäßen Verfahrens bzw. Simulationswerkzeugs kann ein Arzt sein, z.B. ein Radiologe oder ein Strahlentherapeut, zur Bedienung eines Magnetfeld-Applikators ausgebildetes Personal mit technischer und/oder medizinischer Ausbildung, oder sonstige Benutzer bzw. Anwender.

Bei den Nanopartikeln kann es sich generell um Teilchen handeln, die auf irgendeine Weise mit einem Magnetfeld derart wechselwirken, dass es (nach Instillation der Teilchen in den Körper) zu einer Gewebeerwärmung kommt. Es kann sich etwa um magnetische, paramagnetische oder superparamagnetische Nanopartikel handeln, z.B. um Eisenoxid-Nanopartikel mit oder ohne Beschichtung. Die Teilchen können beliebige Raumformen haben, und können bspw. zumindest im Wesentlichen kugelförmig, sphäroidal, ellipsoidal, prismen- oder quaderförmig geformt sein. Obwohl die Teilchen hier als 'Nanopartikel' bezeichnet werden, sollen unter diesen Begriff neben Teilchen mit Abmessungen (z.B. kleinster oder größter Radius oder Durchmesser) im Nanometerbereich auch Teilchen mit anderen Abmessungen fallen, bspw. Teilchen mit Abmessungen im Mikrometerbereich. Bevorzugt sind Eisenoxid-Nanopartikel mit einem Durchmesser (bestimmt im Elektronenmikroskop) von bis zu 100 nm.

Das erfindungsgemäße Simulationsverfahren umfasst die folgenden Schritte: In einem ersten Berechnungsschritt, auch bezeichnet als "T-Vorgabe" ("T" steht für Temperatur), Berechnen eines am Applikator einzustellenden Feldstärkewertes bzw. H-Feldstärkewertes basierend auf einer geometrischen Verteilung der Nanopartikel und mindestens einem vorgegebenen Temperaturgrenzwert, der durch die Hyperthermie-Behandlung nicht überschritten werden soll; in einem optionalen zweiten Berechnungsschritt, auch bezeichnet je nach Ausführungsform als "H-Regler" ("H" steht für magnetische Feldstärke) oder "schneller H-Regler", Berechnen einer zu erwartenden (resultierenden) Temperaturverteilung für mindestens einen Teil des Körpervolumens für jeden H-Feldstärkewert einer Mehrzahl vorgegebener H-Feldstärkewerte, und/oder einen anwenderdefinierten H-Feldstärkewertvorzugsweise in einem optionalen dritten Berechnungsschritt ("H-Vorgabe") automatisches Berechnen, einer zu erwartenden Temperaturverteilung für einen maximal zulässigen H-Feldstärkewert, wenn der im ersten Berechnungsschritt berechnete H-Feldstärkewert größer ist als dieser maximal zulässige H-Feldstärkewert; und Bereitstellen (dem Anwender) des am Applikator einzustellenden berechneten H-Feldstärkewertes und optional mindestens einer berechneten resultierenden Temperaturverteilung, z.B. derjenigen zum vorgenannten H-Feldstärkewert zugehörigen, zur Unterstützung des Anwenders bei der Planung der Thermotherapie.

Bei bestimmten Varianten des erfindungsgemäßen Simulationsverfahrens betrifft der vorgegebene Temperaturgrenzwert oder einer von mehreren vorgegebenen Temperaturgrenzwerten eine maximale Temperatur bzw. ein Temperaturmaximum nur innerhalb des zu erhitzenden Behandlungsvolumens. Der vorgegebene Temperaturgrenzwert kann etwa ein Temperaturmaximum in einem Bereich von 60°C bis 100°C, bevorzugt 70°C bis 90°C, insbesondere 80°C, im Behandlungsvolumen betreffen.

Zusätzlich oder alternativ kann der vorgegebene Temperaturgrenzwert oder einer von mehreren vorgegebenen Temperaturgrenzwerten ein Temperaturmaximum außerhalb des zu erhitzenden Behandlungsvolumens betreffen. Zum Beispiel kann der vorgegebene Temperaturgrenzwert ein Temperaturmaximum in einem Bereich von 40°C bis 45°C, insbesondere 43°C, außerhalb des Behandlungsvolumens betreffen.

Bei einigen Ausführungsformen des erfindungsgemäßen Simulationsverfahrens gehen in den ersten Berechnungsschritt zwei vorgegebene Temperaturgrenzwerte ein, die jeweils unterschiedliche Volumina betreffen. Beispielsweise kann ein vorgegebener Temperaturgrenzwert innerhalb des Behandlungsvolumens ein Temperaturmaximum von 80°C betreffen und ein anderer vorgegebener Temperaturgrenzwert außerhalb des Behandlungsvolumens ein Temperaturmaximum von 43°C betreffen. Die zwei vorgegebenen Temperaturgrenzwerte können dabei vorzugsweise gleichzeitig in den ersten Berechnungsschritt eingehen.

Ein dritter Berechnungsschritt ("H-Vorgabe") kann umfassen, dass für einen als maximal zulässig definierten H-Feldstärkewert eine zu erwartende Temperaturverteilung berechnet wird. Der als maximal zulässig definierte H-Feldstärkewert kann beispielsweise einen maximal einstellbaren H-Feldstärkewert am Applikator, oder einen als maximal zulässig definierten H-Feldstärkewert pro Patient betreffen. Der dritte Berechnungsschritt kann abhängig vom Berechnungsergebnis des ersten Berechnungsschrittes (T-Vorgabe) automatisch dann durchgeführt werden, wenn der im ersten Berechnungsschritt berechnete H-Feldstärkewert größer ist als der als maximal zulässig definierte H-Feldstärkewert.

Bei bestimmten Varianten des erfindungsgemäßen Simulationsverfahrens geht in die Berechnungen im zweiten Berechnungsschritt (H-Regler; schneller H-Regler) und/oder im dritten Berechnungsschritt (H-Vorgabe) kein Temperaturgrenzwert ein. Die ggf. resultierenden simulierten Temperaturverteilungen können daher bei diesen Berechnungen den oder die Grenzwerte, die im ersten Berechnungsschritt beachtet wurden, übertreffen. Es können also bspw. außerhalb des Behandlungsvolumens Temperaturen höher als 43°C und/oder innerhalb des Behandlungsvolumens Temperaturen höher als 80°C auftreten. Dies ermöglicht dem Arzt eine umfassendere Übersicht über die Auswirkungen der geplanten Wärmetherapie als wenn er nur strikt Temperaturgrenzwert-basierten Ergebnisse der Berechnung aus dem ersten Berechnungsschritt zur Verfügung hätte.

Die Berechnungen im zweiten Berechnungsschritt können für eine Mehrzahl vorgegebener, am Applikator einstellbarer H-Feldstärkewerte, bevorzugt zwischen 3 und 20 H-Feldstärkewerten, besonders bevorzugt zwischen 5 und 10 H-Feldstärkewerten, durchgeführt werden. Zusätzlich oder alternativ können die Berechnungen im zweiten Berechnungsschritt für eine Mehrzahl vorgegebener, am Applikator einstellbarer H-Feldstärkewerte, bevorzugt zwischen 3 kA/m und 20 kA/m, besonders bevorzugt zwischen 5 kA/m und 10 kA/m, durchgeführt werden (H-Regler).

Der zweite Berechnungsschritt (H-Regler; schneller H-Regler) kann nach dem ersten Berechnungsschritt (T-Vorgabe) und ggf. dritten Berechnungsschritt (H-Vorgabe) erst durch eine Anwendereingabe ausgelöst werden. Benötigt der Anwender nach der Ausgabe der initialen resultierenden Temperaturverteilung mit dazugehörigem initialen H-Feldstärkewert (als "initial" wird hier das Ergebnis des ersten oder ggf. des dritten Berechnungsschrittes gemeint) keine weitere Orientierung mehr, kann der Anwender auf eine Anwendereingabe verzichten und es können auf diese Weise Ressourcen der Computereinrichtung, auf der das erfindungsgemäße Simulationsverfahren implementiert ist, gespart oder anderweitig verwendet werden.

Varianten des erfindungsgemäßen Simulationsverfahrens führen insbesondere bei der Berechnung des einzustellenden H-Feldstärkewertes im ersten Berechnungsschritt (T-Vorgabe) keine zahlreichen Iterationen nach dem Prinzip "Trial and Error" aus, bei denen aus gewählten Feldstärkewerten resultierende Temperaturverteilungen über numerische Lösung der BHTE berechnet werden, um so iterativ zu dem gesuchten H-Feldstärkewert zu gelangen. Derartige Iterationen, bei denen jedes Mal eine BHTE numerisch gelöst wird, benötigen sehr viele Ressourcen in Bezug auf Rechenleistung, -zeit und Speicherbedarf und sind daher für ein Simulationswerkzeug nicht geeignet, das einem Anwender einen Überblick über geplante Therapieoptionen vermitteln soll. Stattdessen wird im ersten Berechnungsschritt, wie weiter unten ausgeführt, die BHTE lediglich exakt zwei Mal numerisch gelöst.

Bei einer Ausführungsform des erfindungsgemäßen Simulationsverfahrens wird der H-Feldstärkewert im ersten Berechnungsschritt (T-Vorgabe) basierend auf einer vorgegebenen, zum Beispiel aus einer Referenzmessung abgeleiteten Kennlinie berechnet, die einen Zusammenhang zwischen (Referenz-) Leistungsabsorptionsrate und H-Feldstärke angibt.

Bei einer spezifischen Variante dieser Ausführungsform weist der erste Berechnungsschritt die folgenden Schritte auf: Berechnen einer mittleren Leistungsabsorptionsdichte (oder einer hierzu äquivalenten Größe) im Applikator-Magnetfeld im Depotvolumen, wobei eine relative Leistungsabsorptionsdichte (oder einer hierzu äquivalenten Größe) basierend auf einer gemessenen geometrischen Verteilung der Nanopartikel berechnet wird, eine das Modell beschreibende BHTE genau einmal zum Erhalt einer basalen Temperaturverteilung ohne Leistungsabsorption numerisch gelöst wird, und eine das Modell beschreibende BHTE genau einmal zum Erhalt einer relativen Temperaturinkrementverteilung basierend auf der relativen Leistungsabsorptionsdichte numerisch gelöst wird; und wobei die relative Leistungsabsorptionsdichte durch einen temperaturbasierten Skalierungsfaktor skaliert wird, der basierend auf dem mindestens einen vorgegebenen Temperaturgrenzwert, der basalen Temperaturverteilung und der relativen Temperaturinkrementverteilung erhalten wird; Berechnen, basierend auf der berechneten mittleren Leistungsabsorptionsdichte und einer berechneten Masse der Nanopartikel, einer Referenz-Leistungsabsorptionsrate, die zum Beispiel die spezifische Leistungsabsorptionsrate einer die Nanopartikel enthaltenden, unverdünnten Magnetflüssigkeit angibt, üblicherweise bezogen auf die Masse der Nanopartikel in W/g; und Berechnen eines H-Feldstärkewertes basierend auf der berechneten Referenz-Leistungsabsorptionsrate und einer vorgegebenen, z.B. aus einer Referenzmessung abgeleiteten Kennlinie, die einen Zusammenhang zwischen Referenz-Leistungsabsorptionsrate und applizierter H-Feldstärke betrifft; Darüber hinaus optional, Berechnen der resultierenden Temperaturverteilung basierend auf der basalen Temperaturverteilung, der relativen Temperaturinkrementverteilung, und dem temperaturbasierten Skalierungsfaktor.

Die im ersten Berechnungsschritt berechnete basale Temperaturverteilung und/oder die relative Temperaturinkrementverteilung kann/können für mindestens eine weitere Verwendung über den ersten Berechnungsschritt hinaus bereitgestellt werden, bspw. im Arbeitsspeicher und/oder auf einer Festplatte abgespeichert werden. Die Verwendung kann bspw. die schnelle Berechnung, im zweiten Berechnungsschritt (für die Ausführungsform "schneller H-Regler"), einer resultierenden Temperaturverteilung basierend auf einem vom Anwender eingegebenen H-Feldstärkewert betreffen, ohne dass erneut die BHTE zum Erhalt der Temperaturverteilung und/oder der relativen Temperaturinkrementverteilung numerisch gelöst werden muss.

Im zweiten Berechnungsschritt bei der Ausführungsform "schneller "H-Regler" kann unabhängig von der Zahl der vorgegebenen und/oder anwenderdefinierten H-Feldstärkewerte eine bereit gestellte (zuvor im ersten Berechnungsschritt berechnete) basale Temperaturverteilung und/oder eine bereit gestellte (zuvor im ersten Berechnungsschritt berechnete) relative Temperaturinkrementverteilung herangezogen werden, bspw. die vorstehend erwähnten Temperaturverteilungen (basal und relativ inkremental), die aus dem ersten Berechnungsschritt heraus bereit gestellt werden. Damit kann eine erneute Speicher- und Rechenzeit-intensive numerische Lösung der BHTE für die Berechnung der basalen und/oder relativen inkrementalen Temperaturverteilung vermieden werden.

Bei bestimmten Ausführungsformen des erfindungsgemäßen Simulationsverfahrens (Ausführungsform "H-Regler") ist der zweite Berechnungsschritt so ausgelegt, dass die basale Temperaturverteilung und die relative inkrementelle Temperaturverteilung nicht aus dem Arbeitsspeicher bzw. von der Festplatte herangezogen werden, sondern als Teil des zweiten Berechnungsschrittes via numerischer Lösung der BHTE neu berechnet werden müssen. Dabei wird, unabhängig von der Zahl der vorgegebenen und/oder anwenderdefinierten H-Feldstärkewerte, die BHTE maximal zwei Mal numerisch gelöst, nämlich einmal zur Berechnung der basalen Temperaturverteilung und einmal zur Berechnung der relativen Temperaturinkrementverteilung.

Varianten des erfindungsgemäßen Simulationsverfahrens umfassen, dass im zweiten Berechnungsschritt (H-Regler, schneller H-Regler) die zu erwartende resultierende Temperaturverteilung mittels einer leistungsabsorptionsbasierten Skalierung ("K") einer berechneten oder bereit gestellten relativen Temperaturinkrementverteilung berechnet wird. Spezifische Ausführungsformen des Simulationsverfahrens umfassen im zweiten Berechnungsschritt (H-Regler, schneller H-Regler) die folgenden Schritte: Berechnen einer relativen Leistungsabsorptionsdichteverteilung (oder einer hierzu äquivalenten Größe) und einer relativen mittleren Leistungsabsorptionsdichte (oder einer hierzu äquivalenten Größe) basierend auf einer gemessenen geometrischen Verteilung der Nanopartikel; Bereitstellen einer basalen Temperaturverteilung basierend auf einer numerischen Lösung einer das Modell beschreibenden BHTE ohne Leistungsabsorption, und Bereitstellen einer relativen Temperaturinkrementverteilung basierend auf einer numerischen Lösung einer das Modell beschreibenden BHTE mit der berechneten relativen Leistungsabsorptionsdichteverteilung [die Bereitstellung erfolgt über Einlesen der zuvor abgespeicherten Verteilungen (beim "schnellen H-Regler") oder eine erneute numerische Lösung der BHTE(beim "H-Regler")]; Durchführen der folgenden Schritte für jeden H-Feldstärkewert der Mehrzahl vorgegebener H-Feldstärkewerte und/oder den anwenderdefinierten H-Feldstärkewert: Berechnen einer Referenz-Leistungsabsorptionsrate, die zum Beispiel die spezifische Leistungsabsorptionsrate einer die Nanopartikel enthaltenden, unverdünnten Magnetflüssigkeit angibt, wobei die Berechnung auf dem jeweiligen H-Feldstärkewert und einer vorgegebenen, z.B. aus einer Referenzmessung abgeleiteten Kennlinie basiert, die einen Zusammenhang zwischen Referenz-Leistungsabsorptionsrate und applizierter H-Feldstärke betrifft; Berechnen, basierend auf der Referenz-Leistungsabsorptionsrate und einer berechneten Masse der Nanopartikel im Depotvolumen, einer mittleren Leistungsabsorptionsdichte; Leistungsabsorptionsbasierte Skalierung, d.h. Berechnen eines leistungsabsorptionsbasierten Skalierungsfaktors basierend auf der jeweiligen mittleren Leistungsabsorptionsdichte und der relativen Leistungsabsorptionsdichte; Berechnen einer jeweiligen resultierenden Temperaturverteilung basierend auf der basalen Temperaturverteilung, der relativen Temperaturinkrementverteilung, und dem leistungsabsorptionsbasierten Skalierungsfaktor.

Erfindungsgemäß wird ein weiteres computergestütztes Simulationsverfahren (T-Vorgabe) zur Unterstützung einer Thermotherapie-Planung vorgeschlagen. Die Thermotherapie umfasst eine Hyperthermie-Behandlung eines Tumorvolumens in einem Körpervolumen eines menschlichen Körpers. Die Hyperthermie-Behandlung umfasst das Applizieren eines Magnetfeldes in einem Behandlungsvolumen mittels eines Magnetfeld-Applikators. In mindestens einem Depotvolumen ist mittels im Körper deponierter magnetischer, paramagnetischer und/oder superparamagnetischer Nanopartikel durch Leistungsabsorption im applizierten Magnetfeld Wärmeenergie einbringbar. Das Simulationsverfahren betrifft die Berechnung einer am Applikator einzustellenden H-Feldstärke basierend auf einer geometrischen Verteilung der Nanopartikel und mindestens einem vorgegebenen Temperaturgrenzwert, der durch die Hyperthermie-Behandlung nicht überschritten werden soll (T-Vorgabe). Der H-Feldstärkewert wird basierend auf einer vorgegebenen, z.B. aus einer Referenzmessung abgeleiteten Kennlinie berechnet, die einen Zusammenhang zwischen Leistungsabsorptionsrate und H-Feldstärke angibt.

Bei einer bestimmten Ausführungsform weist das Simulationsverfahren die folgenden Schritte auf: Berechnen einer mittleren Leistungsabsorptionsdichte (oder einer hierzu äquivalenten Größe) im Applikator-Magnetfeld im Depotvolumen, wobei eine relative Leistungsabsorptionsdichte basierend auf einer gemessenen geometrischen Verteilung der Nanopartikel berechnet wird, eine das Modell beschreibende BHTE genau einmal zum Erhalt einer basalen Temperaturverteilung ohne Leistungsabsorption numerisch gelöst wird, und eine das Modell beschreibende BHTE, die genau einmal zum Erhalt einer relativen Temperaturinkrementverteilung basierend auf der relativen Leistungsabsorptionsdichte numerisch gelöst wird; und wobei die relative Leistungsabsorptionsdichte durch einen temperaturbasierten Skalierungsfaktor skaliert wird, der basierend auf dem mindestens einen vorgegebenen Temperaturgrenzwert, der basalen Temperaturverteilung und der relativen Temperaturinkrementverteilung erhalten wird; Berechnen, basierend auf der berechneten mittleren Leistungsabsorptionsdichte und einer berechneten Masse der Nanopartikel, einer Referenz-Leistungsabsorptionsrate, die zum Beispiel die spezifische Leistungsabsorptionsrate einer die Nanopartikel enthaltenden, unverdünnten Magnetflüssigkeit angibt; Berechnen eines H-Feldstärkewertes basierend auf der berechneten Referenz-Leistungsabsorptionsrate und einer vorgegebenen, z.B. aus einer Referenzmessung abgeleiteten Kennlinie, die einen Zusammenhang zwischen ReferenzLeistungsabsorptionsrate und applizierter H-Feldstärke betrifft; vorzugsweise Berechnen der resultierenden Temperaturverteilung basierend auf der basalen Temperaturverteilung, der relativen Temperaturinkrementverteilung, und dem temperaturbasierten Skalierungsfaktor; und Bereitstellen des berechneten H-Feldstärkewertes und optional der zu diesem H-Feldstärkewert gehörigen resultierenden Temperaturverteilung, berechnet wie oben beschrieben, zur Unterstützung des Anwenders bei der Planung der Thermotherapie.

Erfindungsgemäß wird ein nochmals weiteres computergestütztes Simulationsverfahren (H-Regler; schneller H-Regler) zur Unterstützung einer Thermotherapie-Planung vorgeschlagen. Die Thermotherapie umfasst eine Hyperthermie-Behandlung eines Tumorvolumens in einem Körpervolumen eines menschlichen Körpers. Die Hyperthermie-Behandlung umfasst das Applizieren eines Magnetfeldes in einem Behandlungsvolumen mittels eines Magnetfeld-Applikators. In mindestens einem Depotvolumen ist mittels im Körper deponierter magnetischer, paramagnetischer und/oder superparamagnetischer Nanopartikel durch Leistungsabsorption im applizierten Magnetfeld Wärmeenergie einbringbar. Das Simulationsverfahren betrifft die Berechnung, für jeden H-Feldstärkewert einer Mehrzahl vorgegebener H-Feldstärkewerte und/oder einen anwenderdefinierten H-Feldstärkewert, einer zu erwartenden Temperaturverteilung für mindestens einen Teil des Körpervolumens (H-Regler). Die zu erwartende Temperaturverteilung wird mittels einer leistungsabsorptionsbasierten Skalierung einer berechneten oder bereit gestellten relativen Temperaturinkrementverteilung berechnet.

Bei bestimmten Varianten wird unabhängig von der Zahl der vorgegebenen und/oder anwenderdefinierten H-Feldstärkewerte , z.B. zum Ziele der Vermeidung der erneuten numerischen Lösung der BHTE eine zuvor (im T-Vorgabe-Schritt) berechnete basale Temperaturverteilung und/oder eine zuvor (im T-Vorgabe-Schritt) berechnete relative Temperaturinkrementverteilung herangezogen (schneller H-Regler). Zusätzlich oder alternativ kann - unabhängig von der Zahl der vorgegebenen und/oder anwenderdefinierten H-Feldstärkewerte - eine das Modell beschreibende BHTE kein Mal (schneller H-Regler), ein Mal (H-Vorgabe) oder maximal zwei Mal (H-Regler) numerisch gelöst werden, um anschließend die resultierende Temperaturverteilung zu bestimmen. Im Fall "kein Mal" (schneller H-Regler) wird die resultierende Temperaturverteilung aus der zuvor (in früheren Berechnungsschritten) via BHTE berechneten basalen Temperaturverteilung und der zuvor berechneten relativen Temperaturinkrementverteilung via leistungsabsorptionbasierter Skalierung zusammengesetzt. Im Fall "ein Mal" (H-Vorgabe) wird die resultierende Temperaturverteilung direkt, d.h. ohne Spaltung in basale und inkrementale Anteile via Lösung der BHTE numerisch berechnet. Im Fall "zwei Mal" (H-Regler) werden die basalen und die inkrementalen Temperaturverteilungen in diesem (zweiten) Berechnungsschritt einzeln via Lösung der BHTE numerisch berechnet und anschließend via leistungsabsorptionbasierter Skalierung zur resultierenden Temperaturverteilung zusammengesetzt.

Bei bestimmten Ausführungsformen weist das Simulationsverfahren die folgenden Schritte auf: Berechnen einer relativen Leistungsabsorptionsdichteverteilung (oder einer hierzu äquivalenten Größe) und einer relativen mittleren Leistungsabsorptionsdichte (oder einer hierzu äquivalenten Größe) basierend auf einer gemessenen geometrischen Verteilung der Nanopartikel; Bereitstellen einer basalen Temperaturverteilung basierend auf einer numerischen Lösung einer das Modell beschreibenden BHTE ohne Leistungsabsorption, und Bereitstellen einer relativen Temperaturinkrementverteilung basierend auf einer numerischen Lösung einer das Modell beschreibenden BHTE mit der berechneten relativen Leistungsabsorptionsdichteverteilung; Durchführen der folgenden Schritte für jeden H-Feldstärkewert der Mehrzahl vorgegebener H-Feldstärkewerte und/oder den benutzerdefinierten H-Feldstärkewert: Berechnen einer Referenz-Leistungsabsorptionsrate, die zum Beispiel die spezifische Leistungsabsorptionsrate einer die Nanopartikel enthaltenden, unverdünnten Magnetflüssigkeit angibt, wobei die Berechnung auf dem jeweiligen H-Feldstärkewert und einer vorgegebenen, z.B. aus einer Referenzmessung abgeleiteten Kennlinie basiert, die einen Zusammenhang zwischen Referenz-Leistungsabsorptionsrate und applizierter H-Feldstärke betrifft; Berechnen, basierend auf der Referenz-Leistungsabsorptionsrate und einer berechneten Masse der Nanopartikel im Depotvolumen, einer mittleren Leistungsabsorptionsdichte; Leistungsabsorptionsbasierte Skalierung, d.h. Berechnen eines leistungsabsorptionsbasierten Skalierungsfaktors basierend auf der jeweiligen mittleren Leistungsabsorptionsdichte und der relativen Leistungsabsorptionsdichte; Berechnen einer jeweiligen resultierenden Temperaturverteilung basierend auf der basalen Temperaturverteilung, der relativen Temperaturinkrementverteilung, und dem leistungsabsorptionsbasierten Skalierungsfaktor; Bereitstellen der berechneten Temperaturverteilungen zur Unterstützung des Anwenders bei der Planung der Thermotherapie.

Erfindungsgemäß wird ferner ein Computerprogramm vorgeschlagen, welches die Ausführung eines der hierin beschriebenen Simulationsverfahrens bewirkt, wenn das Computerprogramm auf einer programmierbaren Computereinrichtung ausgeführt wird, bspw. einem Computer in einer Klinik oder Arztpraxis. Das Computerprogramm kann auf einem maschinenlesbaren Datenträger gespeichert sein bzw. gespeichert vorliegen, bspw. einem permanenten oder wiederbeschreibbaren Medium in oder in Zuordnung zu einer programmierbaren Computereinrichtung oder einer CD-ROM, DVD oder einem USB-Stick. Zusätzlich oder alternativ kann das Computerprogramm zum Herunterladen auf eine programmierbare Computereinrichtung bereitgestellt werden, zum Beispiel über ein Datennetzwerk wie das Internet oder eine Kommunikationsverbindung wie etwa eine Telefonleitung und/oder eine drahtlose Verbindung.

Erfindungsgemäß wird darüber hinaus eine Computereinrichtung bereitgestellt, die zur Unterstützung einer Thermotherapie-Planung ausgebildet ist. Die Thermotherapie umfasst dabei eine Hyperthermie-Behandlung eines Tumorvolumens in einem Körpervolumen eines menschlichen Körpers, wobei die Hyperthermie-Behandlung das Applizieren eines Magnetfeldes in einem Behandlungsvolumen mittels eines Magnetfeld-Applikators umfasst. Dabei ist in mindestens einem Depotvolumen mittels im Körper deponierter magnetischer, paramagnetischer und/oder superparamagnetischer Nanopartikel durch Leistungsabsorption im applizierten Magnetfeld Wärmeenergie einbringbar. Die Computereinrichtung weist die folgenden Komponenten auf: eine erste Berechnungskomponente, die zum Berechnen eines am Applikator einzustellenden H-Feldstärkewertes ausgebildet ist, basierend auf einer geometrischen Verteilung der Nanopartikel und mindestens einem vorgegebenen Temperaturgrenzwert, der durch die Hyperthermie-Behandlung nicht überschritten werden soll; eine zweite Berechnungskomponente, die zum optionalen Berechnen einer zu erwartenden Temperaturverteilung ausgebildet ist, für mindestens einen Teil des Körpervolumens für jeden H-Feldstärkewert einer Mehrzahl vorgegebener H-Feldstärkewerte, und/oder einen anwenderdefinierten H-Feldstärkewert; ; eine optionale dritte Komponente ausgebildet zum Berechnen einer zu erwartenden Temperaturverteilung für einen maximal zulässigen H-Feldstärkewert, wenn der im ersten Berechnungsschritt berechnete H-Feldstärkewert größer ist als dieser maximal zulässige H-Feldstärkewert; und eine Komponente zum Bereitstellen (dem Anwender) des berechneten am Applikator einzustellenden H-Feldstärkewertes und optional mindestens einer berechneten Temperaturverteilung (z.B. derjenigen zum vorgenannten H-Feldstärkewert zugehörigen) zur Unterstützung des Anwenders bei der Planung der Thermotherapie.

Eine weitere erfindungsgemäße Computereinrichtung ("T-Vorgabe") ist ausgebildet zur Unterstützung einer Thermotherapie-Planung, wobei die Thermotherapie eine Hyperthermie-Behandlung eines Tumorvolumens in einem Körpervolumen eines menschlichen Körpers umfasst und wobei die Hyperthermie-Behandlung das Applizieren eines Magnetfeldes in einem Behandlungsvolumen mittels eines Magnetfeld-Applikators umfasst. Dabei ist in mindestens einem Depotvolumen mittels im Körper deponierter magnetischer, paramagnetischer und/oder superparamagnetischer Nanopartikel durch Leistungsabsorption im applizierten Magnetfeld Wärmeenergie einbringbar. Die Computereinrichtung weist eine Komponente auf, die ausgebildet ist zur Berechnung einer am Applikator einzustellenden H-Feldstärke basierend auf einer geometrischen Verteilung der Nanopartikel und mindestens einem vorgegebenen Temperaturgrenzwert, der durch die Hyperthermie-Behandlung nicht überschritten werden soll. Die Komponente weist dabei ein Modul zum Berechnen des H-Feldstärkewertes basierend auf einer vorgegebenen z.B. aus einer Referenzmessung abgeleiteten, Kennlinie auf, wobei die Kennlinie einen Zusammenhang zwischen Leistungsabsorptionsrate und H-Feldstärke angibt. In einer bevorzugten erfindungsgemäßen Variante der Computereinrichtung weist die Computereinrichtung die folgenden Module auf: ein Modul zum Berechnen einer mittleren Leistungsabsorptionsdichte im Applikator-Magnetfeld im Depotvolumen, wobei eine relative Leistungsabsorptionsdichte basierend auf einer gemessenen geometrischen Verteilung der Nanopartikel berechnet wird, eine das Modell beschreibende BHTE genau einmal zum Erhalt einer basalen Temperaturverteilung ohne Leistungsabsorption numerisch gelöst wird, und eine das Modell beschreibende BHTE genau einmal zum Erhalt einer relativen Temperaturinkrementverteilung basierend auf der relativen Leistungsabsorptionsdichte numerisch gelöst wird; und wobei die relative Leistungsabsorptionsdichte durch einen temperaturbasierten Skalierungsfaktor skaliert wird, der basierend auf dem mindestens einen vorgegebenen Temperaturgrenzwert, der basalen Temperaturverteilung und der relativen Temperaturinkrementverteilung erhalten wird; ein Modul zum Berechnen, basierend auf der berechneten mittleren Leistungsabsorptionsdichte und einer berechneten Masse der Nanopartikel, einer Referenz-Leistungsabsorptionsrate, die zum Beispiel die spezifische Leistungsabsorptionsrate einer die Nanopartikel enthaltenden, unverdünnten Magnetflüssigkeit angibt; ein Modul zum Berechnen eines H-Feldstärkewertes basierend auf der berechneten Referenz-Leistungsabsorptionsrate und einer vorgegebenen , z.B. aus einer Referenzmessung abgeleiteten, Kennlinie, die einen Zusammenhang zwischen Referenz-Leistungsabsorptionsrate und applizierter Feldstärke betrifft; ; optional ein Modul zum Berechnen der resultierenden Temperaturverteilung basierend auf der basalen Temperaturverteilung, der relativen Temperaturinkrementverteilung, und dem temperaturbasierten Skalierungsfaktor; ein Modul zum Bereitstellen des berechneten Feldstärkewertes zur Unterstützung des Anwenders bei der Planung der Thermotherapie; und optional ein Modul zum Bereitstellen der zu dem berechneten H-Feldstärkewert gehörigen resultierenden Temperaturverteilung zur Unterstützung des Anwenders es bei der Planung der Thermotherapie.

Eine weitere erfindungsgemäße Computereinrichtung ("H-Regler", "schneller H-Regler") ist ausgebildet zur Unterstützung einer Thermotherapie-Planung, wobei die Thermotherapie eine Hyperthermie-Behandlung eines Tumorvolumens in einem Körpervolumen eines menschlichen Körpers umfasst und wobei die Hyperthermie-Behandlung das Applizieren eines Magnetfeldes in einem Behandlungsvolumen mittels eines Magnetfeld-Applikators umfasst. Dabei ist in mindestens einem Depotvolumen mittels im Körper deponierter magnetischer, paramagnetischer und/oder superparamagnetischer Nanopartikel durch Leistungsabsorption im applizierten Magnetfeld Wärmeenergie einbringbar. Die Computereinrichtung weist eine Komponente auf, die ausgebildet ist zur Berechnung, für jeden Feldstärkewert einer Mehrzahl vorgegebener Feldstärkewerte und/oder einen anwenderdefinierten Feldstärkewert, einer zu erwartenden Temperaturverteilung für mindestens einen Teil des Körpervolumens. Die Komponente weist dabei ein Modul zum Berechnen der zu erwartenden Temperaturverteilung mittels einer leistungsabsorptionsbasierten Skalierung einer berechneten oder bereit gestellten Temperaturinkrementverteilung auf. Vorzugsweise ist die Komponente zum Berechnen der zu erwartenden Temperaturverteilung ausgebildet, um unabhängig von der Zahl der vorgegebenen und/oder anwenderdefinierten H-Feldstärkewerte eine bereit gestellte (zuvor berechnete im T-Vorgabe-Schritt) basale Temperaturverteilung und/oder eine bereit gestellte (zuvor berechnete im T-Vorgabe-Schritt) relative Temperaturinkrementverteilung heranzuziehen (schneller H-Regler).

Weiter vorzugsweise ist die Komponente zum Berechnen der zu erwartenden Temperaturverteilung ausgebildet, unabhängig von der Zahl der vorgegebenen und/oder anwenderdefinierten Feldstärkewerte maximal zwei Temperaturverteilungen zu berechnen (H-Regler), nämlich eine basale Temperaturverteilung und/oder eine relative Temperaturinkrementverteilung.

Die erfindungsgemäße Computereinrichtung weist weiter vorzugsweise die folgenden Module auf ("H-Regler, "schneller H-Regler"): ein Modul zum Berechnen einer relativen Leistungsabsorptionsdichteverteilung und einer relativen mittleren Leistungsabsorptionsdichte basierend auf einer gemessenen geometrischen Verteilung der Nanopartikel; ein Modul zum Bereitstellen einer basalen Temperaturverteilung basierend auf einer numerischen Lösung einer das Modell beschreibenden BHTE ohne Leistungsabsorption, und Bereitstellen einer relativen Temperaturinkrementverteilung basierend auf einer numerischen Lösung einer das Modell beschreibenden BHTE mit der berechneten relativen Leistungsabsorptionsdichteverteilung; ein Modul zum Durchführen der folgenden Schritte für jeden H-Feldstärkewert der Mehrzahl vorgegebener H-Feldstärkewerte: Berechnen einer Referenz-Leistungsabsorptionsrate, die zum Beispiel die spezifische Leistungsabsorptionsrate einer die Nanopartikel enthaltenden, unverdünnten Magnetflüssigkeit angibt, wobei die Berechnung auf dem jeweiligen H-Feldstärkewert und einer vorgegebenen, z.B. aus einer Referenzmessung abgeleiteten Kennlinie basiert, die einen Zusammenhang zwischen Referenz-Leistungsabsorptionsrate und applizierter H-Feldstärke betrifft; Berechnen, basierend auf der Referenz-Leistungsabsorptionsrate und einer berechneten Masse der Nanopartikel im Depotvolumen, einer mittleren Leistungsabsorptionsdichte; leistungsabsoptionsabhängige Skalierung, d.h. Berechnen eines leistungsabsorptionsbasierten Skalierungsfaktors basierend auf der jeweiligen mittleren Leistungsabsorptionsdichte und der relativen Leistungsabsorptionsdichte; Berechnen einer jeweiligen resultierenden Temperaturverteilung basierend auf der basalen Temperaturverteilung, der relativen Temperaturinkrementverteilung, und dem leistungsabsorptionsbasierten Skalierungsfaktor; und ein Modul zum Bereitstellen der berechneten Temperaturverteilungen zur Unterstützung des Anwenders bei der Planung der Thermotherapie.

Erfindungsgemäß wird außerdem ein System vorgeschlagen, welches eine Computereinrichtung wie vorstehend skizziert und einen Magnetfeld-Applikator bzw. seine Teile umfasst. Die erfindungsgemäße Computereinrichtung kann zur Verwendung mit dem Magnetfeld-Applikator ausgebildet sein, bspw. dadurch dass etwa eine am Applikator maximal einstellbare H-Feldstärke und/oder messtechnische Eingangsdaten wie eine, z. B. aus einer Referenzmessung abgeleitete Kennlinie zur Leistungsabsorption in Abhängigkeit von der H-Feldstärke herangezogen werden. Der Magnetfeld-Applikator kann an die erfindungsgemäße Computereinrichtung angepasst werden, indem der Applikator Daten von diesem entgegennimmt, wie etwa einen einzustellenden H-Feldstärkewert (etwa nach Ende der Simulationen als Defaultwert und/oder auf Veranlassung des Anwenders). Die Computereinrichtung kann für diese Ausführungsform auch zur Planung oder Steuerung dienen.

Erfindungsgemäß wird weiterhin ein System vorgeschlagen, dass ein Computerprogramm wie oben skizziert, einen Datenträger wie oben skizziert, eine Computereinrichtung wie oben skizziert, oder ein System wie oben skizziert umfasst, und welches weiterhin eine Magnetflüssigkeit umfasst, die magnetische Nanopartikel enthält. Das erfindungsgemäße Computerprogramm kann auf die Verwendung der Magnetflüssigkeit angepasst sein, indem die für diese spezifische Kennlinie Leistungsabsorption vs. H-Feldstärke bei den Simulationen herangezogen wird. Das Computerprogramm kann für die Verwendung einer Mehrzahl von Magnetflüssigkeiten ausgebildet sein; hierbei müsste der Anwender vor Beginn der Simulationen die tatsächlich zu verwendende Flüssigkeit eingeben, etwa durch Auswahl aus einem Menü in einer GUI der Computereinrichtung.

Statt in einer Magnetflüssigkeit könnten die Nanopartikel prinzipiell auch in einer anderen Darreichungsform in den Körper des Patienten instilliert werden. Alle Darreichungsformen sollen erfindungsgemäß umfasst sein.

Beschrieben wird auch ein Verfahren zur kontrollierten Erwärmung eines Organs oder Gewebes mit den folgenden Schritten: Einbringen von magnetischen, paramagnetischen und/oder superparamagnetischen Nanopartikeln in ein Organvolumen oder Gewebevolumen; Ermitteln der Nanopartikelmenge und/oder -Verteilung in dem Organvolumen oder Gewebevolumen; Berechnen einer einzustellenden H-Feldstärke gemäß einem der entsprechenden, oben skizzierten Verfahren oder einer resultierenden Temperaturverteilung gemäß einem der entsprechenden, oben skizzierten Verfahren; und Deponieren von Wärmeenergie mittels Applikation eines Magnetfeldes, wobei die applizierte H-Feldstärke eingestellt wird, die der berechneten Feldstärke oder der aus einer berechneten Temperaturverteilung abgeleiteten Feldstärke jeweils mit einer Abweichung von +/- 10%, bevorzugt +/- 5%, insbesondere +/- 1% entspricht. Dieses Verfahren kann gegebenenfalls *in vitro* durchgeführt werden.

Ein Verfahren zur Behandlung eines Tumors in einem Patienten enthält die folgenden Schritte: Einbringen einer geeigneten Menge von magnetischen, paramagnetischen und/oder superparamagnetischen Nanopartikeln in ein Tumorvolumen; Ermitteln der Nanopartikelmenge und/oder -Verteilung in dem Tumorvolumen; Berechnen einer einzustellenden Feldstärke gemäß einem der entsprechenden, oben skizzierten Verfahren oder einer resultierenden Temperaturverteilung gemäß einem der entsprechenden, oben skizzierten Verfahren; Deponieren von Wärmeenergie mittels Applikation eines Magnetfeldes, wobei die applizierte Feldstärke eingestellt wird, die der berechneten Feldstärke oder der aus einer berechneten Temperaturverteilung abgeleiteten Feldstärke jeweils mit einer Abweichung von +/- 10%, bevorzugt +/- 5%, insbesondere +/- 1% entspricht.

Geeignete Verfahren zur Behandlung von Tumoren oder zur kontrollierten Erwärmung eines Organs oder Gewebes mittels geeigneter Nanopartikel und Applikation eines Magnetfeldes, die durch die erfindungsgemäßen Simulationsverfahren verbessert werden können, sind aus dem Stand der Technik bekannt. So beschreiben Maier-Hauff et al. (2010, supra) eine erfolgreiche Studie an 66 Patienten mit Gehirntumoren, 59 davon mit Glioblastoma. Darüber hinaus wurden ähnliche Verfahren erfolgreich an Prostatakarzinompatienten durchgeführt (Johannsen, M., et al. (2007), Eur Urol. 52(6): 1653-61. Epub 2006 Nov 17; Johannsen, M., et al. (2010), Int J Hyperthermia 26(8): 790-5.) sowie in einer Studie, in der Patienten mit verschiedenen Tumoren, nämlich Chondrosarkom, Rektalkarzinom, Zervixkarzinom, Rhabdomyosarkom, Pharapharyngealsarkom und Prostatakarzinom, eingeschlossen und behandelt wurden (Wust, P., et al. (2006), Int J Hyperthermia. 22(8): 673-85.). Klinische Daten aus geeigneten Verfahren sind in Thiesen, B. and A. Jordan (2008, Int J Hyperthermia. 24(6): 467-74) zusammengefasst. Weitere Verfahren zur Behandlung von Tumoren bzw. zur kontrollierten Erwärmung eines Organs oder Gewebes mittels geeigneter Nanopartikel und Applikation eines Magnetfeldes sind aus US 20080268061, US 20110052609, WO 2009/100716 und WO 2011/082796 bekannt.

Bevorzugt sind solide Tumore, insbesondere lokale und lokal fortgeschrittene Tumore, oder systemische Tumorerkrankungen, die lokale Probleme bereiten wie inoperable Metastasen. Beispiele sind Gehirntumore, z.B. Glioblastom und Astrozytom, Gehirnmetastasen, Prostatakrebs, Pankreaskrebs, hepatozelluläres Karzinom, Hals- und Nackentumore, Blasenkrebs, Magenkrebs, Darmkrebs, Nierenzellkarzinom, Ovarialkarzinom, Cervixkarzinom, Sarkome, Basalzellkarzinom und Melanom.

Die Verfahren zur kontrollierten Erwärmung eines Organs oder Gewebes können beispielsweise zur Behandlung von Arthrose, Arthritis und anderen rheumatischen Gelenkerkrankungen angewandt werden. Die Behandlung dieser Erkrankungen mittels ähnlicher Verfahren sind z.B. aus WO 01/13949 bekannt.

VORTEILE DER ERFINDUNG

Die Erfindung stellt dem Anwender umfassende Möglichkeiten bereit, sich einen Überblick über verschiedene Therapieoptionen bzw. deren Auswirkungen zu verschaffen und die Therapie entsprechend zu planen. Das erfindungsgemäße Simulationswerkzeug stellt dazu automatisch eine Temperaturverteilung zur Verfügung, die basierend auf einer maximalen Feldstärke, aber unter Berücksichtigung von vorgegebenen /eingegebenen Temperaturgrenzwerten errechnet wurde. Erfindungsgemäß können zwei (oder mehr) Grenzwerte berücksichtigt werden, z.B. eine maximale Temperatur außerhalb des Behandlungsgebietes, um das gesunde Gewebe zu schonen, und/oder eine maximale Temperatur innerhalb des Behandlungsgebietes, um den Tumor zu zerstören aber dennoch einen Leistungseintrag in den Körper des Patienten zu begrenzen. Es ist prinzipiell denkbar, eine geometrische Temperaturgrenzwertverteilung vorzugeben, die bspw. gewebespezifische Besonderheiten berücksichtigt.

Das erfindungsgemäße Simulationswerkzeug kann "intelligent" sein in dem Sinne, dass er einen resultierenden Feldstärkewert verwirft, wenn dieser größer ist als bspw. ein maximaler einstellbarer Wert am Applikator / für einen gegebenen Patienten. In diesem Falle berechnet das Simulationswerkzeug eine neue Temperaturverteilung neu basierend auf dem maximal einstellbaren Feldstärkewert.

Der Anwender kann entscheiden, ob er den hieraus resultierenden Feldstärkewert heranziehen will, ohne weitere Simulationen durchzuführen. Hierdurch eröffnet das erfindungsgemäße Simulationswerkzeug dem erfahrenen Anwender die Möglichkeit, die Planung zu beenden, ohne dass Ressourcen verschwendet werden. Das Simulationswerkzeug kann dann bspw. bereits für den nächsten Patienten verwendet werden.

Der Anwender kann auch entscheiden, sich einen umfassenderen Überblick zu verschaffen. In diesem Fall kann das Simulationswerkzeug z.B. Temperaturverteilungen für mehrere Feldstärkewerte berechnen. Diese Berechnungen können im Hintergrund durchgeführt werden, sobald der Benutzer eine entsprechende Eingabe gemacht hat (ggf. kann das Simulationswerkzeug auch so konfiguriert werden, dass es diese Berechnungen automatisch im Hintergrund startet, nachdem die initiale Temperaturverteilung bereitgestellt wurde). Somit kann der Anwender ohne Zeitverzug bereits mit den Ergebnissen des ersten Berechnungsschrittes arbeiten.

Bei diesen Temperaturverteilungen werden die oben diskutierten Temperaturgrenzwerte nicht berücksichtigt. Somit erhält der Anwender hier eine umfassende Entscheidungsgrundlage über die Auswirkungen bestimmter Feldstärkeeinstellungen und kann sich je nach dem Therapieziel, der Schwere der Erkrankung, dem ggf. betroffenen Gewebe etc. u.U. für eine Therapieoption entscheiden, bei der der Temperaturgrenzwert oder die Grenzwerte nicht überall eingehalten werden.

Zusätzlich oder alternativ nimmt das Simulationswerkzeug einen vom Anwender eingegebenen Feldstärkewert entgegen und berechnet für diesen die resultierende Temperaturverteilung. Bei bestimmten Ausführungsformen der Erfindung geschieht dies zeitsparend ohne erneute numerische Lösung der BHTE, so dass dem Anwender das Ergebnis im Wesentlichen ohne Wartezeit zur Verfügung steht, bspw. bereits nach einer Sekunde oder weniger.

Mittels des erfindungsgemäßen Simulationswerkzeuges ist also das Therapieziel ebenso wie mögliche Nebenwirkungen besser und einfacher vorhersagbar und kann so auch besser und umfassender kontrolliert werden.

Das erfindungsgemäße Simulationswerkzeug benötigt hierbei sowohl für die Berechnung der Temperaturverteilung anhand vorgegebener Temperatur-Grenzwerte (erster Berechnungsschritt, s.u. Temperaturvorgabe bzw. "T-Vorgabe") als auch für die Berechnung einer Skala von Temperaturverteilungen oder eines anwenderdefinierten Feldstärkewertes (zweiter Berechnungsschritt, s.u. H-Feldstärkeregler, "H-Regler" oder "schneller H-Regler") genau zwei numerische Lösungen einer BHTE mit anschließender Skalierung, kommt also im ersten Schritt ohne iterative Trial-and-Error-Annäherungen (via immer wieder neu gestartete numerische Lösungen der BHTE) an den gesuchten Feldstärkewert aus und kann im zweiten Schritt für prinzipiell beliebig viele Feldstärkewerte die entsprechenden Temperaturverteilungen per einfacher Skalierung berechnen. Hierdurch ist das Simulationswerkzeug anwenderfreundlich, weil die Berechnungsergebnisse äußerst genau und dennoch schnell verfügbar sind. Das erfindungsgemäße Simulationswerkzeug benötigt im Vergleich zu einem iterativen Trialand-Error-Ansatz nur einen Bruchteil der CPU-Rechenzeit und macht dadurch eine klinisch praktikable Anwendung erst möglich, sogar unter Benutzung von älteren/ressourcenbegrenzten Rechnern.

KURZBESCHREIBUNG DER FIGUREN

Weitere Aspekte und Vorteile der Erfindung werden nunmehr anhand der beigefügten Figuren eingehender beschrieben. Hierbei zeigt:

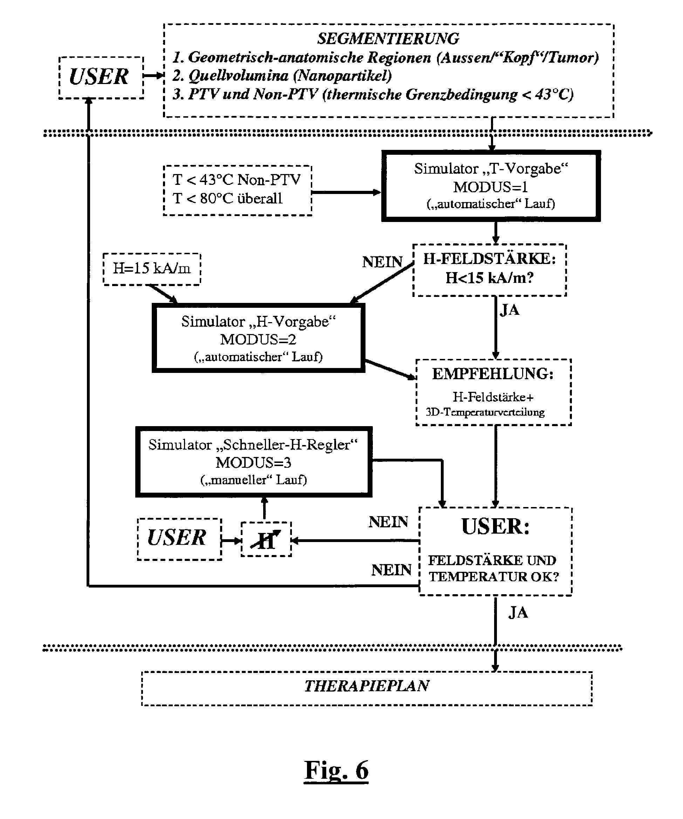

Fig. 1 in Form eines Flussdiagramms einen generellen Programmablauf eines ersten Ausführungsbeispiels eines erfindungsgemäßen Simulationswerkzeugs mit den Hauptschritten: "Patientendaten", "Bildfusionierung", "Segmentierung", "Temperatursimulation" und "Therapieplan". Im Hauptschritt "Temperatursimulation" wird das Programmpaket "Temperatursimulator" (kurz "Simulator" genannt) aufgerufen. In dem ersten Ausführungsbeispiel (Fig. bis Fig.5) beinhaltet das Simulator-Paket drei autarke Simulator-(Ausfiihrungs-) Programme (.exe), jedoch kann der Simulator auch als eine anlinkbare Programmbibliothek mit drei Haupt-Unterprogrammen ("*mainsubroutines*") ausgeführt werden (s. zweites Ausführungsbeispiel Fig.6 bis Fig.9);

Fig. 2 in Form eines Flussdiagramms einen Programmablauf eines ersten Ausführungsbeispiels eines erfindungsgemäßen Simulators, mit einer festgelegten Abfolge von Aufrufen der Simulator-Programme;

Fig. 3 in Form eines Flussdiagramms einen Programmablauf des Simulators aus Fig. 2 im sog. Modus 2 (Ausfiihrungsprogramm "sim_h.exe": Vorgabe der H-Feldstärke, abgekürzt "H-Vorgabe";

Fig. 4 in Form eines Flussdiagramms einen Programmablauf des Simulators aus Fig. 2 im Modus 1 (Ausführungsprogramm "sim_t.exe": Temperaturvorgabe, abgekürzt T-Vorgabe");

Fig. 5 in Form eines Flussdiagramms einen Programmablauf des Simulators aus Fig. 2 im Modus 3 (Ausführungsprogramm "sim_hr.exe": sog. H-Feldstärkeregler, abgekürzt "H-Regler");

Fig. 6 in Form eines Flussdiagramms einen Programmablauf eines zweiten Ausführungsbeispiels eines erfindungsgemäßen Simulators, mit einer festgelegten Abfolge von Aufrufen der Simulator-Haupt-Unterprogramme;

Fig. 7 in Form eines Flussdiagramms einen Programmablauf einer der drei Haupt-Unterprogramme des Simulators aus Fig. 6 im Modus 2, nämlich "mainsubroutine sim h voxel win" (H-Vorgabe);

Fig. 8 in Form eines Flussdiagramms einen Programmablauf einer der drei Haupt-Unterprogramme des Simulators aus Fig. 6 im Modus 1, nämlich "mainsubroutine sim t voxel win" (T-Vorgabe); und

Fig. 9 in Form eines Flussdiagramms einen Programmablauf einer der drei Haupt-Unterprogramme des Simulators aus Fig. 6 im Modus 3, nämlich "mainsubroutine sim hr voxel win" (sog. "Schneller H-Regler").

AUSFÜHRUNGSBEISPIELE DER ERFINDUNG

Nachfolgend wird ein erstes Ausführungsbeispiel eines erfindungsgemäßen Simulationswerkzeugs genauer beschrieben. Fig. 1 zeigt in Form einer schematischen Darstellung einen Programmablauf eines erfindungsgemäßen Simulationsverfahrens in Form einer Simulationssoftware, die mit dem Ziel der Unterstützung der NanoTherm® Therapie entwickelt wurde.

Die Software liefert dem behandelnden Arzt (Neurochirurg, Radiologe) im Rahmen einer Thermotherapie von malignen Tumoren wie bspw. Hirntumoren eine Orientierungshilfe zur Abschätzung der Behandlungstemperaturen und der dazu erforderlichen Magnetfeldstärke auf Basis einer das Modell beschreibenden BHTE.

Zur Abschätzung der Therapiefeldstärke bietet die Software die Möglichkeit, Bilddaten im DICOM-Format z.B. von Kernspin- und CT-Aufnahmen zu importieren und zu registrieren. Ebenso besteht die Möglichkeit, Konturierungen auf allen verwendeten Bilddateien durchzuführen (Segmentierung).

Die Software führt den Anwender durch Abfrage der erforderlichen Parameter schrittweise bis zum Ergebnis. Dies beinhaltet zum einen eine 3-D Visualisierung, aus der die konturierten Bereiche wie Tumor, Katheter und Nanopartikeldepots zu ersehen sind. Zum anderen wird eine Abschätzung der Temperaturverteilung in den gekennzeichneten Bereichen in Abhängigkeit von der Feldstärke und der Therapiezeit dargestellt. Dies ist besonders wichtig für Bereiche, die nur eingeschränkt behandelbar sind, wie zum Beispiel im Kopf frontobasal im Bereich des Chiasma opticum (Hypothalamus), der Sylvischen Fissur in der der Gefäßbaum der Arteria cerebri media verläuft, des Corpus callosum, oder des Hirnstamms.

Die eingegebenen Daten sowie die erstellten Simulationen können gespeichert und ausgedruckt werden und es können verschiedene Szenarien pro Patient erstellt werden. Das Simulationswerkzeug unterstützt so den Arzt oder Anwender bei der Anfertigung des Therapieplans. Die Freigabe des Therapieplans erfolgt bspw. durch Unterschrift des verantwortlichen Arztes auf dem ausgedruckten Therapievorschlag. Die im Therapievorschlag präsentierten Simulationsergebnisse können zur Orientierung dienen und müssen keinen bestimmten Genauigkeitsanspruch erheben.

Die Simulationssoftware kann auf kundeneigener Hardware betrieben werden, entsprechende Mindestanforderungen müssen erfüllt sein. Die Umgebungsbedingungen entsprechen denen einer Umgebung zur Anwendung medizinischer Software.

Überblick über das erste Ausführungsbeispiel

Die Software umfasst als Programmpaket einen "Temperatur-Simulator" (im Folgenden auch kurz als "Simulator" bezeichnet). Nach der Einbringung von Magnetflüssigkeit in einen Tumor ermöglicht der Simulator eine Simulation der Temperaturverteilung im Kopfbereich in Abhängigkeit von der Magnetfeldstärke des Therapiegerätes (Applikator). Der Simulator berechnet eine bestimmte Feldstärke im Sinne einer unverbindlichen Empfehlung. Für die Therapiedurchführung können zusätzlich während der Therapie Temperaturmessungen durchgeführt werden, so dass Simulationsergebnisse und Temperaturmessungen gemeinsam die Basis zur Beurteilung der Therapie durch den Arzt bilden können, wobei bevorzugt die Temperaturmessungen maßgebend sind. Natürlich sind die Simulationsergebnisse weder Voraussetzung für die Durchführbarkeit der Therapie noch sind sie für die Durchführung bindend.

Fig. 1 zeigt die Hauptschritte eines Programmablaufs. Das Programmpaket "Simulator" wird in einem Hauptschritt "Temperatursimulation" aufgerufen. Folgende Hauptaufgaben werden vom Simulator erfüllt:

•	Simulation der dreidimensionalen Temperaturverteilung, die sich durch die Anwendung des Magnetfeldes auf z.B. superparamagnetische oder ferrimagnetische Nanopartikel unter der Annahme eines vereinfachten physikalischen Modells (dieses wird unten genauer beschrieben) voraussichtlich ergibt; und

•	Abschätzung der H-Feldstärke etwa auf Basis von bestimmten Temperaturvorgaben für das Patientenmodell.

Das Simulator-Paket ist nicht direkt Teil des Cores der Simulationssoftware, sondern ist im Sinne einer externen SOUP ("*Software of Unknown Provenience*") über eine fest definierte externe I/O-Schnittstelle an den Core angebunden.

Das Simulator-Paket umfasst drei unabhängige Simulator-Programme sim_t, sim_h, und sim_hr, die in FORTRAN77 geschrieben sind (Ausführungsprogramme "sim_t.exe", "sim_h.exe" und "sim_hr.exe"). Die Reihenfolge der Aufrufe der Simulator-Programme und die Verwaltung der Simulator-Daten übernimmt der Core der Simulationssoftware, hier "Hauptprogramm-Core" genannt. Der Hauptprogramm-Core verwaltet auch alle anderen Hauptschritte, die in Fig.1. dargestellt wurden, wie z:B. Bildfusionierung, Segmentierung, u.a. In diesem ersten Ausführungsbeispiel erfolgt der Datenaustausch zwischen dem Core und dem Simulator, inklusive der Ausgabe von Programmabbruchmeldungen, über das Lesen/Schreiben in bzw. aus einem Festplatten-Verzeichnis.

Der Programm-Hauptschritt "Temperatursimulation" im Detail

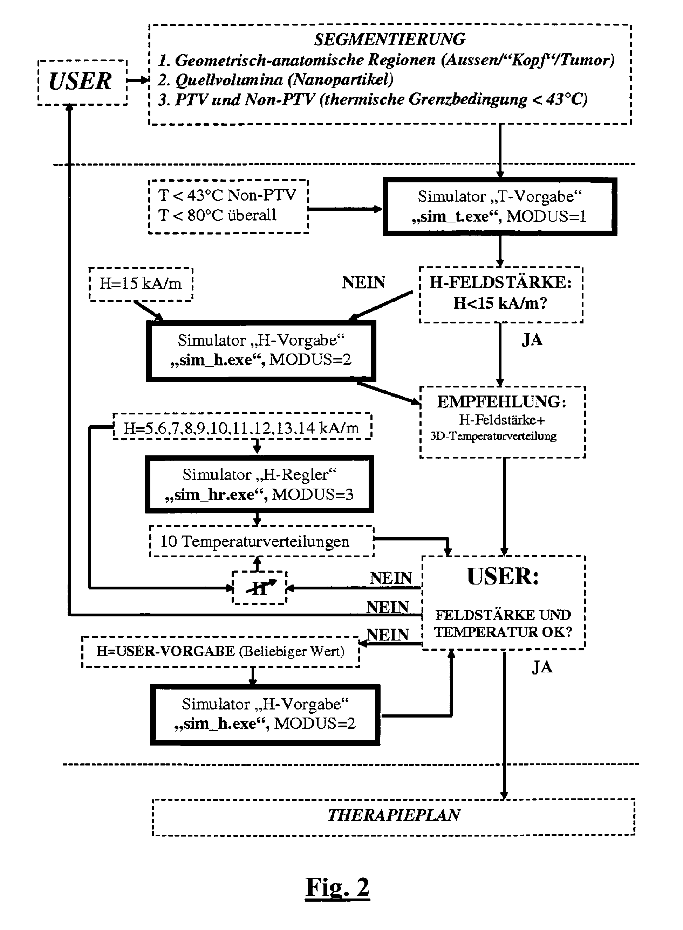

Die zeitliche Abfolge der Aufrufe der Simulator-Programme im Hauptschritt "Temperatursimulation" wird vom Hauptprogramm-Core aus gesteuert bzw. verwaltet und kann nach einem Schema erfolgen, wie es in Fig. 2 dargestellt ist.

Es ist festgelegt, dass nach jeweiligem User-Wechsel in der GUI ("Graphical User Interface", graphische Benutzeroberfläche) beim Wechsel vom Segmentierungs-Menue zum Temperatursimulations-Menue, in diesem Menue als erstes das T-Vorgabe-Programm sim_t.exe (Modus=1) startet. Dieses Programm hat automatisch (d.h. ohne Eingabe des Nutzers) folgende zwei Grenztemperatur-Vorgaben zu erfüllen,:

- Maximal 43°C außerhalb des PTV ("Non-PTV-43°C-Limit")
- Maximal 80°C überall sonst, d.h. de facto innerhalb des PTV ("Whole-Body-80°C-Limit").

Dabei bezeichnet PTV ("Planning Target Volume") das Behandlungsgebiet / Behandlungsvolumen, d.h. das zu behandelnde bzw. zu erwärmende Volumen. Dieses kann vom Anwender im Hauptschritt Segmentierung festgelegt werden (die Simulationssoftware kann, etwa auf Basis einer manuell vorgenommenen Tumorkonturierung, einen Vorschlag für das Behandlungsvolumen machen, bspw. Tumorvolumen plus Saum, der vom Anwender übernommen oder verändert werden kann, also etwa eingeschränkt oder ausgeweitet werden kann). Ein Temperaturwert von 43°C wird als Schwelltemperatur implementiert, oberhalb welcher Schädigungen des gesunden Gewebes vermehrt auftreten können, die möglichst vermieden werden sollen. Ein Temperaturwert von 80°C wird als allgemeine Temperaturbegrenzung implementiert, die im gesamten Körper nicht überschritten werden soll, also etwa auch im Tumorvolumen nicht; hierdurch wird gleichzeitig eine Leistungsabsorption vom Körper insgesamt begrenzt. Von den beiden Grenzbedingungen wird diejenige wirksam, die bei niedrigerer H-Feldstärke eintritt.

Es ist jedoch anzumerken, dass die Grenzwerte von 43°C und 80°C auf bspw. die Behandlung von Tumoren in einem Körpervolumen wie dem menschlichen Kopf angewendet werden. Hyperthermie-Behandlungen bezogen auf andere Körpervolumina wie bspw. Prostatabereich können auf die Beachtung anderer Grenzwerte ausgerichtet sein. Bspw. kann der Grenzwert im PTV 100°C statt 80°C bei einer Thermotherapie eines Prostatatumors betragen.

Nach der Ausführung von sim_t.exe untersucht die Simulationssoftware zunächst intern, d.h. ohne dass dem Nutzer die Werte per GUI mitgeteilt werden, wie hoch der intern als sim_t-Output ausgegebene H-Feldstärkewert ist. Ist dieser Wert größer als 15 kA/m (KiloAmpere pro Meter), so startet automatisch (ohne dass der Nutzer eine Eingabe machen muss) ein Durchlauf von sim_h.exe (Modus 2) mit der Vorgabe 15 kA/m (H-Vorgabe), wobei 15 kA/m der maximale einstellbare H-Feldstärkewert am Magnetfeld-Applikator ist.

Ist der sim_t.exe-Outputwert kleiner oder gleich 15 kA/m, muss kein Programmlauf sim_h.exe automatisch gestartet werden und es folgt direkt der nächste Schritt.

In diesem nächsten Schritt erfolgt die graphische Ausgabe der Temperaturverteilung und der folgenden Werte an die GUI:

- die H-Feldstärke-Empfehlung (der Output von sim_t.exe oder 15 kA/m vom automatischen Lauf sim_h.exe); diese Ausgabe erfolgt im GUI-Fenster; und
- die erreichte Maximaltemperatur außerhalb des PTV, sowie die erreichte Maximaltemperatur im Behandlungsgebiet, sowie ggf. weitere Outputgrößen; diese Ausgabe erfolgt in einem Pop-Up Fenster.

Es kann Fälle geben, in denen die vorgegebene Temperatur von z.B. 43°C außerhalb des PTV nicht erreicht wird, und zwar dann, wenn bereits bei kleinerem H-Feldstärkewert die Grenzbedingung 80°C im PTV erreicht worden ist.

Nach der oben beschriebenen Ausgabe des Initialergebnisses kann der Benutzer veranlassen, dass der "H-Regler" bzw. Feldstärkeregler sim_hr.exe (Modus=3) gestartet wird (bei anderen Ausführungsbeispielen kann der Feldstärkeregler auch automatisch gestartet werden). Das System führt diese Berechnungen im Hintergrund durch, so dass der Nutzer die Gelegenheit hat, sich in der Zwischenzeit die initial ermittelte Temperaturverteilung anzuschauen. Optional kann der Benutzer den Start des H-Reglers überspringen, oder die Berechnung ordentlich abbrechen.

Der H-Regler berechnet im hier beschriebenen Konfigurationsbeispiel zehn Temperaturverteilungen für die folgenden festen Werte der H-Feldstärke (multiple H-Vorgabe): 5 kA/m, 6 kA/m, 7 kA/m, 8 kA/m, 9 kA/m, 10 kA/m, 11 kA/m, 12 kA/m, 13 kA/m und 14 kA/m. Damit ist der gesamte Einstellbereich des bei diesem Beispiel verwendeten Applikators in 1kA/m-Schritten abgedeckt. Wenn der Durchlauf erfolgt ist, bekommt der Nutzer die Möglichkeit, sich mittels der Einstellung einer Position am entsprechenden Balken die Temperaturverteilungen für diese H-Feldstärkewerte anzuschauen.

Die angegebene Liste der H-Feldstärkewerte bezieht sich auf die Behandlung von bspw. Glioblastomen im Kopf. Für Behandlungen in anderen Körperbereichen bzw. -volumina können andere Werte vorgegeben werden. Bspw. kann zur Behandlung von Prostatatumoren eine Schrittweite von nur 0.5 kA/m für Werte zwischen 2 kA/m, 2.5 kA/m, ...., 8 kA/m vorgegeben werden.

Der H-Regler hat keinerlei Begrenzungen hinsichtlich der erreichten Temperaturen (anders als sim_t.exe im Initiallauf). Daher kann es Fälle geben, dass die erreichten Temperaturen höher (oder niedriger) sind als 80°C im Berechnungsgebiet und/oder höher (und/oder niedriger) als 43°C außerhalb von PTV.

Will sich der Nutzer neben der Feldstärkeempfehlung von sim_t.exe und ggf. den 10 Ergebnissen des H-Reglers einen nochmals weiteren Überblick verschaffen, so hat er die Möglichkeit, einen gewünschten Input-H-Feldstärke-Wert in einem GUI-Fenster manuell einzutippen. Dann startet die Berechnung im H-Vorgabe-Modus (sim_h.exe). Dies kann er beliebig wiederholen.

Im H-Vorgabe-Modus gibt es keinerlei Begrenzungen hinsichtlich der erreichten Temperaturen (anders als sim_t.exe im Initiallauf). Daher kann es Fälle geben, dass die erreichten Temperaturen höher (oder niedriger) sind als 80°C im Berechnungs- bzw. Behandlungsgebiet und/oder höher (und/oder niedriger) als 43°C außerhalb des PTV.

Der Nutzer kann außerdem jederzeit zum Segmentierungsschritt zurückkehren, um Korrekturen am PTV oder andere Segmentierungskorrekturen vorzunehmen. In diesem Fall wechselt der Simulator in den Ausgangszustand. Sobald ein neuer segmentierter Datensatz (sog. "Labeled Volume-" bzw. LV-Datensatz, s.u.) vorliegt und der User vom Segmentierungsschritt in das Temperaturberechnungsmenue wechselt, wiederholt sich die ganze Prozedur, d.h. der Simulator startet mit dem Initiallauf sim_t.exe.

Allgemeiner technischer Ansatz

Die Erzeugung der Temperaturerhöhung durch Nanopartikel im Magnetfeld wird gedanklich in zwei Schritte aufgespalten:

1. In einem ersten Schritt bewirken die Nanopartikel im Magnetfeld eine lokale Leistungsabsorption, angegeben durch eine Leistungsabsorptionsdichte (W/m$^3$) bzw. -rate (W/kg), hier allg. mit SAR ("Specific Absorption Rate") bezeichnet;

2. In einem zweiten Schritt fungiert diese SAR als (Haupt-) Quelle der Temperaturerhöhung.

Die Simulation von Temperaturen, die sich aus Werten der H-Feldstärke ergeben, wird im Simulator in zwei Hauptschritte aufgeteilt:

1. Ermittlung einer SAR-Verteilung für eine gegebene H-Feldstärke durch einen hier so bezeichneten "SAR-Solver" (d.h. eine entsprechende Berechnungskomponente bzw. ein Berechnungsmodul); und

2. Ermittlung der Temperaturverteilung ("T-Verteilung") aus der SAR-Verteilung (im sog. "T-Solver").

In dem oben eingeführten SAR-Solver wird die SAR anhand messtechnischer Daten bestimmt, bspw. basierend auf einem CT(Computertomographie)-Datensatz mit markierten Magnetflüssigkeitsdepots (s.u.) und einer messtechnisch ermittelten Abhängigkeit der Eisenkern-SAR von der H-Feldstärke. Physikalische Approximationen können zur Implementierung herangezogen werden wie sie bspw. in Gneveckow et al. 2004 dargestellt sind.

Im T-Solver wird eine zeitabhängige BHTE mit Hilfe von Finiten Differenzen unter Berücksichtigung besonderer Gegebenheiten der Anwendung mit Magnetflüssigkeiten numerisch gelöst. Der T-Solver kann explizite Temperaturgradienten verwenden, wie sie bspw. in Nadobny et al. 2007, Appendix, beschrieben sind.

Im Simulator-Paket bilden der SAR-Solver und der T-Solver keine getrennten Programmeinheiten, sondern sind zu einem gemeinsamen Programm verschmolzen, wobei je nach Vorgabe-Modus die SAR- und T-Solver-Anteile im Programmablauf verschiedenartig untereinander "vermischt" sind. Dies wird unten genauer beschrieben.

Die geometrische Form der SAR-Verteilung (und infolgedessen der Temperaturverteilung) ist stark von den geometrischen Positionen der im Gewebe angedockten Nanopartikel bzw. Depots der magnetischen Flüssigkeit ("Nanodepots", "Depotvolumen") abhängig. Diese Nanopartikel-Positionen müssen dem Simulator als Input mitgeteilt werden, um die SAR zu bestimmen. Hierzu muss im vorangegangenen Hauptschritt "Segmentierung" ein auf der Grundlage des Planungs-CTs erzeugter segmentierter (z.B. binärer) dreidimensionaler Datensatz (hier als "LV.raw" bezeichnet, mit LV für "Labeled Volume", s.u.) mit markierten Nanopartikeln dem Simulator zur Verfügung gestellt werden. Außerdem müssen die Werte der Hounsfield Units an den Stellen der Nanopartikel bekannt sein. Dies geschieht durch das Einlesen des binären CT-Files CT.raw.

Die Leistungsabsorption der Nanopartikel im Magnetfeld kann im Simulator auf unterschiedliche Weise repräsentiert werden. Eine spezifische Leistungsabsorption kann durch eine Leistungsabsorptionsrate ("Specific Absorption Rate", SAR) in Einheiten von Watt pro Kilogramm oder Gramm (W/kg bzw. W/g) quantifiziert werden, wobei hier als Masse die magnetisch wirksame Masse anzusetzen ist, also etwa die Masse der Magnetflüssigkeit (hier wird die SAR üblicherweise in W/kg angegeben) oder die Eisenmasse bei Nanopartikeln mit Eisenkern (hier wird die SAR üblicherweise in W/g angegeben). Statt durch eine Rate kann eine spezifische Leistungsabsorption auch durch eine Leistungsabsorptionsdichte in Einheiten von Watt pro Kubikmeter oder Kubikzentimeter ($W/m^3$ oder $W/cm^3$) angegeben werden. Insoweit es sich um eine spezifische Leistungsabsorptionsdichte handelt, beträfe das Volumen etwa das Volumen der Magnetflüssigkeit.

Eine spezifische Referenz-Leistungsabsorptionsrate oder -dichte kann sich z.B. auf die (gemessene) Leistungsabsorption einer Magnetflüssigkeit mit einem Carrier (wie etwa Wasser) und den darin "gelösten" Nanopartikeln beziehen. Dabei ist die Menge der Nanopartikel im Carrier bei einer solchen Referenzmessung genauestens bekannt (z.B. in molarer Masse). Die Referenzangabe bezieht sich also auf eine Magnetflüssigkeit im Referenzzustand vor der Instillation. Die hier verwendete Eisenkern-SAR "SAR_fe" ist eine solche Referenzangabe, die üblicherweise auf die Eisenmasse bezogen ist und in W/g angegeben wird.

Die tatsächliche Leistungsabsorption ("Absorbed Power Rate" bzw. "Absorbed Power Density", APD, in $W/m^3$) im Gewebe hängt von der dort vorhandenen Dichte der magnetisch wirksamen (Massen der) Nanopartikel ab. Somit ist die tatsächliche bzw. gewebespezifische Leistungsabsorption im Gewebe gegenüber der spezifischen Referenz-Leistungsabsorption in dieser Weise dichteabhängig verändert; i.A. liegen die Nanopartikel im Gewebe in gegenüber der Referenz-Magnetflüssigkeit geringerer Dichte vor (sie sind "verdünnt"). Um die Menge der verwendeten Begriffe zu begrenzen, soll die Verwendung der APD vermieden werden. Wir sprechen daher auch bei der tatsächlichen Leistungsabsorption weiter bspw. von der SAR oder einer räumlichen SAR-Verteilung SAR (x,y,z), auch wenn strenggenommen die Einheit $W/m^3$ anzusetzen wäre. Der Sprachgebrauch ist insofern gerechtfertigt, als die Umrechnung von SAR in APD oder umgekehrt für den Simulator einfach möglich ist: Die tatsächliche Dichte der (Eisenmasse der) Nanopartikel pro Voxel ist bekannt, sie wird aus den CT-Daten (z.B. Grauwerte in HU) abgeleitet. Auch die (spezifische / tatsächliche) Leistungsabsorptionsrate und Leistungsabsorptionsdichte können jeweils durch einen Dichtefaktor (magnetisch wirksame Massendichte in der Magnetflüssigkeit / im Gewebe nach der Instillation) ineinander umgerechnet werden. In diesem Sinne sprechen wir hier auch von einer Volumen-SAR. Somit sind die Begriffe "SAR" bzw. "Volumen-SAR" mit dem Begriff der Leistungsabsorptionsdichte äquivalent, und es ist eine Frage der numerischen Optimierung, in welcher Form die Daten vom Simulator gehalten werden, anders als bei der "(Referenz-) Leistungsabsorptionsrate", z.B. der SAR_fe, die auf die Masse der Nanopartikel in W/g bezogen ist.

Anders gesagt betrifft der Begriff der "Leistungsabsorptionsdichte" wie er hier verwendet wird eine (Volumen-)SAR, während der Begriff der "(Referenz-) Leistungsabsorptionsrate" eine außerhalb des Körpers (in vitro) messtechnisch bestimmte Referenzgröße betrifft, wie die SAR_fe.

Unter einer Volumen-SAR kann also sowohl eine im Körper deponierte Leistungsabsorptionsrate in W/kg verstanden werden, als auch eine im Körper deponierte Leistungsabsorptionsdichte in Einheiten von z.B. $W/m^3$ (Watt pro Kubikmeter)verstanden werden. Daneben wird auch eine (Referenz-) SAR-Rate bzw. (Referenz-) Leistungsabsorptionsrate verwendet, die in Einheiten von z.B. W/kg (Watt pro Kilogramm), W/g (Watt pro Gramm), etc. angegeben wird.

Dem allgemeinen Sprachgebrauch folgend, wird der Zusatz "-verteilung" gelegentlich weggelassen. Es kann sich also bei einer Angabe um eine ortsabhängige Verteilung handeln, also SAR (x,y,z), oder um einen ortsunabhängigen Wert, etwa eine mittlere Leistungsabsorptionsdichte wie die Volumen-SAR SAR_aver. Ob eine Verteilung vorliegt oder nicht, ergibt sich dem Fachmann aus dem allgemeinen Zusammenhang.

Zur Funktionalität einzelner Komponenten bzw. Module beim ersten Ausführungsbeispiel Je nach Input- bzw. Vorgabe-Modus wird das Simulator-Ausführungsprogramm "sim_t.exe", "sim_h.exe" oder "sim_hr.exe" vom Hauptprogramm der Simulationssoftware aus in einer bestimmten zeitlichen Reihenfolge aufgerufen. Mögliche Reihenfolgen wurden oben bereits besprochen.

Der Simulator bietet zwei Wege bzw. Optionen zur Ermittlung (Simulation) einer absoluten Temperaturverteilung:

- Über die sog. "H-Vorgabe" ("H" steht als Symbol für H-Feldstärke). Vorgegeben wird der absolute Wert der Feldstärke H (typischerweise in kA/m). Gesucht wird die Temperaturverteilung T(x,y,z) (in °C).

- Über die sog. "T-Vorgabe" ("T" steht für die Temperatur). Vorgegeben werden Temperaturgrenzwerte T_grenz (in °C), gesucht wird der Feldstärkewert H (in kA/m) und die dazugehörige Temperaturverteilung T(x,y,z) (in °C).

Die T-Vorgabe wird mit dem Ausführungsprogramm "sim_t.exe" und die H-Vorgabe mit dem Ausführungsprogramm sim_h.exe realisiert. Das dritte Programmodul, sim_hr.exe, arbeitet auch mit einer (multiplen) H-Vorgabe. Sim_t.exe und sim_h.exe berechnen jeweils eine Temperaturverteilung, während bei sim_hr.exe (dem sog. "H-Regler") in einem Ablauf für zehn Werte der H-Feldstärkezehn Temperaturverteilungen ermittelt werden.

Dem Berechnungstyp "T-Vorgabe" (sim_t.exe) wird ein Übergabe-Parameter (bzw. Inputwert aus der Sicht des Simulators) "MODUS" mit dem Wert MODUS=1, der "H-Vorgabe" (sim_h.exe) MODUS=2 und dem "H-Regler" (sim_hr.exe) MODUS=3 zugewiesen.

Im Folgenden werden die drei Simulator-Programmodule (die jeweils auch als eigenständige Programme vorliegen könnten) mit ihren Funktionalitäten beispielhaft genauer beschrieben.

H-Vorgabe (MODUS=2, sim h.exe)

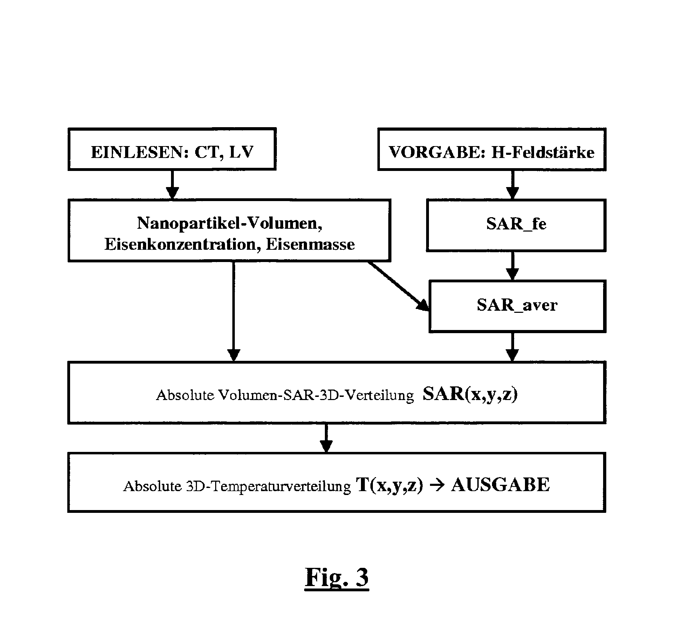

Eine schematische Darstellung des Ablaufs des Programmoduls sim_h.exe ist in Fig. 3 dargestellt. Vorgegeben wird der absolute Wert der H-Feldstärke H. Gesucht wird die Temperaturverteilung T(x,y,z).

Zunächst werden aus der Auswertung der geometrischen Nanopartikel-Verteilung (eingelesen via LV.raw) und Vergleich der Hounsfield-Units ("HU(x,y,z)", eingelesen via CT.raw) das Nanodepot-Volumen (Depotvolumen) bzw. Nanopartikel-Volumen ("V NP") und ein mittlerer HU-Wert "HU_aver" aus allen Werten HU(x,y,z) im V_NP gebildet.

Anschließend wird aus dem Mittelwert "HU_aver" die mittlere Eisenkonzentration und daraus die Eisenmasse "m_fe" (allgemeiner: die Partikelmasse) im V_NP abgeschätzt, wobei hier davon ausgegangen wird, das die Nanopartikel einen Eisenkern als magnetisch wirksame Komponente haben. Diesem Vorgehen liegen also mehrere Approximationen zugrunde: m_fe wird aus der mittleren Eisenkonzentration abgeschätzt, die wiederum aus dem mittlerem HU-Wert abgeschätzt wird. Die Konzentration der Nanopartikel nach der Instillation in den Patienten wird aus den CT-Daten bestimmt. Nur etwa 50% der instillierten Partikel bleiben im Körper und liegen in den "Nanodepots" in schwer vorherzusehenden Konzentrationen bzw. Verteilungen vor.

Unabhängig davon leitet der Simulator aus der H-Feldstärke die Eisenkern-SAR "SAR_fe" ab. Hierbei handelt es sich um eine Referenz-Leistungsabsorptionsrate in Einheiten von z.B. W/g bezogen auf die Eisenmasse, die eine gemessene Leistungsabsorption der Nanopartikel in einem unverdünnten Referenzzustand angibt, also etwa die Leistungsabsorptionsrate einer die Nanopartikel enthaltenden, unverdünnten Magnetflüssigkeit (unverdünnten Charge) vor der Instillation in einen Patienten.

Die Nanopartikel können magnetisch (also etwa ferro- oder ferrimagnetisch), paramagnetisch, und/oder superparamagnetisch sein. Je nach Parametern wie Material, Größenverteilung, etc. kann eine Mischung verschiedener magnetischer Eigenschaften vorliegen.

Die Berechnung der SAR_fe geschieht über Anwendung einer zuvor für die Magnetflüssigkeit messtechnisch bestimmten nicht-linearen Kennlinie SAR_fe = f(H). Die Kennlinie wird für einen konkreten Applikator sowie konkret verwendete Nanopartikel (Magnetflüssigkeit) bestimmt. Es wird angenommen, dass sich das Behandlungsgebiet zentral zwischen Polschuhen des Applikators befindet (z.B. in einem Bereich von <+/-10 Zentimetern), so dass dort überall in guter Näherung derselbe (maximale) H-Feldstärkewert eingesetzt werden kann. Um eine aufwendige tabellarische Darstellung zu vermeiden, kann die Kennlinie SAR_fe = f(H) durch drei Fitting-Faktoren a, b, c, approximiert werden, sodass sie eine Form

$$SAR\_fe = aH^b + c \qquad\qquad (1)$$

annehmen kann, mit den Einheiten SAR_fe in W/g (Watt pro Gramm) und H in kA/m.

Mit m_fe, SAR_fe und V_NP wird nun ein Mittelwert "SAR_aver" der Volumen-SAR(x,y,z) bezogen auf die Gewebemasse in W/kg (oder, äquivalent: multipliziert mit der spezifischen lokalen Dichte in $W/m^3$ oder $W/cm^3$) im Nanodepot abgeschätzt. Hierbei handelt es sich also um eine mittlere Leistungsabsorptionsdichte im deponierten Zustand der Nanopartikel.

Anschließend erfolgt die Bildung einer ortsabhängigen Volumen-SAR-Verteilung, wobei erfindungsgemäß mit guter Näherung angenommen wird, dass in V_NP die SAR-Werte zu den HU-Werten proportional sind, d.h.

$$SAR(x,y,z) = HU(x,y,z) * SAR\_aver / HU\_aver \qquad in\ V\_NP \qquad (2),$$

$$SAR(x,y,z)=0 \qquad \text{außerhalb von V\_NP (3).}$$

Alle Werte HU(x,y,z) sind in V_NP positiv, so dass keine physikalisch unmöglichen negativen SAR-Werte auftreten können.

Mit ortsabhängiger Leistungsabsorptions-"Dichte"-Verteilung SAR(x,y,z) als Quelle der Temperatur wird dann eine BHTE T(x,y,z) = f(SAR(x,y,z)) numerisch gelöst, z.B. basierend auf Nadobny et al. 2007, Eqs. (1)-(2), wobei hierbei ein Finite-Differenzen-Verfahren mit expliziter Temperaturgradienten-Berechnung nach Nadobny et al. 2007, Eqs. (8)-(15) angewandt wird.

Die das Modell beschreibende BHTE ist dynamisch, d.h. zeitabhängig. Nach einiger Zeit wird ein Steady-State-Zustand erreicht, bei dem die Wärmezufuhr durch Leistungsabsorption im applizierten Magnetfeld der Wärmeabfuhr durch Blutfluss, Kühlung an der Umgebung, Perfizsionsterme gleich ist. Erfahrungsgemäß wird ein derartiger Zustand nach ca. 20 min erreicht. Im Simulator kann vorgegeben sein, dass unter Berücksichtigung einer entsprechenden Sicherheitsmarge der Steady-State-Zustand nach z.B. 30 min (Minuten) als erreicht gelten soll (eine Hyperthermie-Behandlung kann z.B. 1 Stunde bis zu 1.5 Stunden dauern). Im Prinzip kann der Anwender auch an der anfänglichen Phase (vor Erreichen eines Steady-State) interessiert sein. Der Simulator arbeitet im Zeitbereich, kann somit auch beliebige Zeitpunkte vor einem Wert von 20 min oder 30 min modellieren und bereitstellen (ausgeben).

Die H-Vorgabe-Option (sim_h.exe) (MODUS=2) benötigt lediglich einen einzigen Durchlauf zur numerischen Lösung der BHTE T(x,y,z) = f(SAR(x,y,z)).

Es wird erfindungsgemäß vorgeschlagen, dass bei der H-Vorgabe die Temperatur nicht begrenzt wird, d.h. je nach der Höhe der H-Feldstärke können sich beliebige Temperaturen im Körper einstellen, die auch über den üblichen Temperaturgrenzwerten (z.B. 43°C im gesunden Gewebe) liegen können.

T-Voragbe (MODUS=1, sim_t.exe)

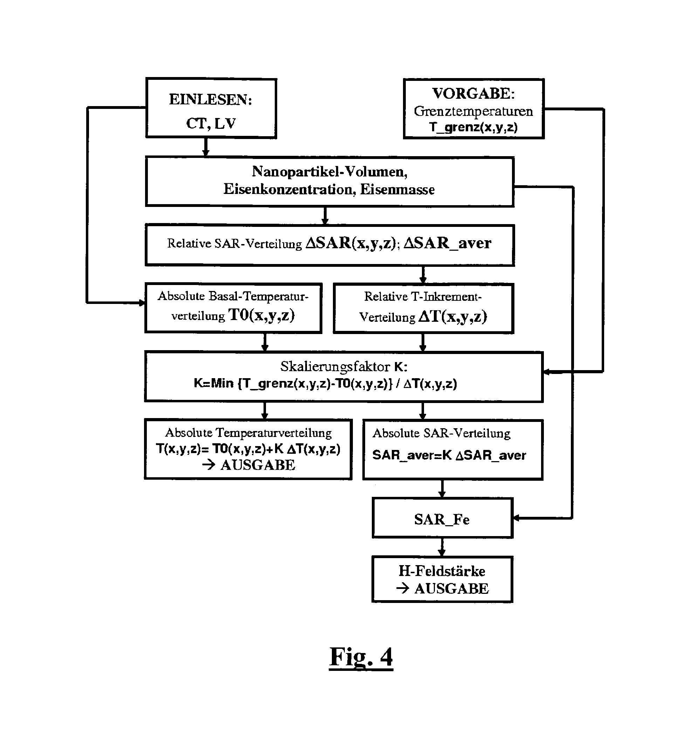

Fig. 4 zeigt schematisch einen Programmablauf des Simulators im T-Vorgabe Modus. Vorgegeben werden Temperaturgrenzwerte T_grenz(x,y,z), gesucht wird der Feldstärkewert H und die dazugehörige Temperaturverteilung T(x,y,z).

Zunächst werden - wie bei sim_h.exe - die Nanopartikel-Verteilung aus LV.raw und die Hounsfield Units via CT.raw eingelesen. Anschließend wird die Eisenkonzentration (allg. die magnetisch wirksame Partikelkonzentration) und daraus die Eisenmasse m_fe (allg. die magnetisch wirksame Partikelmasse) wie bei sim_h.exe bestimmt. Diese Größen sind unabhängig von einer angelegten H-Feldstärke.

Der weitere Verlauf von sim_t.exe ist jedoch anders als bei sim_h.exe, da der absolute Wert der Feldstärke nicht vorgegeben, sondern nunmehr gesucht wird. Die Prozedur umfasst die Berechnung einer entsprechenden Volumen-SAR (in W/kg, oder wahlweise als Leistungsabsorptionsdichte in $W/m^3$), die zur vorgegebenen Grenztemperatur "passt", sowie die Findung der H-Feldstärke aus der berechneten Volumen-SAR.

Die im hier beschriebenen Beispiel gleichzeitig und gleichberechtigt zu erfüllenden Grenztemperatur-Vorgaben T_grenz(x,y,z) sind:

- Für die sog. "Non-PTV"-Region (im Sinne einer Boolschen Operation "Körpervolumen minus PTV") wird ein maximal zulässiger Temperaturwert (=Temperaturgrenzwert) T_grenz(Non-PTV) vorgegeben. Dieser Wert kann z.B. defaultmäßig im Hauptprogramm der Simulationssoftware auf 43°C gesetzt sein und so dem Simulator übergeben werden, d.h. T_grenz(Non-PTV) = 43°C. Die "Non-PTV"-Region entspricht in etwa dem gesunden bzw. nicht zu behandelndem Gewebe.

- Die Temperatur überall im Körpervolumen soll maximal 80°C betragen. Dieser Wert kann z.B. intern im Simulator festgesetzt sein. Im Zusammenspiel mit dem obigen Grenzwert, nach dem im Non-PTV höchstens 43°C herrschen sollen, ist dies eine Begrenzung für das Behandlungsgebiet PTV, daher T_grenz(PTV)=80°C.

Die resultierende Feldstärke H muss beide Vorgaben erfüllen, d.h. sie soll so niedrig sein, dass alle Temperaturen T(x,y,z) kleiner gleich 80°C im PTV und gleichzeitig kleiner gleich 43°C außerhalb des PTV sind. Mit anderen Worten, von zwei Feldstärkewerten, die jeweils eine der beiden T-Vorgaben erfüllen, wird der niedrigere ausgegeben.

Zur Erfüllung dieser T-Vorgaben könnte theoretisch sim_h.exe mehrere Male gestartet werden und die Input-Feldstärke immer wieder nachgestellt werden, so dass am Ende eines solchen iterativen Prozesses die T-Vorgaben erfüllt wären. Dieser iterative und entsprechend ungenaue bzw. aufwendige Weg (vgl. "iterive way " in Nadobny et al. 2007, S.1841) wird hier nicht verfolgt.

Erfindungsgemäß wird hier anders vorgegangen, um den H-Feldstärkewert direkt, ressourcenschonend und dennoch genau zu bestimmen. Dabei ist zu beachten, dass die Problemstellung keineswegs trivial ist, da die SAR (und damit Temperatur) von der H-Feldstärke auf nicht-lineare Weise abhängt. Jedoch lässt sich zumindest für einen Teil der Problemstellung ein linearer Zusammenhang angeben, und zwar zwischen der SAR und der Temperatur (vgl. "decomposition way" in Nadobny et al. 2007, S.1841, Eqs. (5a), (5b), (6)). So wird die Temperaturverteilung T(x,y, z) zunächst in einen basalen Anteil T0(x,y,z), der sich ohne SAR ergibt, und in einen Temperaturanstiegs-Anteil T_anstieg(x,y,z) = K*ΔT(x,y,z) aufgeteilt, so dass:

$$T(x,y,z) = T0(x,y,z) + K * \Delta T(x,y,z), \qquad (4)$$

und für die SAR gilt

$$SAR(x,y,z) = K * \Delta SAR(x,y,z). \qquad (5)$$

K ist ein skalarer Skalierungsfaktor (wir sprechen im MODUS=1 vom "temperaturbasierten" Skalierungsfaktor), ΔT(x,y,z) ist der relative Temperaturinkrement und ΔSAR(x,y,z) ist die relative SAR-Verteilung, die auch als relative Leistungsabsorptionsdichte bezeichnet werden kann bzw. zu dieser äquivalent ist. Anders als bei sim_h_exe wird kein absoluter Wert SAR_aver aus SAR_fe bestimmt, sondern es wird ein beliebiger (relativer) Test-Mittelwert "ΔSAR_aver" für die relative Volumen-SAR ΔSAR(x,y,z) intern vorgegeben. Zum Beispiel kann im Simulator dieser Test-Mittelwert als ΔSAR_aver = 100 W/kg gesetzt sein. Ähnlich wie in Glg. (2) werden erfindungsgemäß daraus die ortsabhängigen ΔSAR(x,y,z)-Werte wie folgt approximiert:

$$\Delta SAR(x,y,z) = HU(x,y,z) * \Delta SAR\_aver / HU\_aver \quad \text{in V\_NP}, \qquad (6)$$

$$\Delta SAR(x,y,z) = 0 \qquad\qquad \text{außerhalb von V\_NP}. \qquad (7)$$

Als Nächstes werden zwei Durchläufe zur numerischen Lösung der BHTE nacheinander gestartet: einmal für T0(x,y,z) (mit SAR(x,y,z)=0) und einmal für den relativen Temperaturinkrement ΔT(x,y,z)=f(ΔSAR(x,y,z)). Anschließend wird der temperaturbasierte Skalierungsfaktor mit Hilfe einer Minimumsuche über alle Stützpunkte bzw. Voxel x,y,z gefunden:

$$K = Min(T\_grenz(x,y,z) - T0(x,y,z)) / \Delta T(x,y,z)), \qquad (8)$$

mit T_grenz(x,y,z) = 80°C im Non_PTV und 43°C im PTV.

Nachdem der temperaturbasierte Skalierungsfaktor K gefunden wurde, können sofort die absolute SAR(x,y,z) und T(x,yz) mit Hilfe der Gln. (5) und (4) angegeben werden, ohne dass noch einmal die BHTE numerisch gelöst werden muss. Für den Mittelwert von SAR(x,y,z) gilt ebenfalls:

$$SAR\_aver = K * \Delta SAR\_aver. \qquad (9)$$

Anschließend werden erfindungsgemäß die Anfangsschritte aus sim_h.exe in inverser Reihenfolge durchgeführt: Als erstes wird aus dem Mittelwert SAR_aver und der zuvor bestimmten Eisenmasse m_fe die Eisenkern-SAR SAR_fe bestimmt. Der letzte Schritt ist die Anwendung einer im allgemeinen nicht-linearen Kennlinie SAR_fe = f(H) im inversen Sinne, d.h. aus dem Wert SAR_fe wird der H-Feldstärkewert ermittelt, der dann schließlich - neben der Temperaturverteilung T(x,y,z) - als Output dem Nutzer mitgeteilt wird.

H-Regler (MODUS=3, sim hr.exe)

Fig. 5 zeigt schematisch einen Programmablauf des Simulators im H-Regler Modus. Das Simulator-Programm sim_hr.exe simuliert in einem Aufruf gleich mehrere Temperaturverteilungen im Sinne einer multiplen H-Vorgabe, d.h. für verschiedene Absolutwerte von H, die als Eingabewerte zur Verfügung gestellt werden.

In dem hier beschriebenen Beispiel wird ein Satz von zehn Temperaturverteilungen für einen Satz von zehn fest vorgegebenen Feldstärkewerten (5, 6, 7, 8, 9, 10, 11, 12, 13 und 14 kA/m) bestimmt. Der H-Regler ist jedoch nicht einfach ein zehnfach nacheinander durchlaufendes sim_h.exe mit zehn Durchläufen zur numerischen Lösung der BHTE. Um Rechenzeit zu sparen, werden vielmehr - ähnlich wie in sim_t.exe - nur zwei Durchläufe benötigt, nämlich ebenfalls für T0(x,y,z) und ΔT(x,y,z).

Zunächst wird - ähnlich wie bei sim_h.exe - aus der Auswertung der geometrischen Nanopartikel-Verteilung (eingelesen via LV.raw) und Vergleich der Hounsfield-Units (eingelesen via CT.raw) der mittlere HU-Wert HU_aver, daraus die mittlere Partikel- bzw. Eisenkonzentration und daraus die Partikel- bzw. Eisenmasse m_fe bestimmt. Diese Werte sind für alle H-Werte gleich.

Anschließend erfolgt - wie in sim_t-exe - für einen relativen Wert von ΔSAR_aver=100 die Abschätzung von ΔSAR_aver und eine Approximation von ΔSAR(x,y,z) nach den obigen Gln. (6) und (7).

Ähnlich wie in sim_t_exe werden als nächstes zwei Durchläufe zur numerischen Lösung der BHTE für T0(x,y,z) und ΔT(x,y,z) durchgeführt.

Als Nächstes startet eine Schleife mit i=10 Wiederholungen. Jedes Mal wird der entsprechende H-Feldstärke-Wert H(i) eingelesen, also z.B. 5...14 kA/m in 1 kA/m-Schritten. Aus dem Wert wird - wie bei sim_h.exe - jedes Mal über die Anwendung der Kennlinie SAR_fe=f(H) nach Glg. (1) der entsprechende Wert SAR_fe(i) berechnet und aus diesem (und aus der anfangs bestimmten Eisenmasse m_fe) der absolute Mittelwert der Volumen-SAR, SAR_aver(i), abgeschätzt.

Damit kann für jede Schleifenwiederholung ein Skalierungsfaktor K(i) erfindungsgemäß bestimmt werden aus (wir sprechen im MODUS=3 erfindungsgemäß vom "leistungsabsorptionsbasierten" Skalierungsfaktor):

$$K(i) = SAR\_aver(i) \, / \, \Delta SAR\_aver. \qquad\qquad (10)$$

Mit dem leistungsabsorptionsbasierten Skalierungsfaktor K(i) ergibt sich für jedes i eine absolute Temperaturverteilung:

$$T(x,y,z,i) = T0(x,y,z) + K(i)*\Delta T(x,y,z), \qquad\qquad (11)$$

die anschließend ausgegeben wird. Damit werden nacheinander 10 absolute (skalierte) Temperaturverteilungen ausgegeben, obwohl nur zwei Durchläufe zur numerischen Lösung der BHTE notwendig waren.

Es wird darauf hingewiesen, dass die beim H-Regler (MODUS=3) vorgenommene leistungsabsorptionsbasierte Skalierung (leistungsabsorptionsbasierter Skalierungsfaktor K gem. Glg. 10) auf der spezifischen Leistungs-Absorptionsrate (SAR) basiert und sich damit grundlegend von der Skalierung unterscheidet, die in der T-Vorgabe (MODUS=1) (grenz-)temperaturbasiert vorgenommen wird (temperaturbasierter Skalierungsfaktor K gem. Glg. 8). Weiterhin wird angemerkt, dass der leistungsabsorptionsbasierte Skalierungsfaktor K beim H-Regler (Glg. 10) nicht einfach durch die Bildung eines Verhältnisses von H-Werten gebildet werden kann, was sich aus der im allgemeinen vorliegenden Nichtlinearität der Kennlinie SAR_fe=f(H) erklärt.

Binäre Eingabedatei CT.raw

Dieser 3-D-Datensatz kann ein reguläres 3D-Gitter repräsentieren, dessen Elementen (Pixeln) CT-Dichtewerte (Hounsfield Units, als "Short"-Zahlen) zugeordnet sind. Der geometrische Bezug zur Position (x,y,z) eines Elementes im CT-Gitter ergibt sich über den x,y,z-Index und die Angaben zur Bounding Box, die zusätzlich an den Simulator übergeben werden. Die x-Koordinate ist die sich am schnellsten ändernde (innere Schleife), die z-Koordinate am langsamsten (äußere Schleife). Der CT-Datensatz muss dem postoperativen Planungs-CT entsprechen und Nanopartikel beinhalten (nach erfolgter Instillation der magnetischen Flüssigkeit). Für den Simulator sind insbesondere die CT-Werte in den Nanopartikel-Pixeln von Interesse. Aus diesen Werten leitet der Simulator die Information über die aktuelle (nach der Instillation im Körper vorliegende) Eisenkonzentration ab, die relevant für die Berechnung der SAR- und/oder der Temperatur ist.

Binäre Eingabedatei LV.raw

Dieser Datensatz (LV steht für "Labeled Volume") kann ebenfalls ein reguläres 3D-Gitter repräsentieren, dessen Elementen kodierte Labels ("Byte"-Zahlen) zugeordnet sind. Der geometrische Bezug zur 3D-Position (x,y,z) eines Elements im LV-Gitter ergibt sich über den Index und die Angaben zur Bounding Box. Die Bounding Box von LV.raw kann identisch mit der von CT.raw sein. Der Datensatz LV.raw wird im vorangegangenen Programm-Hauptschritt "Segmentierung" bspw. auf der Grundlage des Planungs-CTs erzeugt. Mit den Labels werden folgende drei Arten von Regionen beschrieben/kodiert:

- Geometrisch-anatomische Regionen (Außenraum, "Kopf", Tumor): Diese Informationen sind erforderlich, um die Berechnung der Temperaturverteilung auszuführen. Beispielsweise wird im Simulator die thermische Grenzfläche zwischen dem Körper und dem Außenraum modelliert und daher muss die entsprechende Geometrie bekannt sein. Auch wird erwartet, dass der Arzt den Tumor einzeichnet, obwohl der Simulator auch ohne segmentierten Tumor rechnen kann. Es wird unterschieden zwischen Körper(teil)volumen, also etwa "Kopf", Behandlungsvolumen (PTV), also etwa "Tumor + Saum um den Tumor", und Depotvolumen, also ein oder mehrere (viele) Voxel enthaltend Nanopartikel. "Non-PTV" wäre also im Beispiel "Kopf minus Tumor (inkl. Saum)".

- Nanopartikel-Bereiche (Quellvolumina): Geometrische Positionen (markiert als Labels) der im Gewebe angedockten Nanopartikel bzw. Depots der magnetischen Flüssigkeit ("Nanodepots", Depotvolumen). Diese Nanopartikel-Positionen müssen dem Simulator als Input mitgeteilt werden, um die SAR zu bestimmen. Nur an den Positionen der Nanopartikel entsteht die SAR. Die Nanopartikel-Bereiche können sich mit den geometrisch-anatomischen Regionen und den thermischen Grenzbedingungs-Regionen überlappen. Die geometrische Verteilung der Nanopartikel innerhalb des Depotvolumens oder der Depotvolumina ist wichtig für die Berechnungen etwa im T-Solver oder H-Regler.

- Thermische Grenzbedingungs-Regionen: Regionen oder Organe wo bestimmte Temperaturen T_grenz(x,y,z) nicht überschritten werden sollen. Es wird z.B. unterschieden zwischen dem Behandlungsgebiet ("PTV", "Planning Target Volume") und dem Rest des Kopfes im Sinne des gesunden Gewebes ("Non-PTV-Region", d.h. Region außerhalb des Behandlungsgebietes). Die Grenzbedingungs-Regionen können sich mit den geometrisch-anatomischen Regionen und den Nanopartikel-Bereichen überlappen. Als Standard-Option bietet der Segmentierungseditor als PTV den Tumor zzgl. eines Tumorsaumes von 1 cm an.

Die Kodierung des LV.raw wird so realisiert, dass für jedes 8-Bit-Label 6 Bits für die Kodierung der geometrisch-anatomischen Information, und je ein Bit für die Kodierung der Nanopartikel (JA/NEIN) und des PTV (JA/NEIN) verwendet werden.

Nachfolgend wird ein zweites Ausführungsbeispiel eines erfindungsgemäßen Simulationswerkzeugs genauer beschrieben. Ein Programmpaket implementiert einen Temperatur-Simulator (im Folgenden gelegentlich auch nur als "Simulator" bezeichnet), der Teil einer Simulationssoftware ist, die mit dem Ziel der Unterstützung von Krebs-Therapien entwickelt wurde. Wie das vorstehend beschriebene erste Ausführungsbeispiel ist auch das zweite Ausführungsbeispiel für Simulationen im Kopfbereich vorgesehen.

Als Folge der Einbringung der Magnetflüssigkeit in den Tumorbereich ("Instillation" oder "Implantation") befinden sich sog. "Nanopartikel-Depots" bzw. "Nanodepots" in diesem Bereich. Während der Therapie können diese Nanopartikel mittels hoher niederfrequenter externer Magnetfelder aktiviert werden, d.h. der Einfluss des Magnetfeldes kann zu einer lokalen Temperaturerhöhung führen. Auf der Basis der CT-Daten erstellt der Simulator eine Simulation (Vorhersage) der Temperaturverteilung im Kopfbereich in Abhängigkeit von der Magnetfeldstärke des Therapiegerätes (Magnetfeld-Aktivator). Dies geschieht nach der Instillation, aber noch vor der Therapie. Die vom Simulator bereitgestellten Ergebnisse sind weder Voraussetzung für die Durchführbarkeit einer Therapie noch sind sie für deren Durchführung bindend, bspw. in Bezug auf eine bestimmte vom Simulator berechnete Applikations-Feldstärke. Die Therapiedurchführung kann bspw. durch eine während der Therapie durchgeführte Temperaturmessung beeinflusst werden. Diese hat für den Arzt maßgebenderen Charakter als Simulationsergebnisse. Simulationsergebnisse und - maßgebend - Temperaturmessung können dem Arzt Hinweise für eine Beurteilung der Therapie geben.

Ähnlich wie im ersten Ausführungsbeispiel erfüllt der Simulator u.a. folgende Hauptaufgaben:

- Simulation einer dreidimensionalen Temperaturverteilung, wie sie sich durch die Anwendung des Magnetfeldes auf Nanopartikel unter der Annahme eines vereinfachten physikalischen Modells voraussichtlich ergibt;

- Abschätzung einer magnetische Feldstärke (H-Feldstärke) auf der Basis von bestimmten Temperaturvorgaben für das Patientenmodell.

Ähnlich wie im ersten Ausführungsbeispiel ist der Simulator hier kein Teil eines Hauptprogramm-Cores, sondern ist im Sinne einer externen SOUP ("Software of Unknown Provenience") an den Hauptprogramm-Core angebunden. Allerdings erfolgt der Datenaustausch zwischen dem Simulator und dem Hauptprogramm-Core nicht mehr über externe starre Verzeichnis-Schnittstelle (komplettes Ausschreiben und Einlesen der Daten über Festplatte), sondern der Simulator wird als Programmbibliothek angelinkt und daher erfolgt der Datenaustausch via Arbeitsspeicher. Wichtige Änderungen, die sich durch diese andere Softwarestruktur bzw. -architektur ergeben, umfassen:

- Anbindung des Simulators als Programmbibliothek anstelle von Ausführungsprogrammen wie sim_t.exe, sim_h.exe und sim_hr.exe;

- Einführung des "schnellen H-Reglers" anstelle des "H-Reglers";

- Dynamische Speicherplatzverwaltung der FORTRAN-Arrays.

Die Modifizierung des H-Reglers wird im Detail weiter unten beschrieben. Im Folgenden wird zunächst kurz die Anbindung als Programmbibliothek behandelt.

Beim ersten Ausführungsbeispiel umfasste der Simulator drei eigenständige FORTRAN-Ausführungsprogramme "sim_t.exe", "sim_h.exe" und "sim_hr.exe", wobei jedes Programm für einen bestimmten Vorgabe-Modus verantwortlich war (Modi 1, 2 und 3). Entsprechend wurde dem Berechnungstyp "T-Vorgabe" ("T" für "Temperatur"; Programm sim_t.exe) ein Übergabe-Parameter (bzw. Inputwert aus Sicht des Simulators) "MODUS" mit dem Wert MODUS=1 übergeben, der sog. "H-Vorgabe" ("H" steht für "magnetisch", d.h. Vorgabe einer H-Feldstärke; Programm sim_h.exe) MODUS=2 übergeben, und dem sog. "H-Regler" (Mehrere gewünschte Feldstärkewerte, Programm sim_hr.exe) MODUS=3 zugewiesen.

Bei dem hier diskutierten zweiten Ausführungsbeispiel wird die prinzipielle Aufteilung des Programms in drei Modi beibehalten (MODUS=1, MODUS=2 und MODUS=3), wobei der MODUS=3 softwaremäßig modifiziert wurde (vgl. weiter unten). Allerdings besteht der Simulator nicht mehr aus drei eigenständigen exe-Programmen, sondern fungiert als eine Programmbibliothek. Damit erfolgt die Kommunikation bzw. der Datenaustausch zwischen dem Hauptprogramm-Core und dem Simulator nicht mehr über externe Verzeichnisschnittstellen, sondern via Arbeitsspeicher. Vorteile dieser Lösung liegen u.a. darin, dass das Aus- und Einlesen von großen Datenmengen über externe Verzeichnisse (d.h. von bzw. auf eine Festplatte) nicht mehr erforderlich ist und sich so die Programmausführungsgeschwindigkeiten erhöhen und/oder mögliche Fehlerquellen entfallen.

Der als Programmbibliothek implementierte Simulator ist vom Hauptprogramm-Core aus aufrufbar. Das Hauptprogramm der Programmbibliothek bildet eine Schnittstelle zum Hauptprogramm-Core, übernimmt also "SOUP-seitig" die Aufgaben der externen Schnittstelle im ersten Ausführungsbeispiel, welche dort die Bereitstellung von CT.raw, LV.raw gewährleistet hatte.

Alle Hauptprogramme im ersten Ausführungsbeispiel, d.h. sim_t.exe (für MODUS=1, T-Vorgabe), sim_h.exe (für MODUS=2, H-Vorgabe) und sim_hr.exe (für MODUS=3, H-Regler), werden beim zweiten Ausführungsbeispiel als Fortran-(Haupt-)-Unterprogramme bereitgestellt, die nun alle sequentiell (je nach MODUS) vom Hauptprogramm aufgerufen werden. Diese angepassten Haupt-Unterprogramme werden hier als "Haupt-Subroutinen" ("mainsubroutines") bezeichnet. Die Reihenfolge des Aufrufens der Simulator-Modi und Verwaltung der Simulator-Daten wird weiterhin vom Hauptprogramm-Core aus gesteuert bzw. verwaltet.

Es gibt folgende Haupt-Unterprogramme:

- "mainsubroutine_sim_t_voxel_win" (für MODUS=1, T-Vorgabe),
- "mainsubroutine_sim_h_voxel_win" (für MODUS=2, H-Vorgabe), und
- "mainsubroutine_sim_hr_voxel_win" (für MODUS=3, Schneller H-Regler).

Die CT-Daten und die segmentierten Label-Daten werden nicht, so wie beim ersten Ausführungsbeispiel, als binäre Datensätze CT.raw (CT-Datensatz) und LV.raw (Datensatz mit kodierten Labels) vom Hauptprogramm-Core auf die Festplatte geschrieben und dann wieder von der Festplatte als binärer Input für den Simulator eingelesen. Vielmehr werden diese Daten hier als Arrays im Arbeitsspeicher via dem Simulator übergeben.

Eine Text-Datei SimInput.txt (diese stellt den Input für das erste Ausführungsbeispiel des Simulators bereit) wird hier nicht benötigt. Alle Inputparameter werden in Argumentlisten vom Hauptprogramm-Core zum Hauptprogramm des Simulators und weiter zu den drei mainsubroutines gereicht. Dies betrifft u.a. folgende Inputparameter: MODUS (1,2,3), gewünschte H-Feldstärke (H=magnetische Feldstärke, Angabe in KA/m), gewünschte maximale Temperatur im Non-PTV-Gebiet, Molarität und Fitting-Faktoren a,b,c (Verlauf der Kennlinie SAR_fe=f(H)), alle CT-File-Dimensionsparameter (Anzahl der Elemente, Bounding Box, Angaben zum benutzten Koordinatensystem (m, cm, mm), Anzahl der Regionen).

Es werden hier auch keine Output-Temperaturdatensätze vom Simulator auf die Festplatte geschrieben und dann wieder in den Hauptprogramm-Core eingelesen. Stattdessen werden entsprechende Arrays vom Hauptprogramm des Simulators dem Hauptprogramm-Core via Arbeitsspeicher übergeben.

Alle im ersten Ausführungsbeispiel des Simulationsprogramms berechneten und anschließend in die Ausgabedatei SimOutput.txt geschriebenen Outputparameter werden hier vom Simulationsprogramm via Argumentlisten von den mainsubroutines zum Hauptprogramm des Simulators und weiter zum Hauptprogramm-Core via Arbeitsspeicher übergeben. Die wichtigsten Outputparameter sind: MODUS (1,2,3), resultierende H-Feldstärke, resultierende maximale Temperatur im Non-PTV-Gebiet, resultierende maximale Temperatur im PTV-Gebiet, prozentueller Anteil von Tumor mit resultierenden Temperaturen > 39 Grad, Nanopartikel-Volumen, Tumor-Volumen, resultierende mittlere Volumen-SAR, resultierender mittlerer Hounsfield-Unit-Wert im Nanopartikel-Volumen, mittlere Eisenkern-SAR.

Die folgenden zusätzlichen Parameter werden in den Argumentlisten der mainsubroutines dem Hauptprogramm des Simulators übergeben: Fehlermeldungen, und Parameter, welche die Reihenfolge der Aufrufe der mainsubroutines überwachen (MODUS=3 darf nicht vor MODUS=1 aufgerufen werden, s.u.).

<u>Überblick über die Abläufe im zweiten Ausführungsbeispiel</u>

Der Simulator bietet dem Anwender bzw. Benutzer zwei Optionen zur Ermittlung der absoluten Temperaturverteilung an:

- über die H-Vorgabe: Vorgegeben wird der absolute Wert der H-Feldstärke (in KA/m); gesucht wird die Temperaturverteilung $T(x,y,z)$ (in °C); und
- über die T-Vorgabe: Vorgegeben werden Temperaturgrenzwerte $T\_grenz$ (in °C), gesucht wird der Feldstärkewert H (in kA/m) und die dazugehörige Temperaturverteilung $T(x,y,z)$ (in °C).

Die T-Vorgabe (MODUS=1) wird über den Aufruf des Haupt-Unterprogramms mainsubroutine_sim_t_voxel_win realisiert. Die H-Vorgabe (MODUS=2) erfordert den Aufruf des anderen Haupt-Unterprogramms mainsubroutine_sim_h_voxel_win. Der schnelle H-Regler (MODUS=3), der im Prinzip auch eine H-Vorgabe darstellt, wird über den Aufruf des Haupt-Unterprogramms mainsubroutine_sim_hr_voxel_win realisiert, erfordert jedoch zuvor mindestens einen Aufruf von mainsubroutine_sim_t voxel_win, da er Output von mainsubroutine_sim_t_voxel_win als Input verwendet.

Die zeitliche Abfolge der Aufrufe der Simulator-Programme wird vom Hauptprogramm-Core aus gesteuert bzw. verwaltet und erfolgt nach einem festen Schema, welches in Fig. 6 dargestellt ist.

Es ist zwingend festgelegt, dass nach einem Umschalten vom GUI-Segmentierungseditor zum GUI-Temperatursimulationseditor als erstes immer der MODUS=1 automatisch gestartet wird, d.h. das T-Vorgabe-Unterprogramm mainsubroutine_sim_t_voxel_win, d.h. ohne das hierzu eine Eingabe des Nutzers bzw. Anwenders erforderlich wäre oder nötig ist. Dieses Programm erfüllt zwei feste Grenztemperatur-Vorgaben:

- maximal 43°C außerhalb des PTV ("Non-PTV-43°C-Limit"), und
- maximal 80°C überall sonst, d.h. de facto innerhalb des PTV ("Whole-Body-80°C-Limit").

Zum Non-PTV-43°C-Limit wird angemerkt, dass dieser Wert als Schwelltemperatur angesehen wird, oberhalb dessen Schädigungen des gesunden Gewebes vermehrt auftreten können. Das Whole-Body-Limit von 80°C könnte auch anders gewählt werden, bspw. könnte ein Wert im Bereich zwischen 50°C und unter 100°C, bevorzugt zwischen 60°C und 90°C gewählt werden. Von den beiden Grenzbedingungen wird diejenige wirksam, die bei niedrigerer H-Feldstärke eintritt.

Nach dem Durchlaufen des ersten, d.h. automatischen, Laufs im MODUS=1 untersucht der Hauptprogramm-Core intern (ohne dass eine Ausgabe per GUI erfolgt), wie hoch der intern als Output aus dem Hauptprogramm der Simulator-Programmbibliothek ausgegebene H-Feldstärkewert ist. Ist dieser Wert größer als 15 kA/m, so startet wiederum automatisch ein Durchlauf im MODUS=2 mit der H-Vorgabe 15 kA/m, bzw. allgemein dem physikalisch maximal einstellbaren H-Feldstärkewert am Magnetfeldaktivator. Ist der Outputwert von MODUS=1 kleiner oder gleich 15 kA/m, muss kein Programmlauf im MODUS=2 gestartet werden und es folgt direkt der nächste Schritt.

Nach dem Beenden der automatischen Läufe wie oben beschrieben erfolgt die Ausgabe folgender Daten an die GUI:

- Temperaturverteilung;
- H-Feldstärke-Empfehlung, d.h.

  o der Output von MODUS=1 wenn H<15kA/m, oder
  o der Wert 15 kA/m (Begrenzungslauf im MODUS=2 wurde durchgeführt),

- erreichte Maximaltemperatur außerhalb von PTV, erreichte Maximaltemperatur im ganzen Berechnungsgebiet, und weitere Größen.

Es kann Fälle geben, in denen die Maximaltemperatur außerhalb des PTV kleiner als die maximal zulässige Temperatur (z.B. 43°C) ist, und zwar dann, wenn die Grenzbedingung von z.B. 80°C im PTV erreicht worden ist.

Nach der Betrachtung dieses Initialergebnisses (des Ergebnisses der automatischen Läufe) am Monitor entscheidet der Benutzer, ob er mit dem Ergebnis zufrieden ist. Ist dies nicht der Fall, so kann er einen beliebigen H-Feldstärkewert eintippen und für diesen Wert eine schnelle Berechnung der Temperaturverteilung im MODUS=3 (schneller H-Regler) so oft wie gewünscht starten.

Der schnelle H-Regler hat keinerlei Begrenzungen hinsichtlich der erreichten Temperaturen, anders als die T-Vorgabe im Initiallauf. Daher kann es Fälle geben, bei denen die erreichten Temperaturen höher (oder niedriger) sind als 80°C im Berechnungsgebiet und/oder höher (und/oder niedriger) als 43°C außerhalb von PTV.

Der Nutzer kann jederzeit zum Programm-Hauptschritt "Segmentierung" zurückkehren, um Segmentierungskorrekturen wie etwa Korrekturen am PTV vorzunehmen. In diesem Fall wechselt der Simulator in den Ausgangszustand. Sobald als Output der Segmentierung neue LV-Daten vorliegen und der User vom Programm-Hauptschritt "Segmentierung" in den Temperaturberechnungseditor wechselt, wiederholt sich die oben beschriebene Prozedur, d.h. der Simulator startet mit dem Initiallauf "T-Vorgabe", etc. Der Initiallauf im MODUS=1 kann aber auch ohne Rückkehr zum Segmentierungseditor mit Hilfe eines GUI-Buttons "Automatische Temperatursimulation neu starten" ausgelöst werden. Auch in diesem Falle wiederholt sich die Prozedur, bei welcher der Simulator mit dem Initiallauf "T-Vorgabe" startet, etc.

Nachfolgend werden die drei Simulator-Modi nach ihren Zielen und Funktionen beschrieben.

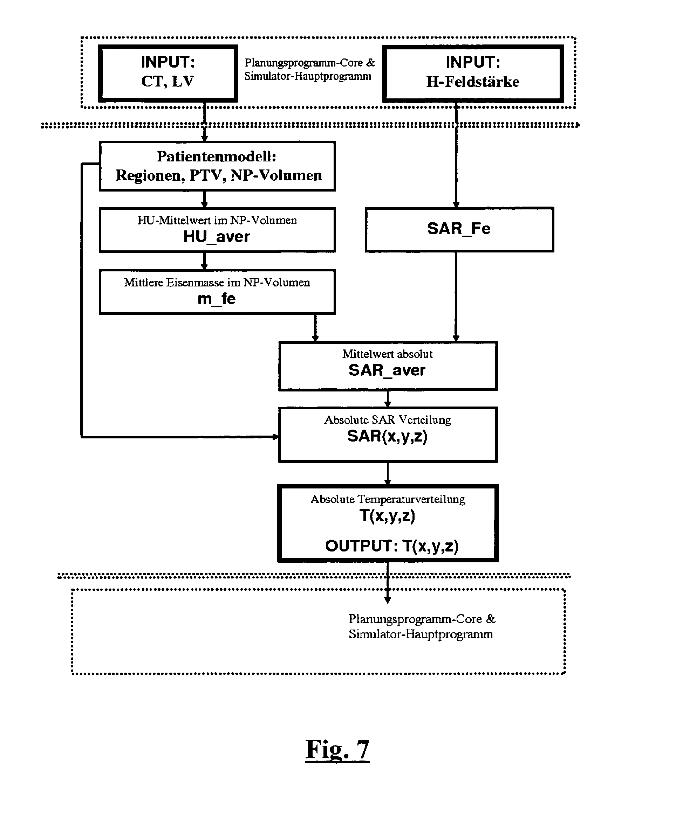

MODUS=2 (H-Vorgabe, aufgerufen wird mainsubroutine sim h voxel win) Vorgegeben wird der absolute Wert der H-Feldstärke. Gesucht wird die Temperaturverteilung T(x,y,z), vgl. den in Fig. 7 schematisch dargestellten Ablauf. Zunächst wird das Patientenmodell generiert. Dieses basiert auf der Kombination von zwei Datensätzen (Arrays):

- CT-Datensatz (Array), der vom Simulationsprogramm als Teil einzulesender Patientendaten eingelesen wird (vgl. hierzu Fig.3; in diesem grundsätzlichem Ablauf sind sich erstes und zweites Ausführungsbeispiel sehr ähnlich);
- LV-Datensatz (Array) ("LV" steht für "Labeled Volume"), d.h. ein kodiertes Label-Array, welches vom Simulationsprogramm während der Segmentierung erzeugt wird (vgl.Fig.3).

Als Basis für das Patientenmodell dienen die LV-Elemente ("Labels"), in denen voxelbasiert folgende Informationen kodiert sind:

- die geometrisch-anatomischen Regionen ("Kopf", "Tumor", etc.);
- die thermisch relevanten Regionen (Behandlungsgebiet, auch als PTV, "Planning Target Volume" bezeichnet);
- geometrische Verteilung der Nanopartikel (NP).

Die Auswertung der NP-Verteilung führt zur Ermittlung des NP-Volumens V_NP. Nun erfolgt der Vergleich mit dem CT-Datensatz, wobei der mittlere Hounsfield Unit(HU)-Wert "HU_aver" über die Mittelwertbildung derjenigen Werte von HU(x,y,z) bestimmt wird, die in das V_NP fallen.

Als nächstes wird aus dem Mittelwert HU_aver die mittlere Eisenkonzentration und daraus die mittlere Eisenmasse m_fe im V_NP abgeschätzt.

Unabhängig, z.B. parallel zu diesen Schritten leitet der Simulator aus der H-Feldstärke die Eisenkern-SAR ab ("SAR_fe"). Dies geschieht über Anwendung einer experimentell für die verwendete Magnetflüssigkeit bestimmten, im allgemeinen nicht-linearen Kennlinie SAR_fe = f(H). Dabei wird angenommen, dass sich das Behandlungsgebiet zentral zwischen den Polschuhen des Magnetfeld-Applikators befindet, wo in guter Näherung dieselbe (maximale, konstante) H-Feldstärke vorliegt. Um eine aufwendige tabellarische Darstellung zu vermeiden, kann die Kennlinie SAR_fe=f(H) durch drei Fitting-Faktoren a, b, c, etwa wie folgt approximiert werden:

$$SAR\_fe = a\,H^b + c \qquad\qquad (12)$$

mit SAR_fe in W/g (Watt pro Gramm) und H in kA/m (Kiloampere pro Meter).

Mit m_fe, SAR_fe und V_NP wird nun der Mittelwert "SAR_aver" der Volumen-SAR(x,y,z) im Nanodepot abgeschätzt, vgl. etwa Gleichung (2) in Gneveckow et al. 2004. Anschließend erfolgt die Bildung der ortsabhängigen Volumen-SAR-Verteilung, wobei angenommen wird, dass in V_NP die SAR-Werte zu den HU-Werten proportional sind, d.h.

$$SAR(x,y,z) = HU(x,y,z)*SAR\_aver/HU\_aver \ \ in \ V\_NP \qquad\qquad (13)$$

$$SAR(x,y,z)=0 \text{ außerhalb von V\_NP.} \qquad (14)$$

Alle Werte HU(x,y,z) sollen in V_NP positiv sein, damit keine physikalisch unmöglichen negativen SAR-Werte auftreten. Dies kann etwa bereits im Segmentierungseditor durch eine entsprechende Filterung bzw. Threshold-Setzung gewährleistet werden und ggf. im Simulator noch einmal kontrolliert werden.

Mit ortsabhängiger SAR(x,y,z) als Quelle der Temperatur wird dann die BHTE T(x,y,z)=f(SAR(x,y,z)) numerisch gelöst, vgl. Nadobny et al. 2007, Glg. (1)-(2). Zur Lösung kann ein Finite-Differenz-Verfahren mit expliziter Temperaturgradienten-Berechnung verwendet werden, wie es etwa in Nadobny et al. 2007, Glg. (8)-(15) beschrieben wird.

Anders als im MODUS=1 und MODUS=3 wird bei MODUS=2 die das Modell beschreibende BHTE für absolute Werte der SAR gelöst, die sich aus dem absoluten H-Vorgabe-Wert ableiten. Die H-Vorgabe-Option im MODUS=2 benötigt daher - anders als in MODUS=1 und MODUS=3, lediglich einen einzigen Durchlauf zur numerischen Lösung der BHTE T(x,y,z)=f(SAR(x,y,z).

Bei der H-Vorgabe wird die Temperatur nicht begrenzt, d.h. je nach H-Feldstärke können sich beliebige Temperaturen im Körper einstellen, die auch über den üblichen Temperaturgrenzwerten (z.B. 43°C im gesunden Gewebe) liegen.

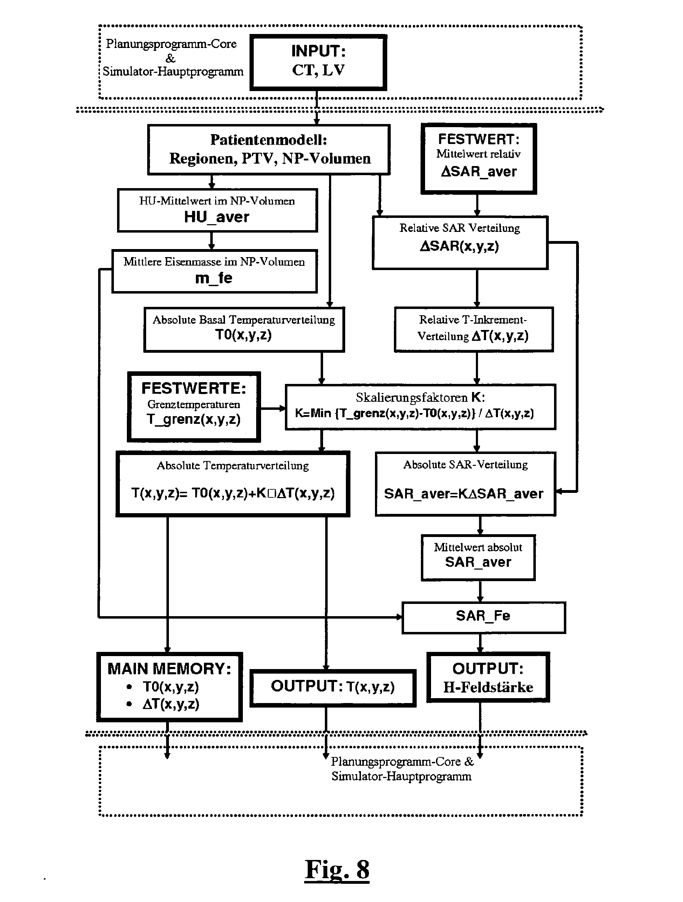

MODUS=1 (T-Vorgabe, aufgerufen wird: mainsubroutine sim t voxel win) Vorgegeben werden Temperaturgrenzwerte T_grenz(x,y,z), gesucht wird der Feldstärkewert H und die dazugehörige Temperaturverteilung T(x,y,z), vgl. die schematische Darstellung in Fig. 8. Im Unterschied zur T-Vorgabe im ersten Ausführungsbeispiel werden für den späteren Aufruf von MODUS=3 (schneller H-Regler) bestimmte Datensätze ausgegeben und im Arbeitsspeicher temporär bereitgestellt. Zunächst wird - wie im MODUS=2 - aus den LV- und CT-Daten das Patientenmodell erstellt und anschließend werden HU_aver und m_fe für das NP-Volumen bestimmt (diese Größen sind unabhängig von angelegter H-Feldstärke). Der weitere Ablauf im MODUS=1 ist jedoch anders als bei MODUS=2, da der absolute Wert der Feldstärke nicht vorgegeben, sondern gesucht ist. Zu einer Prozedur zur Ermittlung einer entsprechenden Volumen-SAR, die zu der vorgegebenen Grenztemperatur korrespondiert, kommt bei der Anwendung mit Nanopartikeln noch ein zusätzlicher Schritt hinzu, nämlich die Berechnung der H-Feldstärke aus der Volumen-SAR.

Die gleichzeitig und gleichberechtigt zu erfüllenden Grenztemperatur-Vorgaben T_grenz(x,y,z) sind:

- Die "Non-PTV"- Region entspricht in etwa dem gesunden bzw. nicht zu behandelndem Gewebe. Für diese Non-PTV-Region, bei der es sich im Booleschen Sinne um "Körper minus PTV" bzw. "Kopf minus PTV" handelt, wird ein maximal zulässiger Temperaturwert (=Temperaturgrenzwert) T_grenz(Non-PTV) vorgegeben. Dieser Wert kann durch den Anwender veränderbar sein, oder aber kann fest vorgegeben sein. Der Wert kann default-mäßig auf 43°C gesetzt sein, d.h. T_grenz(Non-PTV)=43°C.

- Die Temperatur im Körper soll nirgendwo einen Maximalwert von bspw. 80°C überschreiten. Dieser Wert kann bspw. im Simulator fest vorgegeben sein. Da gleichzeitig im Non-PTV höchstens 43°C herrschen sollen, wirkt die zweite Begrenzung für das Behandlungsgebiet PTV, d.h. T_grenz(PTV)=80°C.

Die resultierende Feldstärke H muss beide Vorgaben erfüllen, d.h. sie soll so niedrig sein, dass alle Temperaturen T(x,y,z) kleiner oder gleich 80°C im PTV sind, und gleichzeitig kleiner oder gleich 43°C außerhalb von PTV sind. Von den beiden Feldstärkewerten, die jeweils eine der beiden T-Vorgaben erfüllen, wird der niedrigere ausgegeben.

Zur Erfüllung dieser T-Vorgaben könnte MODUS=2 mehrere Male gestartet werden, wobei die Input-Feldstärke immer wieder nachgestellt würde, so dass am Ende eines solchen iterativen Prozesses die T-Vorgaben erfüllt wären. Dieser iterative und entsprechend ungenaue bzw. aufwendige Weg (vgl. "iterive way" in Nadobny et al. 2007, S.1841) wird hier nicht verfolgt.

Erfindungsgemäß wird hier anders vorgegangen, um den H-Feldstärkewert direkt, ressourcenschonend und dennoch genau zu bestimmen. Dabei ist zu beachten, dass die Problemstellung keineswegs trivial ist, da die SAR (und damit Temperatur) von der H-Feldstärke auf nicht-lineare Weise abhängt. Jedoch lässt sich zumindest für einen Teil der Problemstellung ein linearer Zusammenhang angeben, und zwar zwischen der SAR und der Temperatur (vgl. "decomposition way" in Nadobny et al. 2007, S.1841, Eqs. (5a),(5b),(6)). So wird die Temperaturverteilung T(x,y,z) zunächst in einen basalen Anteil T0(x,y,z) (wie sie sich ohne SAR ergeben würde) und in einen Temperaturanstiegs-Anteil T_anstieg(x,y,z)=K*ΔT(x,y,z) aufgeteilt wird, mit:

$$T(x,y,z)=T0(x,y,z)+K*\Delta T(x,y,z), \qquad (15)$$

wobei für die SAR gilt

$$SAR(x,y,z)= K*\Delta SAR(x,y,z). \qquad (16)$$

K ist ein - zu ermittelnder - skalarer Skalierungsfaktor (wir sprechen im MODUS=1 vom "temperaturbasierten" Skalierungsfaktor), $\Delta T(x,y,z)$ ist das relative Temperaturinkrement, und $\Delta SAR(x,y,z)$ ist die relative SAR Verteilung.

Im Unterschied zum ersten Ausführungsbeispiel umfasst der Output von mainsubroutine_sim_t_voxel_win nicht nur die eigentliche Temperaturverteilung $T(x,y,z)$, sondern auch die Verteilungen $T0(x,y,z)$ und $\Delta T(x,y,z)$. Diese Temperaturverteilungen sind im Detail wie folgt charakterisiert:

- $T(x,y,z)$: Absolute resultierende Temperaturverteilung, die zu einer bestimmten absoluten SAR korrespondiert, welche notwendig ist, um bestimmte Randbedingungen zu erfüllen, z.B. im MODUS=1 stellen die Grenz-Temperaturen solche Randbedingungen dar. $T(x,y,z)$ wird auch im Simulationsprogramm gemäß dem ersten Ausführungsbeispiel ausgegeben. $T(x,y,z)$ gehört zu den Daten, die in der GUI ("Graphical User Interface") des Simulationsprogramms visualisiert werden.
- $T0(x,y,z)$: Absolute "basale" Temperaturverteilung, wie sie sich ohne SAR ergibt; Anfangstemperatur und basale Temperatur zur numerischen Lösung der zeitabhängigen BHTE sind identisch wie bei der Lösung nach $T(x,y,z)$. Diese Temperaturverteilung wird im Arbeitsspeicher temporär abgelegt und liegt dann als Input für den schnellen H-Regler vor.
- $\Delta T(x,y,z)$: Die Verteilung des relativen Temperaturanstiegs (=Temperaturinkrements), die sich für einen willkürlichen, fest vorgegebenen und/oder durch den Nutzer bzw. Anwender definierten SAR-Level ("Festwert" in Fig. 8) ergibt. Da hier nicht die Temperatur, sondern das Temperaturinkrement simuliert wird, sind Anfangstemperatur und basale Temperatur zur numerischen Lösung der zeitabhängigen BHTE gleich Null. Diese Temperaturverteilung wird ebenfalls temporär im Arbeitsspeicher abgelegt und liegt dann als Input für den schnellen H-Regler vor.

Anders als im MODUS=2 wird kein absoluter Wert SAR_aver aus SAR_fe bestimmt, sondern es wird ein beliebiger (relativer) Test-Mittelwert $\Delta SAR\_aver$" für die relative Volumen-SAR $\Delta SAR(x,y,z)$ intern im Sinne eines "Festwertes" (Konstante, vgl. Fig.8) vorgegeben (im Simulator ist dieser Test-Mittelwert als "$\Delta SAR\_aver=100$ W/kg gesetzt). Ähnlich wie in Glg. (13) werden daraus die ortsabhängigen $\Delta SAR(x,y,z)$-Werte wie folgt approximiert:

$$\Delta SAR(x,y,z)=HU(x,y,z)* \Delta SAR\_aver/HU\_aver \quad \text{in V\_NP}, \qquad (17)$$

$$\Delta SAR(x,y,z) \quad \text{außerhalb von V\_NP.} \qquad (18)$$

Als Nächstes werden zwei Durchläufe zur numerischen Lösung der BHTE nacheinander gestartet: einmal für $T0(x,y,z)=f(SAR=0)$ und einmal für das relative Temperaturinkrement $\Delta T(x,y,z)=f(\Delta SAR(x,y,z))$. Anschließend wird der Skalierungsfaktor mit Hilfe einer Minimumsuche über alle Stützpunkte (Voxel) x,y,z gefunden ("temperaturbasierter Skalierungsfaktor"):

$$K=Min(T\_grenz(x,y,z)-T0(x,y,z))/\Delta T(x,y,z) \qquad (19)$$

mit T_grenz(x,y,z)=80°C in Non_PTV und 43°C in PTV.

Nachdem der temperaturbasierte Skalierungsfaktor K gefunden wurde, können sofort die absolute SAR(x,y,z) und $T(x,y,z)$ mit Hilfe der Gln. (16) und (15) angegeben werden, ohne dass noch einmal die BHTE numerisch gelöst werden muss. Für den Mittelwert von SAR(x,y,z) gilt ebenfalls:

$$SAR\_aver=K*\Delta SAR\_aver. \qquad (20)$$

Anschließend werden die Anfangsschritte aus MODUS=2 in inverser Reihenfolge durchgeführt: i) es wird aus dem Mittelwert SAR_aver und der zuvor bestimmten Eisenmasse m_fe die Eisenkern-SAR SAR_fe bestimmt; ii) es wird die nicht-lineare Kennlinie SAR_fe=f(H) (vgl. Gneveckow et al. 2004, Fig.5) im inversen Sinne angewendet, d.h. aus dem Wert SAR_fe wird der H-Feldstärkewert ermittelt. Dieser wird als Output von mainsubroutine_sim_t_voxel_win zum Hauptprogramm der Programmbibliothek und weiter zum Hauptprogramm-Core bzw. an die GUI übergeben. Zwar bilden die drei Datensätze $T(x,y,z)$, $T0(x,y,z)$ und $\Delta T(x,y,z)$ den Output von mainsubroutine_sim_t_voxel_win. Allerdings wird nur T(x,y,z) zur GUI weitergereicht. T0(x,y,z) und ΔT(x,y,z) werden im Arbeitsspeicher temporär abgelegt, um anschließend als Input von mainsubroutine_sim_hr_voxel_win im MODUS=3 bereitgestellt zu werden.

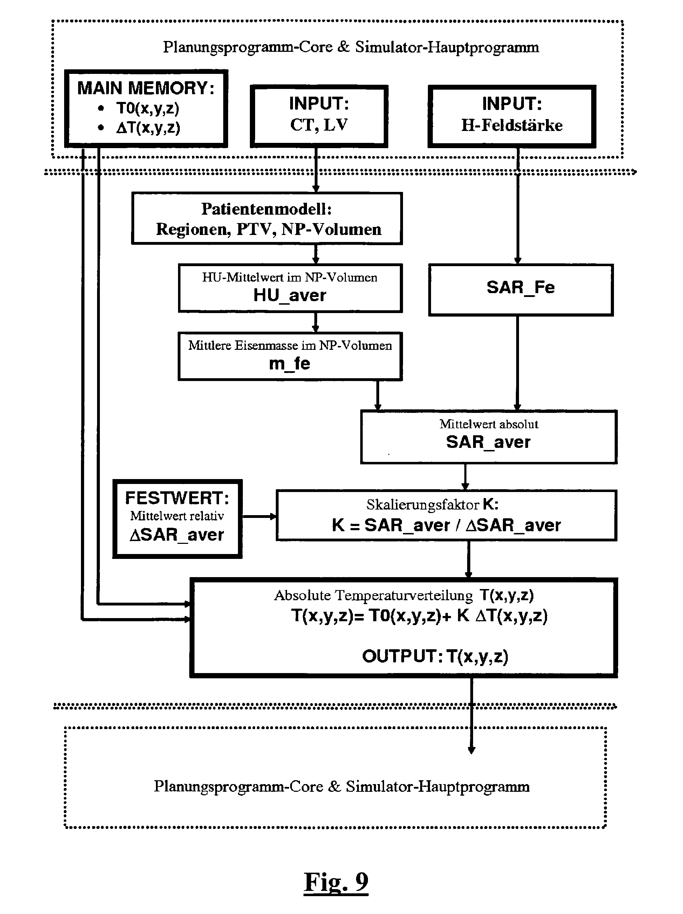

<u>MODUS=3 ("Schneller H-Regler", aufgerufen wird mainsubroutine sim hr voxel win)</u> Im MODUS=3 wird sekundenschnell eine Temperaturverteilung für beliebige H-Vorgabe ermittelt, ohne dass die BHTE bspw. mit einem Finite-Differenzen-Programm gelöst werden muss. Die Voraussetzung ist das Einlesen von T0(x,y,z) und ΔT(x,y,z) als Input von mainsubroutine_sim_hr_voxel_win. Diese Temperaturdatensätze sollten bereits im Arbeitsspeicher vorhanden sein, d.h. dass für ein gegebenes Patientenmodell z.B. schon mindestens einmal MODUS=1 erfolgreich aufgerufen und beendet wurde, vgl. Fig. 9.

Zunächst wird - wie im MODUS=1 bzw. MODUS=2 - aus den LV- und CT-Daten das Patientenmodell erstellt. Anschließend werden HU_aver und m_fe für das NP-Volumen bestimmt. Diese Größen sind unabhängig von angelegter H-Feldstärke. Die mittlere Eisenmasse m_fe wurde bereits in MODUS=1 (T-Vorgabe) berechnet. Alternativ zur Neuberechnung ist es auch denkbar, dass die mittlere Eisenmasse m_fe nach der Berechnung im MODUS=1 (T-Vorgabe) im Arbeitsspeicher für den Zugriff durch MODUS=3 (schneller H-Regler) gehalten wird. Nachdem das zugrundeliegende Patientenmodell unabhängig vom MODUS stets dasselbe ist, könnte eine Berechnung wie diejenige für die mittlere Eisenmasse m_fe auch ganz ausgelagert werden, etwa in den Segmentierungsschritt (vgl. Fig. 1). Die lokale Berechnung derartiger Größen kann allerdings vorteilhaft sein etwa in Bezug auf Aspekte wie eine modulare Struktur des Programmpakets, Durchführung von Softwaretests, etc.

Unabhängig von der Berechnung der mittleren Eisenmasse wird - ähnlich wie bei MODUS=2 - ein vom User in der GUI eingegebener Feldstärkewert H eingelesen und über die Anwendung der Kennlinie SAR_fe=f(H) der entsprechende Wert der Eisenkern-SAR SAR_fe berechnet. Aus diesem Wert und aus der zuvor bestimmten Eisenmasse m_fe, wird dann der absolute Mittelwert der Volumen-SAR, SAR_aver, ermittelt. Gleichzeitig erfolgt - wie im MODUS=1 - die Vorgabe eines relativen Festwertes von ΔSAR_aver=100 W/kg, wobei der Festwert ΔSAR_aver in MODUS=3 identisch mit dem Festwert ΔSAR_aver in MODUS=1 sein muss.

Damit kann erfindungsgemäß der Skalierungsfaktor K bestimmt werden aus (wir sprechen im MODUS=3 erfindungsgemäß vom "leistungsabsorptionsbasierten" Skalierungsfaktor):

$$K = SAR\_aver \: / \: \Delta SAR\_aver. \qquad\qquad (21)$$

Mit dem berechneten leistungsabsorptionsbasierten Skalierungsfaktor K und mit den zuvor in MODUS=1 berechneten und im Arbeitsspeicher vorhandenen T0(x,y,z) und ΔT(x,y,z) ergibt sich durch die Anwendung der Glg. (15) die gesuchte Temperaturverteilung T(x,y,z), die anschließend an die GUI ausgegeben wird. Da beim schnellen H-Regler keine BHTE gelöst werden muss, läuft das oben beschriebene Verfahren mit einem üblichen Prozessor sekundenschnell ab.

Es wird darauf hingewiesen, dass die beim schnellen H-Regler (MODUS=3) vorgenommene leistungsabsorptionsbasierte Skalierung (leistungsabsorptionsbasierter Skalierungsfaktor K gem. Glg. 21) auf der spezifischen Leistungs-Absorptionsrate (SAR) basiert und sich damit grundlegend von der Skalierung unterscheidet, die in der T-Vorgabe (MODUS=1) (grenz-)temperaturbasiert vorgenommen wird (temperaturbasierter Skalierungsfaktor K gem. Glg. 19). Weiterhin wird angemerkt, dass der leistungsabsorptionsbasierte Skalierungsfaktor K beim schnellen H-Regler (Glg. 21) nicht einfach durch die Bildung eines Verhältnisses von H-Werten gebildet werden kann, was sich aus der im allgemeinen vorliegenden Nichtlinearität der Kennlinie SAR_fe=f(H) erklärt.

Bei dem hier beschriebenen zweiten Ausführungsbeispiel werden die basale Temperaturverteilung und die relative Temperaturinkrementverteilung im MODUS=1 (T-Vorgabe) berechnet und im Arbeitsspeicher als Basis für den u.U. nachfolgend aufgerufenen schnellen H-Regler (MODUS=3) bereitgestellt. Andere Ausführungsbeispiele sind ebenso denkbar. Zum Beispiel könnten die beiden Temperaturverteilungen (erst) beim ersten Aufruf des schnellen H-Reglers berechnet und im Arbeitsspeicher bereitgestellt werden. In diesem Falle würde die Reaktion des Simulators auf den ersten Aufruf des schnellen H-Reglers langsamer erfolgen, während auf die nachfolgenden Aufrufe des schnellen H-Reglers dann sehr schnell, d.h. praktisch ohne Wartezeiten, mit der Ausgabe der aus der eingegebenen H-Feldstärke resultierenden Temperaturverteilung reagiert würde.

Das hier beschriebene zweite Ausführungsbeispiel verfügt über zwei Programmkomponenten zur Berechnung einer konkreten Temperaturverteilung basierend auf einem vorgegebenem oder einem anwenderdefiniertem H-Feldstärkewert, nämlich einmal der H-Vorgabe (MODUS=2) und außerdem dem (schnellen) H-Regler. Bei einem anderen Ausführungsbeispiel könnte auch nur der (schnelle) H-Regler vorgesehen sein. Es wird allerdings angemerkt, dass die H-Vorgabe eine konkrete Temperaturverteilung unabhängig von einer basalen Temperaturverteilung und einer relativen Temperaturinkrementverteilung berechnet. Das Vorliegen zweier unabhängiger Berechnungswege in einem Programmpaket kann etwa für Testzwecke wie auch zur Pflege und Weiterentwicklung vorteilhaft sein.

Auch kann einer der beiden Berechnungswege in einem konkreten Umfeld, z.B. Hardwareumgebung, oder je nach konkreten Anforderungen besser geeignet sein. So kann bei einem bestimmten Ausführungsbeispiel etwa der Simulator konfigurierbar gestaltet sein. Ein erfahrener Bediener würde den Simulator z.B. so einstellen, dass im MODUS=1 (T-Vorgabe) basale Temperaturverteilung und relative Temperaturinkrementverteilung nicht herausgeschrieben werden, um so Arbeitsspeicher zu sparen. Der Bediener favorisiert aus seiner Erfahrung und der Ausgabe aus der T-Vorgabe (MODUS=1) ohnehin bereits einen bestimmten H-Wert, so dass die resultierende, endgültige Temperaturverteilung durch einmaligen Aufruf der H-Vorgabe berechnet werden kann. Nachdem hierbei der Arbeitsspeicherbedarf begrenzt ist, eignet sich eine derartige Konfiguration unter Umständen auch für eine Hardwarekonfiguration mit entsprechend begrenzten Ressourcen. Bei einer nochmals anderen Variante wird auf den automatischen Start der T-Vorgabe (MODUS=1) verzichtet. Vielmehr erwartet der Simulator eine Benutzereingabe. Umfasst diese ein entsprechendes Kommando, kann die Berechnung der T-Vorgabe beginnen, z.B. ausgehend von den vorgegebenen Temperaturgrenzwerten. Umfasst die Benutzereingabe einen H-Feldstärkewert, kann die H-Vorgabe gestartet werden, oder der schnelle H-Regler (dies kann wiederum von den verfügbaren Hardwareressourcen abhängig sein). Stehen letzterem basale Temperaturverteilung und relative Temperaturinkrementverteilung noch nicht zur Verfügung, müssten diese beim ersten Aufruf erzeugt werden.

Schnittstellen zwischen Temperatur-Simulator und Hauptprogramm-Core

Im ersten Ausführungsbeispiel wurden die Inputwerte für den Simulator über eine externe Text-Verzeichnisschnittstelle von Hauptprogramm-Core aus dem Simulator übergeben. Hierzu wurde ein Textfile SimInput.txt gebildet, der eine bestimmte festdefinierte zeilenbezogene Struktur aufwies. Die Inputwerte wurden bei Aufruf der exe-Programme aus SimInput.txt gelesen. Im hier (im zweiten Ausführungsbeispiel) beschriebenen Simulator existiert SimInput.txt nicht mehr und die Datenübergabe erfolgt über eine interne Schnittstelle zwischen dem Hauptprogramm-Core und der Simulator-Programmbibliothek via Arbeitsspeicher, d.h. die Datenübergabe erfolgt über eine interne Programmschnittstelle.

Simulatorseitig wird diese Schnittstelle über Argumentlisten realisiert. Der Simulator-Input wird von Hauptprogramm-Core aus generiert (etwa in C++) und an die FORTRAN-Simulationsbibliothek über Argumentlisten übergeben. Die Input-Arrays und Inputparameter umfassen z.B.:

- 1-D Array mit CT-Daten, d.h. HU-Werte;
- 1-D Array mit LV-Daten, d.h. die segmentierten Labels;
- Inputparameter in Argumentlisten, vergleichbar mit denjenigen aus der ehemaligen Textdatei SimInput.txt des ersten Ausführungsbeispiels.

Der Simulator-Output wird in der FORTRAN-Bibliothek generiert und an den Hauptprogramm-Core bzw. die GUI über Argumentlisten übergeben. Die Output-Arrays bzw. Parameter umfassen z.B.:

- 1-D-Array mit den Temperaturwerten $T(x,y,z)$; und
- Outputparameter in Argumentlisten, die in der GUI und zur Erstellung eines Therapieplans benötigt werden können (ein Teil dieser Parameter wird im ersten Ausführungsbeispiel in ein Outputfile SimOutput.txt geschrieben).

Die 3D-CT-Daten können in Form dynamischer 1D-FORTRAN-Arrays repräsentiert werden, die im Hauptprogramm-Core im Schritt "Patientendaten" und im Schritt "Segmentierung" eingelesen werden können.

Ein Array kann die 3D-CT-Daten eines regulären 3D-Gitters repräsentieren, dessen Elemente (Pixel) CT-Dichtewerte ("Hounsfield Units"- Werte, d.h. "HU-Werte") zugeordnet sind. Der geometrische Bezug zur Position (x,y,z) eines Elementes im CT-Gitter ergibt sich über den x,y,z-Index und die Angaben zur Bounding Box, die im Arbeitsspeicher vom Hauptprogramm-Core an den Simulator übergeben werden. Der CT-Datensatz muss dem postoperativen Planungs-CT entsprechen und Bildinformationen über die Nanopartikel beinhalten (nach Instillation der magnetischen Flüssigkeit). Für den Simulator sind insbesondere die HU-Werte in den Nanopartikel-Pixeln von Interesse. Aus diesen Werten leitet der Simulator Informationen über die nach der Instillation im Körper vorliegende Eisenkonzentration ab. Diese sind relevant für die Berechnung der SAR und anschließend der Temperatur.

Ein weiteres Array kann kodierte "Labeled Volume" (LV)-Daten (Labels) repräsentieren, die als Folge der Segmentierung auf der Grundlage des Planungs-CTs generiert werden. Im geometrischen Sinne bildet dieses Array ein reguläres 3D-Gitter ab, dessen Elementen kodierte Labels (Zahlen) zugeordnet sind. Der geometrische Bezug zur 3D-Position (x,y,z) eines Elements im LV-Gitter ergibt sich über den Index und die Angaben zur Bounding Box. Die Bounding Box eines solchen LV-Datensatzes bzw. -Gitters ist identisch mit der des CT-Datensatzes. Mit den LV-Daten (Labels) werden folgende drei Kategorien von Regionen beschrieben:

- Geometrisch-anatomische Regionen (Außenraum, "Kopf", "Tumor"): Diese Informationen sind für die Berechnung der Temperaturverteilung erforderlich. Insbesondere wird im Simulator die thermische Grenzfläche zwischen dem Körper und dem Außenraum modelliert und daher muss ihre Geometrie bekannt sein. Auch sollte der Tumor repräsentiert sein, obwohl der Simulator auch ohne segmentierten Tumor rechnen kann. Die "Kopf"-Region bedeutet im präzisen Sinne "Kopf minus Tumor".

- Nanopartikel-Bereiche (Quellvolumina): geometrische Positionen (markiert als Labels) der im Gewebe angedockten Nanopartikel ("Nanodepots"). Diese Nanopartikel-Positionen müssen dem Simulator als Input für die Bestimmung der SAR mitgeteilt werden. Nur an den Positionen der Nanopartikel entsteht die SAR. Die Nanopartikel-Bereiche können mit den geometrisch-anatomischen Regionen und den Regionen mit z.B. thermischen Grenzbedingungen überlappen. Beim Nichtvorhandensein segmentierten Nanopartikelregionen kommt es zum Programmabbruch.

- Thermische Grenzbedingungs-Regionen: Regionen (z.B. Organe), in denen bestimmte Temperaturen T_grenz(x,y,z) nicht überschritten werden sollen. Im zweiten Ausführungsbeispiel wird lediglich zwischen dem Behandlungsgebiet (PTV) und dem Rest des Kopfes (Non-PTV-Region) unterschieden, d.h. der Region außerhalb des Behandlungsgebietes, mithin dem gesunden Gewebe. Die Grenzbedingungs-Regionen können sich mit den geometrisch-anatomischen Regionen und den Nanopartikel-Bereichen überlappen. Als Standard-Option kann im Segmentierungseditor die PTV-Region als Tumor zzgl. eines Tumorsaumes von gewünschter Breite erzeugt werden. Beim Nichtvorhandensein der segmentierten PTV-Region kommt es zum Programmabbruch.

Die Kodierung der LV-Daten kann im Hauptschritt "Segmentierung" realisiert werden, indem für jedes Element eines 8-Bit-Labels 6 Bit für die Kodierung der geometrischanatomischen Information, und je ein Bit für die Kodierung der Nanopartikel (JA/NEIN) und des PTV (JA/NEIN) verwendet werden.

Zu den Unterschieden zwischen dem ersten und zweiten Ausführungsbeispiel

Das anhand der Figuren 2 - 5 beschriebene erste Ausführungsbeispiel unterscheidet sich unter anderen in den folgenden Aspekten vom anhand der Figuren 6 - 9 beschriebenen zweiten Ausführungsbeispiel:

- Anbindung des Temperatur-Simulators als Programmbibliothek ("SOUP Integration");
- Andere Programmierung / Implementierung des H-Reglers bzw. schnellen H-Reglers.

Beim ersten Ausführungsbeispiel ist der Temperatur-Simulator in Form von getrennten / autarken Ausführungsprogrammen implementiert. Beim zweiten Ausführungsbeispiel ist der Temperatur-Simulator als Programmbibliothek implementiert. Dabei werden die Temperatur-Simulator-Programme nicht als eigenständige exe-Files über eine externe Verzeichnisschnittstelle aufgerufen, sondern diese sind als Methoden einer mit dem Hauptprogramm-Core verknüpften Programmbibliothek über eine interne, vom Hauptprogramm-Core aus direkt ansteuerbare Schnittstelle zu verwalten. Eine der Vorteile dieser Lösung liegt darin, dass das Schreiben bzw. Einlesen von großen Datenmengen über externe Verzeichnisse auf die bzw. von der Festplatte (wie beim ersten Ausführungsbeispiel) nicht erforderlich ist. Somit entfallen die vergleichsweise langsamen Festplatten-Schreib- bzw. -Lesevorgänge, was die Ausführungsgeschwindigkeit des Simulators erhöhen kann. Auch wird so eine mögliche Fehlerquelle beseitigt.

Zur Funktionsweise des "H-Reglers" im ersten Ausführungsbeispiel im Unterschied zum "schnellen H-Regler" des zweiten Ausführungsbeispiels wird angemerkt, dass beim einfachen H-Regler zwei Temperaturverteilungen, nämlich die basale Temperaturverteilung und die relative Temperaturinkrementverteilung, ähnlich wie in Modus=1, durch zweifache Lösung der BHTE numerisch berechnet werden. Anschließend wird eine Schleife mit z.B. i=10 Durchgängen aufgerufen. Für jeden Durchgang dient ein vordefinierter H-Feldstärke-Wert H(i) als Input, so dass Berechnungen für insgesamt 10 H-Feldstärkewerte, z.B. von 5 kA/m bis 14 kA/m, in 1 kA/m-Schritten durchgeführt werden. Bei jedem Schleifen-Durchlauf i=1...10 wird ein leistungsabsorptionsbasierte Skalierungsfaktor K(i) bestimmt, mit dem die Temperaturinkrementverteilung multipliziert wird. Es ergeben sich i=10 resultierende Temperaturverteilungen, gebildet nach dem weiter oben beschriebenen Skalierungsschema. Anschließend werden diese i=1...10 Temperaturverteilungen nacheinander in ein externes Festplattenverzeichnis geschrieben. Von dort aus können diese nach Wunsch des Users eingeladen werden.

Hinter diesem Lösungsansatz steht die Annahme, dass die 10 Verteilungen (i=1...10) im Hintergrund (ähnlich einem Batchjob) gerechnet werden können, während sich der User etwa die im ersten automatischen Lauf (Modus=1) ermittelte Temperaturverteilung am Bildschirm anschaut. Somit geht dem User keine Zeit verloren, und wenn er mit dem Betrachten der Temperaturverteilung fertig ist, stehen ihm 10 weitere Temperaturverteilungen zur Verfügung, die den gesamten einstellbaren H-Feldstärkebereich abdecken. Somit kann er zwischen diesen Temperaturverteilungen umschalten. Interessiert er sich für einen speziellen Wert, der zwischen den fest vorgegebenen H(i)-Werten liegt, könnte er für diesen Wert noch einmal einen Lauf mit Modus=2 (H-Vorgabe) rechnen.

In der praktischen Anwendung benötigt man hierbei allerdings für große Datensätze vergleichsweise viel Speicherplatz auf der Festplatte, um alle 10 Verteilungen zu speichern; ein typischer Wert liegt hier in der Größenordnung von 2GByte. Das Schreiben der 10 Temperaturverteilungen auf die Festplatte und das Lesen von der Festplatte dauert entsprechend lang. Das sequentielle Einladen der Temperatur-Datensätze von der Festplatte ist mit einer Reihe von Operationen verbunden, wie etwa dem Laden der Konturen, Berechnen eines Katheter-Splines, etc., wobei diese vom Ressourcenverbrauch her mit einem Durchgang der BHTE vergleichbar sein können. Unter Umständen kann so der Vorteil des Umschaltens zwischen den verschiedenen vorab berechneten Datensätzen nicht ausgeschöpft werden.

Der "einfache" H-Regler gemäß erstem Ausführungsbeispiel stellt nur Temperaturverteilungen für fest vordefinierte H-Feldstärken zur Verfügung. Ist ein Wert zwischen den vordefinierten Werten interessant, muss ein Temperatur-Simulator-Durchlauf z.B. unter der H-Vorgabe (Modus=2) gestartet werden. Dies bedeutet mindestens eine numerische Lösung der BHTE, also eine zeitintensive Berechnung.

Das zweite Ausführungsbeispiel verwirklicht einen anderen Ansatz für den H-Regler, der hier als "schneller H-Regler" bezeichnet wird. Dieser zeichnet sich gegenüber dem einfachen H-Regler durch seine Schnelligkeit aus, da er keine numerische Lösung der BHTE erfordert, sondern eine Skalierung der bereit gestellten (zuvor im T-Vorgabe-Schritt berechneten) relativen Temperaturinkrementverteilung durchführt. Mit anderen Worten, der schnelle H-Regler führt die Ermittlung des leistungsabsorptionsbasierten Skalierungsfaktors K für einen einzigen H-Feldstärke-Wert durch, und dann wird die jeweilige Temperaturverteilung nach dem weiter oben beschriebenen Skalierungsansatz gebildet.

Der schnelle H-Regler führt somit keine zeit- und speicherplatzintensive Vorberechnung von mehreren (bspw. 10) Verteilungen durch, und ist somit besonders geeignet etwa für große Datensätze. Jeder beliebige H-Feldstärkewert ist einstellbar und ressourcenschonend berechenbar. Allerdings benötigt der schnelle H-Regler einen vorherigen Lauf im Modus=1. Die dort berechneten beiden Temperaturverteilungen müssen dem schnellen H-Regler als Input zur Verfügung stehen. Entsprechender Arbeitsspeicherplatz muss zur Verfügung stehen.

Das erste Ausführungsbeispiel eines Simulators für ein Simulationswerkzeug braucht zur Ausführung somit tendenziell länger, benötigt auch mehr Festplattenspeicher (dies stellt bei heute üblichen Systemen in der Regel kein Problem dar), jedoch weniger Arbeitsspeicher. Damit ist dieser Simulator unter Umständen besonders geeignet zur Ausführung auf einem PC, etwa einem Einzelplatz-PC, oder auch einem mobilem Rechner wie einem Notebook oder dgl. Demgegenüber arbeitet das zweite Ausführungsbeispiel tendenziell schneller, insbesondere bei der Verarbeitung vom Benutzer oder Anwender eingegebener H-Feldstärkewerte, und benötigt weniger Festplattenspeicherplatz, jedoch einen größeren Arbeitsspeicher. Damit ist das zweite Ausführungsbeispiel eines Simulators für ein Simulationswerkzeug tendenziell für den Einsatz auf leistungsfähigen Rechnern wie etwa Workstations oder in Mainframe-Systemen geeignet.

Es sind weitere Abwandlungen der Ausführungsbeispiele denkbar, die zu Mischformen zwischen den beschriebenen Funktionalitäten des ersten und zweiten Ausführungsbeispiels führen. So ist es denkbar, dass eine ausführbare Datei wie z.B. sim_t.exe des ersten Ausführungsbeispiels die basale Temperaturverteilung sowie die relative Temperaturinkrementverteilung auf die Festplatte schreibt, von wo sie dann durch ein anderes ausführbare Datei wie etwa sim_hr.exe wie im ersten Ausführungsbeispiel beschrieben wieder eingelesen werden kann. Auf diese Weise kann ein "schneller H-Regler" ohne erneute numerische Lösung der BHTE realisiert werden, allerdings dürfte das Schreiben und Auslesen der Daten auf die bzw. von der Festplatte den Ablauf verzögern. Dennoch kann diese Abwandlung für bestimmte Softwarekonfigurationen, Hardwarekonfigurationen, und/oder Anwendungsfälle insgesamt vorteilhaft sein.

Die Erfindung ist nicht auf die hier beschriebenen Ausführungsbeispiele und die darin hervorgehobenen Aspekte beschränkt; vielmehr sind innerhalb des durch die anhängenden Ansprüche angegebenen Bereichs eine Vielzahl von Abwandlungen möglich, die im Rahmen fachmännischen Handelns liegen.

Zu illustrativen Zwecken ist nachfolgend Folgendes kurz zusammengefasst beschrieben:

Gegenstand 1. Computergestütztes Simulationsverfahren zur Unterstützung einer Thermotherapie-Planung, wobei die Thermotherapie eine Hyperthermie-Behandlung eines Tumorvolumens in einem Körpervolumen eines menschlichen Körpers umfasst, wobei die Hyperthermie-Behandlung das Applizieren eines Magnetfeldes in einem Behandlungsvolumen mittels eines Magnetfeld-Applikators umfasst, wobei in mindestens einem Depotvolumen mittels im Körper deponierter magnetischer, paramagnetischer und/oder superparamagnetischer Nanopartikel durch Leistungsabsorption im applizierten Magnetfeld Wärmeenergie einbringbar ist, und wobei das Verfahren die folgenden Schritte umfasst:

- in einem ersten Berechnungsschritt ("T-Vorgabe"), Berechnen eines am Applikator einzustellenden Feldstärkewertes basierend auf einer geometrischen Verteilung der Nanopartikel und mindestens einem vorgegebenen Temperaturgrenzwert, der durch die Hyperthermie-Behandlung nicht überschritten werden soll;
- in einem optionalen zweiten Berechnungsschritt ("H-Regler"), Berechnen, für jeden Feldstärkewert einer Mehrzahl vorgegebener Feldstärkewerte, einer zu erwartenden Temperaturverteilung für mindestens einen Teil des Körpervolumens; und
- Bereitstellen des berechneten Feldstärkewertes und optional der berechneten Temperaturverteilungen zur Unterstützung des Anwenders bei der Planung der Thermotherapie.

Gegenstand 2. Verfahren nach Gegenstand 1, wobei der Temperaturgrenzwert oder einer von mehreren Temperaturgrenzwerten eine maximale Temperatur nur innerhalb des zu erhitzenden Behandlungsvolumens betrifft.

Gegenstand 3. Verfahren nach Gegenstand 2, wobei der Temperaturgrenzwert ein vorgegebenes Temperaturmaximum in einem Bereich von 60°C bis 100°C, bevorzugt 70°C bis 90°C, insbesondere 80°C, im Behandlungsvolumen betrifft.

Gegenstand 4. Verfahren nach einem der vorhergehenden Gegenstände, wobei der Temperaturgrenzwert oder einer von mehreren Temperaturgrenzwerten eine maximale Temperatur außerhalb des zu erhitzenden Behandlungsvolumens betrifft.

Gegenstand 5. Verfahren nach Gegenstand 4, wobei der Temperaturgrenzwert ein vorgegebenes Temperaturmaximum in einem Bereich von 40°C bis 45°C, insbesondere 43°C, außerhalb des Behandlungsvolumens betrifft.

Gegenstand 6. Verfahren nach einem der vorhergehenden Gegenstände, wobei in den ersten Berechnungsschritt ("T-Vorgabe") zwei Temperaturgrenzwerte eingehen, die jeweils unterschiedliche Volumina betreffen.

Gegenstand 7. Verfahren nach einem der vorhergehenden Gegenstände, wobei abhängig vom Berechnungsergebnis des ersten Berechnungsschrittes ("T-Vorgabe") automatisch dann ein dritter Berechnungsschritt ("H-Vorgabe") durchgeführt wird, wenn der im ersten Berechnungsschritt ("T-Vorgabe") berechnete Feldstärkewert größer ist als ein vorgegebener maximaler Feldstärkewert, insbesondere ein maximal einstellbarer Feldstärkewert am Applikator, wobei im dritten Berechnungsschritt ("H-Vorgabe") für den vorgegebenen maximalen Feldstärkewert eine zu erwartende Temperaturverteilung berechnet wird.

Gegenstand 8. Verfahren nach einem der vorhergehenden Gegenstände, wobei in die Berechnungen im zweiten Berechnungsschritt ("H-Regler") und/oder im dritten Berechnungsschritt ("H-Vorgabe") kein Temperaturgrenzwert eingeht.

Gegenstand 9. Verfahren nach einem der vorhergehenden Gegenstände, wobei die Berechnungen im zweiten Berechnungsschritt ("H-Regler") für eine Mehrzahl vorgegebener, am Applikator einstellbarer Feldstärkewerte, bevorzugt zwischen 3 und 20 Feldstärkewerten, besonders bevorzugt zwischen 5 und 10 Feldstärkewerten, durchgeführt werden.

Gegenstand 10. Verfahren nach einem der vorhergehenden Gegenstände, wobei der zweite Berechnungsschritt ("H-Regler") nach dem ersten ("T-Vorgabe") und ggf. dritten Berechnungsschritt ("H-Vorgabe") durch eine Anwendereingabe ausgelöst wird. Gegenstand 11. Verfahren nach einem der vorhergehenden Gegenstände, wobei nach Ausgabe der Berechnungsergebnisse ein vierter Berechnungsschritt ("H-Vorgabe") durchgeführt wird, bei dem eine Anwendereingabe einer Feldstärke entgegengenommen wird und basierend auf der entgegengenommenen Feldstärke eine zu erwartende Temperaturverteilung berechnet wird.

Gegenstand 12. Verfahren nach einem der vorhergehenden Gegenstände, wobei die Berechnung des einzustellenden Feldstärkewertes im ersten Berechnungsschritt ("T-Vorgabe") keine Iteration umfasst, bei der aus gewählten Feldstärkewerten Temperaturverteilungen berechnet werden, um so iterativ zu dem gesuchten Feldstärkewert zu gelangen.

Gegenstand 13. Verfahren nach einem der vorhergehenden Gegenstände, wobei der erste Berechnungsschritt ("T-Vorgabe") die folgenden Schritte aufweist:

- Berechnen einer mittleren Leistungsabsorptionsdichte ("SAR_aver") im Applikator-Magnetfeld im Depotvolumen, wobei eine relative Leistungsabsorptionsdichte basierend auf einer gemessenen geometrischen Verteilung der Nanopartikel berechnet wird, eine vorgegebene Bio-Heat-Transfer-Gleichung genau einmal zum Erhalt einer basalen Temperaturverteilung ohne Leistungsabsorption gelöst wird, und die Bio-Heat-Transfer-Gleichung genau einmal zum Erhalt einer relativen Temperaturinkrementverteilung basierend auf der relativen Leistungsabsorptionsdichte gelöst wird; und wobei die relative Leistungsabsorptionsdichte durch einen Skalierungsfaktor skaliert wird, der basierend auf dem mindestens einen vorgegebenen Temperaturgrenzwert, der basalen Temperaturverteilung und der relativen Temperaturinkrementverteilung erhalten wird;
- Berechnen, basierend auf der berechneten mittleren Leistungsabsorptionsdichte und einer berechneten Masse der Nanopartikel, einer Referenz-Leistungsabsorptionsrate ("SAR_Fe"), die zum Beispiel die spezifische Leistungsabsorptionsrate einer die Nanopartikel enthaltenden, unverdünnten Magnetflüssigkeit angibt; und

- Berechnen eines Feldstärkewertes basierend auf der berechneten Referenz-Leistungsabsorptionsrate und einer vorgegebenen Kennlinie, die einen Zusammenhang zwischen Referenz-Leistungsabsorptionsrate und applizierter Feldstärke betrifft.

Gegenstand 14. Verfahren nach einem der vorhergehenden Gegenstände, wobei die Berechnung der Temperaturverteilungen im zweiten Berechnungsschritt ("H-Regler") unabhängig von der Zahl der vorgegebenen Feldstärkewerte genau zwei Berechnungen von Temperaturverteilungen umfasst.

Gegenstand 15. Verfahren nach einem der vorhergehenden Gegenstände, wobei der zweite Berechnungsschritt ("H-Regler") die folgenden Schritte aufweist:

- Berechnen einer relativen Leistungsabsorptionsdichteverteilung ("ΔSAR(x,y,z)") und einer relativen mittleren Leistungsabsorptionsdichte ("ΔSAR_aver") basierend auf einer gemessenen geometrischen Verteilung der Nanopartikel;
- Bereitstellen einer basalen Temperaturverteilung ("T0(x,y,z)") basierend auf einer Lösung einer vorgegebenen Bio-Heat-Transfer-Gleichung ohne Leistungsabsorption, und Bereitstellen einer relativen Temperaturinkrementverteilung ("ΔT(x,y,z)") basierend auf einer Lösung der Bio-Heat-Transfer-Gleichung mit der berechneten relativen Leistungsabsorptionsdichteverteilung ("ΔSAR(x,y,z)");
- Durchführen der folgenden Schritte für jeden Feldstärkewert der Mehrzahl vorgegebener Feldstärkewerte:

  o Berechnen einer Referenz-Leistungsabsorptionsrate ("SAR_Fe(i)"), die zum Beispiel die spezifische Leistungsabsorptionsrate einer die Nanopartikel enthaltenden, unverdünnten Magnetflüssigkeit angibt, wobei die Berechnung auf dem jeweiligen Feldstärkewert und einer vorgegebenen Kennlinie basiert, die einen Zusammenhang zwischen Referenz-Leistungsabsorptionsrate und applizierter Feldstärke betrifft;
  ◦ Berechnen, basierend auf der Referenz-Leistungsabsorptionsrate ("SAR_Fe(i)") und einer berechneten Masse der Nanopartikel im Depotvolumen, einer mittleren Leistungsabsorptionsdichte ("SAR_aver(i)");
  ◦ Berechnen eines Skalierungsfaktors ("K(i)") basierend auf der jeweiligen mittleren Leistungsabsorptionsdichte ("SAR_aver(i)") und der relativen mittleren Leistungsabsorptionsdichte (("ΔSAR_aver");
  ◦ Berechnen einer jeweiligen Temperaturverteilung ("T(x,y,z,i)") basierend auf der basalen Temperaturverteilung ("T0(x,y,z)"), der relativen Temperaturinkrementverteilung ("ΔT(x,y,z)"), und dem Skalierungsfaktor ("K(i)").

Gegenstand 16. Computergestütztes Simulationsverfahren ("T-Vorgabe") zur Unterstützung einer Thermotherapie-Planung, wobei die Thermotherapie eine Hyperthermie-Behandlung eines Tumorvolumens in einem Körpervolumen eines menschlichen Körpers umfasst, wobei die Hyperthermie-Behandlung das Applizieren eines Magnetfeldes in einem Behandlungsvolumen mittels eines Magnetfeld-Applikators umfasst, wobei in mindestens einem Depotvolumen mittels im Körper deponierter magnetischer, paramagnetischer und/oder superparamagnetischer Nanopartikel durch Leistungsabsorption im applizierten Magnetfeld Wärmeenergie einbringbar ist, wobei das Verfahren die Berechnung einer am Applikator einzustellenden Feldstärke basierend auf einer geometrischen Verteilung der Nanopartikel und mindestens einem vorgegebenen Temperaturgrenzwert, der durch die Hyperthermie-Behandlung nicht überschritten werden soll, betrifft ("T-Vorgabe");
wobei das Verfahren die folgenden Schritte aufweist:

- Berechnen einer mittleren Leistungsabsorptionsdichte ("SAR_aver") im Applikator-Magnetfeld im Depotvolumen, wobei eine relative Leistungsabsorptionsdichte basierend auf einer gemessenen geometrischen Verteilung der Nanopartikel berechnet wird, eine vorgegebene Bio-Heat-Transfer-Gleichung genau einmal zum Erhalt einer basalen Temperaturverteilung ohne Leistungsabsorption gelöst wird, und die Bio-Heat-Transfer-Gleichung genau einmal zum Erhalt einer relativen Temperaturinkrementverteilung basierend auf der relativen Leistungsabsorptionsdichte gelöst wird; und wobei die relative Leistungsabsorptionsdichte durch einen Skalierungsfaktor skaliert wird, der basierend auf dem mindestens einen vorgegebenen Temperaturgrenzwert, der basalen Temperaturverteilung und der relativen Temperaturinkrementverteilung erhalten wird;
- Berechnen, basierend auf der berechneten mittleren Leistungsabsorptionsdichte und einer berechneten Masse der Nanopartikel, einer Referenz-Leistungsabsorptionsrate ("SAR_Fe"), die zum Beispiel die spezifische Leistungsabsorptionsrate einer die Nanopartikel enthaltenden, unverdünnten Magnetflüssigkeit angibt;
- Berechnen eines Feldstärkewertes basierend auf der berechneten Referenz-Leistungsabsorptionsrate und einer vorgegebenen Kennlinie, die einen Zusammenhang zwischen Referenz-Leistungsabsorptionsrate und applizierter Feldstärke betrifft; und

- Bereitstellen des berechneten Feldstärkewertes zur Unterstützung des Anwenders bei der Planung der Thermotherapie.

Gegenstand 17. Computergestütztes Simulationsverfahren ("H-Regler") zur Unterstützung einer Thermotherapie-Planung, wobei die Thermotherapie eine Hyperthermie-Behandlung eines Tumorvolumens in einem Körpervolumen eines menschlichen Körpers umfasst, wobei die Hyperthermie-Behandlung das Applizieren eines Magnetfeldes in einem Behandlungsvolumen mittels eines Magnetfeld-Applikators umfasst, wobei in mindestens einem Depotvolumen mittels im Körper deponierter magnetischer, paramagnetischer und/oder superparamagnetischer Nanopartikel durch Leistungsabsorption im applizierten Magnetfeld Wärmeenergie einbringbar ist, wobei das Verfahren die Berechnung, für jeden Feldstärkewert einer Mehrzahl vorgegebener Feldstärkewerte, einer zu erwartenden Temperaturverteilung für mindestens einen Teil des Körpervolumens betrifft ("H-Regler"); und wobei das Verfahren die folgenden Schritte aufweist:

- Berechnen einer relativen Leistungsabsorptionsdichteverteilung ("ΔSAR(x,y,z)") und einer relativen mittleren Leistungsabsorptionsdichte ("ΔSAR_aver") basierend auf einer gemessenen geometrischen Verteilung der Nanopartikel;
- Bereitstellen einer basalen Temperaturverteilung ("T0(x,y,z)") basierend auf einer Lösung einer vorgegebenen Bio-Heat-Transfer-Gleichung ohne Leistungsabsorption, und Bereitstellen einer relativen Temperaturinkrementverteilung ("ΔT(x,y,z)") basierend auf einer Lösung der Bio-Heat-Transfer-Gleichung mit der berechneten relativen Leistungsabsorptionsdichte ("ΔSAR(x,y,z)");;
- Durchführen der folgenden Schritte für jeden Feldstärkewert der Mehrzahl vorgegebener Feldstärkewerte:

  ◦ Berechnen einer Referenz-Leistungsabsorptionsrate ("SAR_Fe(i)"), die zum Beispiel die spezifische Leistungsabsorptionsrate einer die Nanopartikel enthaltenden, unverdünnten Magnetflüssigkeit angibt, wobei die Berechnung auf dem jeweiligen Feldstärkewert und einer vorgegebenen Kennlinie basiert, die einen Zusammenhang zwischen Referenz-Leistungsabsorptionsrate und applizierter Feldstärke betrifft;
  ◦ Berechnen, basierend auf der Referenz-Leistungsabsorptionsrate ("SAR_Fe(i)") und einer berechneten Masse der Nanopartikel im Depotvolumen, einer mittleren Leistungsabsorptionsdichte ("SAR_aver(i)");
  ◦ Berechnen eines Skalierungsfaktors ("K(i)") basierend auf der jeweiligen mittleren Leistungsabsorptionsdichte ("SAR_aver(i)") und der relativen Leistungsabsorptionsdichte (("ΔSAR_aver");
  ◦ Berechnen einer jeweiligen Temperaturverteilung ("T(x,y,z,i)") basierend auf der basalen Temperaturverteilung ("T0(x,y,z)"), der relativen Temperaturinkrementverteilung ("ΔT(x,y,z)"), und dem Skalierungsfaktor ("K(i)");

- Bereitstellen der berechneten Temperaturverteilungen zur Unterstützung des Anwenders bei der Planung der Thermotherapie.

Gegenstand 18. Computerprogramm zur Durchführung des Verfahrens nach einem der vorhergehenden Gegenstände, wenn das Computerprogramm auf einer programmierbaren Computereinrichtung ausgeführt wird.

Gegenstand 19. Datenträger, auf dem das Computerprogramm nach Gegenstand 18 aufgezeichnet ist.

Gegenstand 20. Computereinrichtung ausgebildet zur Unterstützung einer Thermotherapie-Planung,
wobei die Thermotherapie eine Hyperthermie-Behandlung eines Tumorvolumens in einem Körpervolumen eines menschlichen Körpers umfasst,
wobei die Hyperthermie-Behandlung das Applizieren eines Magnetfeldes in einem Behandlungsvolumen mittels eines Magnetfeld-Applikators umfasst,
wobei in mindestens einem Depotvolumen mittels im Körper deponierter magnetischer, paramagnetischer und/oder superparamagnetischer Nanopartikel durch Leistungsabsorption im applizierten Magnetfeld Wärmeenergie einbringbar ist, und wobei die Computereinrichtung die folgenden Komponenten umfasst:

- eine erste Berechnungskomponente ("sim_t.exe") ausgebildet zum Berechnen eines am Applikator einzustellenden Feldstärkewertes basierend auf einer geometrischen Verteilung der Nanopartikel und mindestens einem vorgegebenen Temperaturgrenzwert, der durch die Hyperthermie-Behandlung nicht überschritten werden soll;
- eine zweite Berechnungskomponente ("sim_hr.exe") ausgebildet zum optionalen Berechnen, für jeden Feldstärkewert einer Mehrzahl vorgegebener Feldstärkewerte, einer zu erwartenden Temperaturverteilung für mindestens einen Teil des Körpervolumens; und
- eine Komponente zum Bereitstellen des berechneten Feldstärkewertes und optional der berechneten Temperaturverteilungen zur Unterstützung des Anwenders es bei der Planung der Thermotherapie.

Gegenstand 21. Computereinrichtung ausgebildet zur Unterstützung einer Thermotherapie-Planung, wobei die Thermotherapie eine Hyperthermie-Behandlung eines Tumorvolumens in einem Körpervolumen eines menschlichen Körpers umfasst, wobei die Hyperthermie-Behandlung das Applizieren eines Magnetfeldes in einem Behandlungsvolumen mittels eines Magnetfeld-Applikators umfasst, wobei in mindestens einem Depotvolumen mittels im Körper deponierter magnetischer, paramagnetischer und/oder superparamagnetischer Nanopartikel durch Leistungsabsorption im applizierten Magnetfeld Wärmeenergie einbringbar ist, wobei die Computereinrichtung eine Komponente ("sim_t.exe") aufweist, die ausgebildet ist zur Berechnung einer am Applikator einzustellenden Feldstärke basierend auf einer geometrischen Verteilung der Nanopartikel und mindestens einem vorgegebenen Temperaturgrenzwert, der durch die Hyperthermie-Behandlung nicht überschritten werden soll; und wobei die Komponente ("sim_t.exe") die folgenden Module aufweist:

- ein Modul zum Berechnen einer mittleren Leistungsabsorptionsdichte im Applikator-Magnetfeld im Depotvolumen, wobei eine relative Leistungsabsorptionsdichte basierend auf einer gemessenen geometrischen Verteilung der Nanopartikel berechnet wird, eine vorgegebene Bio-Heat-Transfer-Gleichung genau einmal zum Erhalt einer basalen Temperaturverteilung ohne Leistungsabsorption gelöst wird, und die Bio-Heat-Transfer-Gleichung genau einmal zum Erhalt einer relativen Temperaturinkrementverteilung basierend auf der relativen Leistungsabsorptionsdichte gelöst wird; und wobei die relative Leistungsabsorptionsdichte durch einen Skalierungsfaktor skaliert wird, der basierend auf dem mindestens einen vorgegebenen Temperaturgrenzwert, der basalen Temperaturverteilung und der relativen Temperaturinkrementverteilung erhalten wird;
- ein Modul zum Berechnen, basierend auf der berechneten mittleren Leistungsabsorptionsdichte und einer berechneten Masse der Nanopartikel, einer Referenz-Leistungsabsorptionsrate, die zum Beispiel die spezifische Leistungsabsorptionsrate einer die Nanopartikel enthaltenden, unverdünnten Magnetflüssigkeit angibt;
- ein Modul zum Berechnen eines Feldstärkewertes basierend auf der berechneten Referenz-Leistungsabsorptionsrate und einer vorgegebenen Kennlinie, die einen Zusammenhang zwischen Referenz-Leistungsabsorptionsrate und applizierter Feldstärke betrifft; und
- ein Modul zum Bereitstellen des berechneten Feldstärkewertes zur Unterstützung des Anwenders bei der Planung der Thermotherapie.

Gegenstand 22. Computereinrichtung ausgebildet zur Unterstützung einer Thermotherapie-Planung, wobei die Thermotherapie eine Hyperthermie-Behandlung eines Tumorvolumens in einem Körpervolumen eines menschlichen Körpers umfasst, wobei die Hyperthermie-Behandlung das Applizieren eines Magnetfeldes in einem Behandlungsvolumen mittels eines Magnetfeld-Applikators umfasst, wobei in mindestens einem Depotvolumen mittels im Körper deponierter magnetischer, paramagnetischer und/oder superparamagnetischer Nanopartikel durch Leistungsabsorption im applizierten Magnetfeld Wärmeenergie einbringbar ist, wobei die Computereinrichtung eine Komponente ("sim_hr.exe") aufweist, die ausgebildet ist zur Berechnung, für jeden Feldstärkewert einer Mehrzahl vorgegebener Feldstärkewerte, einer zu erwartenden Temperaturverteilung für mindestens einen Teil des Körpervolumens; und wobei die Komponente ("sim_hr.exe") die folgenden Module aufweist:

- ein Modul zum Berechnen einer relativen Leistungsabsorptionsdichteverteilung und einer relativen mittleren Leistungsabsorptionsdichte basierend auf einer gemessenen geometrischen Verteilung der Nanopartikel;
- ein Modul zum Bereitstellen einer basalen Temperaturverteilung basierend auf einer Lösung einer vorgegebenen Bio-Heat-Transfer-Gleichung ohne Leistungsabsorption, und Bereitstellen einer relativen Temperaturinkrementverteilung basierend auf einer Lösung der Bio-Heat-Transfer-Gleichung mit der berechneten relativen Leistungsabsorptionsdichteverteilung;
- ein Modul zum Durchführen der folgenden Schritte für jeden Feldstärkewert der Mehrzahl vorgegebener Feldstärkewerte:

  ◦ Berechnen einer Referenz-Leistungsabsorptionsrate, die zum Beispiel die spezifische Leistungsabsorptionsrate einer die Nanopartikel enthaltenden, unverdünnten Magnetflüssigkeit angibt, wobei die Berechnung auf dem jeweiligen Feldstärkewert und einer vorgegebenen Kennlinie basiert, die einen Zusammenhang zwischen Referenz-Leistungsabsorptionsrate und applizierter Feldstärke betrifft;
  ◦ Berechnen, basierend auf der Referenz-Leistungsabsorptionsrate und einer berechneten Masse der Nanopartikel im Depotvolumen, einer mittleren Leistungsabsorptionsdichte;
  ◦ Berechnen eines Skalierungsfaktors basierend auf der jeweiligen mittleren Leistungsabsorptionsdichte und der relativen Leistungsabsorptionsdichte;
  ◦ Berechnen einer jeweiligen Temperaturverteilung basierend auf der basalen Temperaturverteilung, der relativen Temperaturinkrementverteilung, und dem Skalierungsfaktor;

- ein Modul zum Bereitstellen der berechneten Temperaturverteilungen zur Unterstützung des Anwenders bei der Planung der Thermotherapie.

Gegenstand 23. System, umfassend eine Computereinrichtung nach einem der Gegenstände 20 bis 22, und einen Magnetfeld-Applikator.

Gegenstand 24. System, umfassend ein Computerprogramm nach Gegenstand 18, einen Datenträger nach Gegenstand 19, eine Computereinrichtung nach einem der Gegenstände 20 bis 22, oder ein System nach Gegenstand 23, sowie umfassend eine Magnetflüssigkeit enthaltend magnetische Nanopartikel.

Gegenstand 25. Verfahren zur kontrollierten Erwärmung eines Organs oder Gewebes enthaltend die Schritte

A) Einbringen von magnetischen, paramagnetischen und/oder superparamagnetischen Partikeln in ein Organvolumen oder Gewebevolumen,
B) Ermitteln der Partikelmenge und/oder -Verteilung in dem Organvolumen oder Gewebevolumen,
C) Berechnen einer einzustellenden Feldstärke gemäß dem Verfahren nach Gegenstand 1 oder 16 oder einer Temperaturverteilung gemäß dem Verfahren nach Gegenstand 17,
D) Deponieren von Wärmeenergie mittels Applikation eines Magnetfeldes, wobei die applizierte Feldstärke der berechneten Feldstärke oder der aus einer berechneten Temperaturverteilung abgeleiteten Feldstärke jeweils mit einer Abweichung von +/- 10%, bevorzugt +/- 5%, insbesondere +/- 1% entspricht.

Gegenstand 26. Verfahren zur Behandlung eines Tumors in einem Patienten enthaltend die Schritte

A) Einbringen von magnetischen, paramagnetischen und/oder superparamagnetischen Partikeln in ein Tumorvolumen,
B) Ermitteln der Partikelmenge und/oder -Verteilung in dem Tumorvolumen,
C) Berechnen einer einzustellenden Feldstärke gemäß dem Verfahren nach Gegenstand 1 oder 16 oder einer Temperaturverteilung gemäß dem Verfahren nach Gegenstand 17,
D) Deponieren von Wärmeenergie mittels Applikation eines Magnetfeldes, wobei die applizierte Feldstärke der berechneten Feldstärke oder der aus einer berechneten Temperaturverteilung abgeleiteten Feldstärke jeweils mit einer Abweichung von +/- 10%, bevorzugt +/- 5%, insbesondere +/- 1% entspricht.

**Patentansprüche**

1. Computergestütztes Simulationsverfahren zur Unterstützung einer Thermotherapie-Planung,
wobei die Thermotherapie eine Hyperthermie-Behandlung eines Tumorvolumens in einem Körpervolumen eines menschlichen Körpers umfasst,
wobei die Hyperthermie-Behandlung das Applizieren eines Magnetfeldes in einem Behandlungsvolumen mittels eines Magnetfeld-Applikators umfasst,
wobei in mindestens einem Depotvolumen mittels im Körper deponierter magnetischer, paramagnetischer und/oder superparamagnetischer Nanopartikel durch Leistungsabsorption im applizierten Magnetfeld Wärmeenergie einbringbar ist,
und wobei das Verfahren die folgenden Schritte umfasst:

- in einem ersten Berechnungsschritt ("T-Vorgabe"), Berechnen eines am Applikator einzustellenden Feldstärkewertes basierend auf einer geometrischen Verteilung der Nanopartikel und mindestens einem vorgegebenen Temperaturgrenzwert, der durch die Hyperthermie-Behandlung nicht überschritten werden soll, wobei der Feldstärkewert im ersten Berechnungsschritt ("T-Vorgabe") basierend auf einer vorgegebenen Kennlinie berechnet wird, die einen Zusammenhang zwischen (Referenz-) Leistungsabsorptionsrate und Feldstärke angibt; und
- Bereitstellen des berechneten Feldstärkewertes und optional mindestens einer berechneten Temperaturverteilung zur Unterstützung des Anwenders bei der Planung der Thermotherapie.

2. Verfahren nach Anspruch 1, wobei das Verfahren den folgenden weiteren Schritt nach dem ersten Berechnungsschritt umfasst:

- in einem zweiten Berechnungsschritt ("H-Regler", "schneller H-Regler"), Berechnen einer zu erwartenden Temperaturverteilung für mindestens einen Teil des Körpervolumens für

 o jeden Feldstärkewert einer Mehrzahl vorgegebener Feldstärkewerte, und/oder
 o einen anwenderdefinierten Feldstärkewert.

3. Verfahren nach Anspruch 1 oder 2, wobei
der Temperaturgrenzwert oder einer von mehreren Temperaturgrenzwerten eine maximale Temperatur nur innerhalb des zu erhitzenden Behandlungsvolumens betrifft, wobei vorzugsweise der Temperaturgrenzwert ein vorgegebenes Temperaturmaximum in einem Bereich von 60°C bis 100°C, bevorzugt 70°C bis 90°C, insbesondere 80°C, im Behandlungsvolumen betrifft, und/oder
der Temperaturgrenzwert oder einer von mehreren Temperaturgrenzwerten eine maximale Temperatur außerhalb des zu erhitzenden Behandlungsvolumens betrifft, wobei vorzugsweise der Temperaturgrenzwert ein vorgegebenes Temperaturmaximum in einem Bereich von 40°C bis 45°C, insbesondere 43°C, außerhalb des Behandlungsvolumens betrifft.

4. Verfahren nach einem der Ansprüche 2 oder 3, wobei

a) in die Berechnungen im zweiten Berechnungsschritt ("H-Regler", "schneller H-Regler") kein Temperaturgrenzwert eingeht, oder
b) die Berechnungen im zweiten Berechnungsschritt ("H-Regler") für eine Mehrzahl vorgegebener, am Applikator einstellbarer Feldstärkewerte, bevorzugt zwischen 3 kA/m und 20 kA/m, besonders bevorzugt zwischen 5 kA/m und 10 kA/m, durchgeführt werden, oder
c) der zweite Berechnungsschritt ("H-Regler", "schneller H-Regler") nach dem ersten ("T-Vorgabe") und ggf. einem dritten Berechnungsschritt ("H-Vorgabe") durch eine Anwendereingabe ausgelöst wird.

5. Verfahren nach einem der Ansprüche 1 bis 3, wobei

a) abhängig vom Berechnungsergebnis des ersten Berechnungsschrittes ("T-Vorgabe") automatisch dann ein dritter Berechnungsschritt ("H-Vorgabe") durchgeführt wird, wenn der im ersten Berechnungsschritt ("T-Vorgabe") berechnete Feldstärkewert größer ist als ein vorgegebener maximaler Feldstärkewert, insbesondere ein maximal einstellbarer Feldstärkewert am Applikator, wobei im dritten Berechnungsschritt ("H-Vorgabe") für den vorgegebenen maximalen Feldstärkewert eine zu erwartende Temperaturverteilung berechnet wird, oder
b) in die Berechnungen im dritten Berechnungsschritt ("H-Vorgabe") kein Temperaturgrenzwert eingeht, oder
c) die Berechnung des einzustellenden Feldstärkewertes im ersten Berechnungsschritt ("T-Vorgabe") keine Iteration umfasst, bei der aus gewählten Feldstärkewerten Temperaturverteilungen berechnet werden, um so iterativ zu dem gesuchten Feldstärkewert zu gelangen.

6. Verfahren nach Anspruch 1 oder 2, wobei der erste Berechnungsschritt ("T-Vorgabe") die folgenden Schritte aufweist:

- Berechnen einer mittleren Leistungsabsorptionsdichte ("SAR_aver") im Applikator-Magnetfeld im Depotvolumen, wobei eine relative Leistungsabsorptionsdichte ("$\Delta SAR(x,y,z)$") basierend auf einer gemessenen geometrischen Verteilung der Nanopartikel berechnet wird, eine das Modell beschreibende Bio-Heat-Transfer-Gleichung genau einmal zum Erhalt einer basalen Temperaturverteilung ("$T0(x,y,z)$") ohne Leistungsabsorption numerisch gelöst wird, und die Bio-Heat-Transfer-Gleichung genau einmal zum Erhalt einer relativen Temperaturinkrementverteilung ("$\Delta T(x,y,z)$") basierend auf der relativen Leistungsabsorptionsdichte numerisch gelöst wird; und wobei die relative Leistungsabsorptionsdichte ("$\Delta SAR(x,y,z)$") durch einen temperaturbasierten Skalierungsfaktor ("K") skaliert wird, der basierend auf dem mindestens einen vorgegebenen Temperaturgrenzwert, der basalen Temperaturverteilung und der relativen Temperaturinkrementverteilung erhalten wird;
- Berechnen, basierend auf der berechneten mittleren Leistungsabsorptionsdichte und einer berechneten Masse der Nanopartikel, einer Referenz-Leistungsabsorptionsrate ("SAR_Fe"), die zum Beispiel die spezifische Leistungsabsorptionsrate einer die Nanopartikel enthaltenden, unverdünnten Magnetflüssigkeit angibt;
- Berechnen eines Feldstärkewertes ("H") basierend auf der berechneten Referenz-Leistungsabsorptionsrate ("SAR_Fe") und einer vorgegebenen Kennlinie, die einen Zusammenhang zwischen Referenz-Leistungsabsorptionsrate und applizierter Feldstärke ("H") betrifft; und
- Optionales Berechnen einer jeweiligen Temperaturverteilung ("$T(x,y,z)$") basierend auf der basalen Temperaturverteilung ("$T0(x,y,z)$"), der relativen Temperaturinkrementverteilung ("$\Delta T(x,y,z)$"), und dem temperaturbasierten Skalierungsfaktor ("K");

wobei vorzugsweise die basale Temperaturverteilung ("T0(x,y,z)") und/oder die relative Temperaturinkrementverteilung ("ΔT(x,y,z)") für mindestens eine weitere Verwendung ("schneller H-Regler") über den ersten Berechnungsschritt hinaus bereit gestellt werden.

7. Verfahren nach einem der Ansprüche 2 bis 6, wobei

a) im zweiten Berechnungsschritt ("schneller H-Regler") unabhängig von der Zahl der vorgegebenen und/oder anwenderdefinierten Feldstärkewerte eine bereit gestellte (zuvor im ersten Berechnungsschritt berechnete) basale Temperaturverteilung ("T0(x,y,z)") und/oder eine bereit gestellte (zuvor im ersten Berechnungsschritt berechnete) relative Temperaturinkrementverteilung ("ΔT(x,y,z)") herangezogen werden, oder
b) im zweiten Berechnungsschritt ("H-Regler") unabhängig von der Zahl der vorgegebenen und/oder anwenderdefinierten Feldstärkewerte maximal zwei Temperaturverteilungen berechnet werden, nämlich eine basale Temperaturverteilung ("T0(x,y,z)") und/oder eine relative Temperaturinkrementverteilung ("ΔT(x,y,z)").

8. Verfahren nach einem Ansprüche 2 bis 7, wobei im zweiten Berechnungsschritt ("H-Regler", "schneller H-Regler") die zu erwartende Temperaturverteilung mittels einer leistungsabsorptionsbasierten Skalierung ("K") einer berechneten oder bereitgestellten relativen Temperaturinkrementverteilung ("ΔT(x,y,z)") berechnet wird.

9. Verfahren nach einem der Ansprüche 7 und 8, wobei der zweite Berechnungsschritt ("H-Regler", "schneller H-Regler") die folgenden Schritte aufweist:

- Berechnen einer relativen Leistungsabsorptionsdichteverteilung ("ΔSAR(x,y,z)") und einer relativen mittleren Leistungsabsorptionsdichte ("ΔSAR_aver") basierend auf einer gemessenen geometrischen Verteilung der Nanopartikel;
- Bereitstellen einer basalen Temperaturverteilung ("TO(x,y,z)") basierend auf einer numerischen Lösung einer das Modell beschreibenden Bio-Heat-Transfer-Gleichung ohne Leistungsabsorption, und Bereitstellen einer relativen Temperaturinkrementverteilung ("ΔT(x,y,z)") basierend auf einer numerischen Lösung der Bio-Heat-Transfer-Gleichung mit der berechneten relativen Leistungsabsorptionsdichteverteilung ("ΔSAR(x,y,z)");
- Durchführen der folgenden Schritte für jeden Feldstärkewert der Mehrzahl vorgegebener Feldstärkewerte und/oder den anwenderdefinierten Feldstärkewert:

  ◦ Berechnen einer Referenz-Leistungsabsorptionsrate ("SAR_Fe"), die zum Beispiel die spezifische Leistungsabsorptionsrate einer die Nanopartikel enthaltenden, unverdünnten Magnetflüssigkeit angibt, wobei die Berechnung auf dem jeweiligen Feldstärkewert ("H") und einer vorgegebenen Kennlinie basiert, die einen Zusammenhang zwischen Referenz-Leistungsabsorptionsrate ("SAR_Fe") und applizierter Feldstärke ("H") betrifft;
  ◦ Berechnen, basierend auf der Referenz-Leistungsabsorptionsrate ("SAR_Fe") und einer berechneten Masse der Nanopartikel im Depotvolumen, einer mittleren Leistungsabsorptionsdichte ("SAR_aver");
  ◦ Berechnen eines leistungsabsorptionsbasierten Skalierungsfaktors ("K") basierend auf der jeweiligen mittleren Leistungsabsorptionsdichte ("SAR_aver") und der relativen mittleren Leistungsabsorptionsdichte ("ΔSAR_aver");
  ◦ Berechnen einer jeweiligen Temperaturverteilung ("T(x,y,z)") basierend auf der basalen Temperaturverteilung ("T0(x,y,z)"), der relativen Temperaturinkrementverteilung ("ΔT(x,y,z)"), und dem leistungsabsorptionsbasierten Skalierungsfaktor ("K").

10. Verfahren nach Anspruch 1 ("T-Vorgabe"), wobei das Verfahren ferner die folgenden Schritte aufweist ("T-Vorgabe"):

- Berechnen einer mittleren Leistungsabsorptionsdichte ("SAR_aver") im Applikator-Magnetfeld im Depotvolumen, wobei eine relative Leistungsabsorptionsdichte ("ΔSAR(x,y,z)") basierend auf einer gemessenen geometrischen Verteilung der Nanopartikel berechnet wird, eine das Modell beschreibende Bio-Heat-Transfer-Gleichung genau einmal zum Erhalt einer basalen Temperaturverteilung ("T0(x,y,z)") ohne Leistungsabsorption numerisch gelöst wird, und die Bio-Heat-Transfer-Gleichung genau einmal zum Erhalt einer relativen Temperaturinkrementverteilung ("ΔT(x,y,z)") basierend auf der relativen Leistungsabsorptionsdichte numerisch gelöst wird; und wobei die relative Leistungsabsorptionsdichte ("ΔSAR(x,y,z)") durch einen temperaturbasierten Skalierungsfaktor ("K") skaliert wird, der basierend auf dem mindestens einen vorgegebenen Temperaturgrenzwert, der basalen Temperaturverteilung und der relativen Temperaturinkrementverteilung erhalten wird;
- Berechnen, basierend auf der berechneten mittleren Leistungsabsorptionsdichte und einer berechneten Masse der Nanopartikel, einer Referenz-Leistungsabsorptionsrate ("SAR_Fe"), die zum Beispiel die spezifische Leistungsabsorptionsrate einer die Nanopartikel enthaltenden, unverdünnten Magnetflüssigkeit angibt;

- Berechnen eines Feldstärkewertes ("H") basierend auf der berechneten Referenz-Leistungsabsorptionsrate ("SAR_Fe") und einer vorgegebenen Kennlinie, die einen Zusammenhang zwischen Referenz-Leistungsabsorptionsrate ("SAR_Fe") und applizierter Feldstärke ("H") betrifft;

- Optionales Berechnen einer jeweiligen Temperaturverteilung ("T(x,y,z)") basierend auf der basalen Temperaturverteilung ("T0(x,y,z)"), der relativen Temperaturinkrementverteilung ("ΔT(x,y,z)"), und dem temperaturbasierten Skalierungsfaktor ("K");

- Bereitstellen des berechneten Feldstärkewertes ("H") zur Unterstützung des Anwenders bei der Planung der Thermotherapie; und

- Optionales Bereitstellen der berechneten Temperaturverteilung ("T(x,y,z)") zur Unterstützung des Anwenders bei der Planung der Thermotherapie.

11. Computergestütztes Simulationsverfahren ("H-Regler", "schneller H-Regler") zur Unterstützung einer Thermotherapie-Planung,

wobei die Thermotherapie eine Hyperthermie-Behandlung eines Tumorvolumens in einem Körpervolumen eines menschlichen Körpers umfasst,

wobei die Hyperthermie-Behandlung das Applizieren eines Magnetfeldes in einem Behandlungsvolumen mittels eines Magnetfeld-Applikators umfasst,

wobei in mindestens einem Depotvolumen mittels im Körper deponierter magnetischer, paramagnetischer und/oder superparamagnetischer Nanopartikel durch Leistungsabsorption im applizierten Magnetfeld Wärmeenergie einbringbar ist,

wobei das Verfahren die Berechnung, für jeden Feldstärkewert einer Mehrzahl vorgegebener Feldstärkewerte und/oder einen anwenderdefinierten Feldstärkewert, einer zu erwartenden Temperaturverteilung für mindestens einen Teil des Körpervolumens betrifft;

und wobei die zu erwartende Temperaturverteilung mittels einer leistungsabsorptionsbasierten Skalierung ("K") einer berechneten oder bereit gestellten relativen Temperaturinkrementverteilung ("ΔT(x,y,z)") berechnet wird, wobei vorzugsweise

a) unabhängig von der Zahl der vorgegebenen und/oder anwenderdefinierten Feldstärkewerte eine bereit gestellte (zuvor im T-Vorgabe-Schritt berechnete) basale Temperaturverteilung ("T0(x,y,z)") und/oder eine bereit gestellte (zuvor im T-Vorgabe-Schritt berechnete) relative Temperaturinkrementverteilung ("ΔT(x,y,z)") herangezogen werden ("schneller H-Regler"), oder

b) unabhängig von der Zahl der vorgegebenen und/oder anwenderdefinierten Feldstärkewerte maximal zwei Temperaturverteilungen berechnet werden ("H-Regler"), nämlich eine basale Temperaturverteilung ("T0(x,y,z)") und/oder eine relative Temperaturinkrementverteilung ("ΔT(x,y,z)").

12. Verfahren nach Anspruch 11, wobei das Verfahren die folgenden Schritte aufweist ("H-Regler, "schneller H-Regler"):

- Berechnen einer relativen Leistungsabsorptionsdichteverteilung ("ΔSAR(x,y,z)") und einer relativen mittleren Leistungsabsorptionsdichte ("ASAR_aver") basierend auf einer gemessenen geometrischen Verteilung der Nanopartikel;

- Bereitstellen einer basalen Temperaturverteilung ("T0(x,y,z)") basierend auf einer numerischen Lösung einer das Modell beschreibenden Bio-Heat-Transfer-Gleichung ohne Leistungsabsorption, und Bereitstellen einer relativen Temperaturinkrementverteilung ("ΔT(x,y,z)") basierend auf einer numerischen Lösung der Bio-Heat-Transfer-Gleichung mit der berechneten relativen Leistungsabsorptionsdichte ("ΔSAR(x,y,z)");

- Durchführen der folgenden Schritte für jeden Feldstärkewert der Mehrzahl vorgegebener Feldstärkewerte und/oder den benutzerdefinierten Feldstärkewert:

◦ Berechnen einer Referenz-Leistungsabsorptionsrate ("SAR_Fe"), die zum Beispiel die spezifische Leistungsabsorptionsrate einer die Nanopartikel enthaltenden, unverdünnten Magnetflüssigkeit angibt, wobei die Berechnung auf dem jeweiligen Feldstärkewert ("H") und einer vorgegebenen Kennlinie basiert, die einen Zusammenhang zwischen Referenz-Leistungsabsorptionsrate ("SAR_Fe") und applizierter Feldstärke ("H") betrifft;

◦ Berechnen, basierend auf der Referenz-Leistungsabsorptionsrate ("SAR_Fe") und einer berechneten Masse der Nanopartikel im Depotvolumen, einer mittleren Leistungsabsorptionsdichte ("SAR_aver");

◦ Berechnen eines leistungsabsorptionsbasierten Skalierungsfaktors ("K") basierend auf der jeweiligen mittleren Leistungsabsorptionsdichte ("SAR_aver") und der relativen Leistungsabsorptionsdichte ("ΔSAR_aver");

∘ Berechnen einer jeweiligen Temperaturverteilung ("T(x,y,z)") basierend auf der basalen Temperaturverteilung ("T0(x,y,z)"), der relativen Temperaturinkrementverteilung ("ΔT(x,y,z)"), und dem leistungsabsorptionsbasierten Skalierungsfaktor ("K");

- Bereitstellen der berechneten Temperaturverteilungen zur Unterstützung des Anwenders bei der Planung der Thermotherapie.

13. Computerprogramm zur Durchführung des Verfahrens nach einem der vorhergehenden Ansprüche, wenn das Computerprogramm auf einer programmierbaren Computereinrichtung ausgeführt wird.

14. Datenträger, auf dem das Computerprogramm nach Anspruch 13 aufgezeichnet ist.

15. Computereinrichtung ausgebildet für ein computergestütztes Simulationsverfahren nach einem der Ansprüche 1 bis 9,
wobei die Computereinrichtung die folgenden Komponenten umfasst:

- eine erste Berechnungskomponente ("sim_t.exe", "mainsubroutine_sim_t_voxe!_win") ausgebildet zum Berechnen ("T-Vorgabe") eines am Applikator einzustellenden Feldstärkewertes basierend auf einer geometrischen Verteilung der Nanopartikel und mindestens einem vorgegebenen Temperaturgrenzwert, der durch die Hyperthermie-Behandlung nicht überschritten werden soll;
- eine zweite Berechnungskomponente ("sim_hr.exe", "mainsubroutine_sim_hr_voxel win") ausgebildet zum optionalen Berechnen ("H-Regler, schneller H-Regler") einer zu erwartenden Temperaturverteilung für mindestens einen Teil des Körpervolumens für

∘ jeden Feldstärkewert einer Mehrzahl vorgegebener Feldstärkewerte, und/oder
∘ einen anwenderdefinierten Feldstärkewert; und

- eine Komponente zum Bereitstellen des berechneten Feldstärkewertes und optional mindestens einer berechneten Temperaturverteilung zur Unterstützung des Anwenders bei der Planung der Thermotherapie.

16. Computereinrichtung ausgebildet für ein computergestütztes Simulationsverfahren nach Anspruch 10 ("T-Vorgabe"), wobei die Computereinrichtung eine Komponente ("sim_t.exe", "mainsubroutine_sim_t_voxel_win") aufweist, die ausgebildet ist zur Berechnung einer am Applikator einzustellenden Feldstärke basierend auf einer geometrischen Verteilung der Nanopartikel und mindestens einem vorgegebenen Temperaturgrenzwert, der durch die Hyperthermie-Behandlung nicht überschritten werden soll;
und wobei die Komponente ("sim_t.exe", "mainsubroutine_sim_t_voxel_win") ein Modul zum Berechnen des Feldstärkewertes basierend auf einer vorgegebenen Kennlinie aufweist, wobei die Kennlinie einen Zusammenhang zwischen Leistungsabsorptionsrate und Feldstärke angibt, wobei vorzugsweise die Computereinrichtung die folgenden Module aufweist ("T-Vorgabe"):

- ein Modul zum Berechnen einer mittleren Leistungsabsorptionsdichte im Applikator-Magnetfeld im Depotvolumen, wobei eine relative Leistungsabsorptionsdichte basierend auf einer gemessenen geometrischen Verteilung der Nanopartikel berechnet wird, eine das Modell beschreibende Bio-Heat-Transfer-Gleichung genau einmal zum Erhalt einer basalen Temperaturverteilung ohne Leistungsabsorption numerisch gelöst wird, und die Bio-Heat-Transfer-Gleichung genau einmal zum Erhalt einer relativen Temperaturinkrementverteilung basierend auf der relativen Leistungsabsorptionsdichte numerisch gelöst wird; und wobei die relative Leistungsabsorptionsdichte durch einen Skalierungsfaktor skaliert wird, der basierend auf dem mindestens einen vorgegebenen Temperaturgrenzwert, der basalen Temperaturverteilung und der relativen Temperaturinkrementverteilung erhalten wird ("temperaturbasierter Skalierungsfaktor");
- ein Modul zum Berechnen, basierend auf der berechneten mittleren Leistungsabsorptionsdichte und einer berechneten Masse der Nanopartikel, einer Referenz-Leistungsabsorptionsrate, die zum Beispiel die spezifische Leistungsabsorptionsrate einer die Nanopartikel enthaltenden, unverdünnten Magnetflüssigkeit angibt;
- ein Modul zum Berechnen eines Feldstärkewertes basierend auf der berechneten Referenz-Leistungsabsorptionsrate und einer vorgegebenen Kennlinie, die einen Zusammenhang zwischen Referenz-Leistungsabsorptionsrate und applizierter Feldstärke betrifft;
- ein Modul zum Bereitstellen des berechneten Feldstärkewertes zur Unterstützung des Anwenders bei der Planung der Thermotherapie,
- optional ein Modul zum Berechnen einer jeweiligen Temperaturverteilung basierend auf der basalen Temperaturverteilung, der relativen Temperaturinkrementverteilung, und dem temperaturbasierten Skalierungsfaktor; und

- optional ein Modul zum Bereitstellen der berechneten Temperaturverteilungen zur Unterstützung des Anwenders bei der Planung der Thermotherapie.

17. Computereinrichtung ausgebildet für ein computergestütztes Simulationsverfahren nach Anspruch 11 ("H-Regler", "schneller H-Regler"), wobei die Computereinrichtung eine Komponente ("sim_hr.exe", "mainsubroutine_sim_hr_voxel_win") aufweist, die ausgebildet ist zur Berechnung, für jeden Feldstärkewert einer Mehrzahl vorgegebener Feldstärkewerte und/oder einen anwenderdefinierten Feldstärkewert, einer zu erwartenden Temperaturverteilung für mindestens einen Teil des Körpervolumens; und wobei die Komponente ("sim_hr.exe", "mainsubroutine_sim_hr_voxel_win") ein Modul zum Berechnen der zu erwartenden Temperaturverteilung mittels einer leistungsabsorptionsbasierten Skalierung ("K") einer berechneten oder bereit gestellten Temperaturinkrementverteilung ("$\Delta T(x,y,z)$") aufweist, wobei vorzugsweise

a) die Komponente ("mainsubroutine_sim_hr_voxel_win") zum Berechnen der zu erwartenden Temperaturverteilung ausgebildet ist, um unabhängig von der Zahl der vorgegebenen und/oder anwenderdefinierten Feldstärkewerte eine bereit gestellte (zuvor in mainsubroutine_sim_t_voxel_win berechnete) basale Temperaturverteilung ("T0(x,y,z)") und/oder eine bereit gestellte (zuvor in mainsubroutine_sim_t_voxel_win berechnete) relative Temperaturinkrementverteilung ("$\Delta T(x,y,z)$") heranzuziehen ("schneller H-Regler"), oder

b) die Komponente ("sim_hr.exe") zum Berechnen der zu erwartenden Temperaturverteilung ausgebildet ist, unabhängig von der Zahl der vorgegebenen und/oder anwenderdefinierten Feldstärkewerte maximal zwei Temperaturverteilungen zu berechnen ("H-Regler"), nämlich eine basale Temperaturverteilung ("T0(x,y,z)") und/oder eine relative Temperaturinkrementverteilung ("$\Delta T(x,y,z)$").

18. Computereinrichtung nach Anspruch 17, wobei die Computereinrichtung die folgenden Module aufweist ("H-Regler, "schneller H-Regler"):

- ein Modul zum Berechnen einer relativen Leistungsabsorptionsdichteverteilung und einer relativen mittleren Leistungsabsorptionsdichte basierend auf einer gemessenen geometrischen Verteilung der Nanopartikel;
- ein Modul zum Bereitstellen einer basalen Temperaturverteilung basierend auf einer numerischen Lösung einer das Modell beschreibenden Bio-Heat-Transfer-Gleichung ohne Leistungsabsorption, und Bereitstellen einer relativen Temperaturinkrementverteilung basierend auf einer numerischen Lösung der Bio-Heat-Transfer-Gleichung mit der berechneten relativen Leistungsabsorptionsdichteverteilung;
- ein Modul zum Durchführen der folgenden Schritte für jeden Feldstärkewert der Mehrzahl vorgegebener Feldstärkewerte und/oder den benutzerdefinierten Feldstärkewert:

◦ Berechnen einer Referenz-Leistungsabsorptionsrate, die zum Beispiel die spezifische Leistungsabsorptionsrate einer die Nanopartikel enthaltenden, unverdünnten Magnetflüssigkeit angibt, wobei die Berechnung auf dem jeweiligen Feldstärkewert und einer vorgegebenen Kennlinie basiert, die einen Zusammenhang zwischen Referenz-Leistungsabsorptionsrate und applizierter Feldstärke betrifft;
◦ Berechnen, basierend auf der Referenz-Leistungsabsorptionsrate und einer berechneten Masse der Nanopartikel im Depotvolumen, einer mittleren Leistungsabsorptionsdichte;
◦ Berechnen eines leistungsabsorptionsbasierten Skalierungsfaktors basierend auf der jeweiligen mittleren Leistungsabsorptionsdichte und der relativen Leistungsabsorptionsdichte;
◦ Berechnen einer jeweiligen Temperaturverteilung basierend auf der basalen Temperaturverteilung, der relativen Temperaturinkrementverteilung, und dem leistungsabsorptionsbasierten Skalierungsfaktor;

- ein Modul zum Bereitstellen der berechneten Temperaturverteilungen zur Unterstützung des Anwenders bei der Planung der Thermotherapie.

19. System, umfassend eine Computereinrichtung nach einem der Ansprüche 15 bis 18, und einen Magnetfeld-Applikator.

20. System, umfassend eine Magnetflüssigkeit enthaltend magnetische Nanopartikel und

a) ein Computerprogramm nach Anspruch 13, oder
b) einen Datenträger nach Anspruch 14, oder

c) eine Computereinrichtung nach einem der Ansprüche 15 bis 18, oder

d) eine Computereinrichtung nach einem der Ansprüche 15 bis 18 und einen Magnetfeld-Applikator.

**Claims**

1. A computer-aided simulation method for providing assistance in thermotherapy planning,
   wherein the thermotherapy comprises hyperthermia treatment of a tumor volume in a body volume of a human body,
   wherein the hyperthermia treatment comprises the application of a magnetic field in a treatment volume by means of a magnetic field applicator,
   wherein thermal energy can be introduced into at least one deposit volume by power absorption in the applied magnetic field by means of magnetic, paramagnetic and/or superparamagnetic nanoparticles deposited in the body,
   and wherein the method comprises the following steps:

   - in a first calculation step ("T selection"), calculation of a field strength value that needs to be set on the applicator on the basis of a geometric distribution of the nanoparticles and at least one prescribed temperature limit value which is not meant to be exceeded by the hyperthermia treatment, wherein the field strength value is calculated in the first calculation step ("T selection") on the basis of a prescribed characteristic curve which indicates a relationship between (reference) power absorption rate and field strength; and
   - provision of the calculated field strength value and optionally of at least one calculated temperature distribution for the purpose of supporting the user in planning the thermotherapy.

2. The method as claimed in claim 1, wherein the method comprises the following further step after the first calculation step:

   - in a second calculation step ("H controller", "fast H controller"), calculation of a temperature distribution that is to be expected for at least part of the body volume for

     ◦ each field strength value from a plurality of prescribed field strength values, and/or
     ◦ a user-defined field strength value.

3. The method as claimed in claim 1 or 2, wherein
   the temperature limit value or one of a plurality of temperature limit values relates to a maximum temperature only within the treatment volume that is to be heated, preferably wherein the temperature limit value relates to a prescribed temperature maximum in a range from 60°C to 100°C, preferably 70°C to 90°C, particularly 80°C, in the treatment volume, and/or
   the temperature limit value or one of a plurality of temperature limit values relates to a maximum temperature outside the treatment volume that is to be heated, preferably wherein the temperature limit value relates to a prescribed temperature maximum in a range from 40°C to 45°C, particularly 43°C, outside the treatment volume.

4. The method as claimed in claim 2 or 3, wherein

   a) no temperature limit value is used in the calculations in the second calculation step ("H controller", "fast H controller"), or
   b) the calculations in the second calculation step ("H controller") are performed for a plurality of prescribed field strength values that can be set on the applicator, preferably between 3 kA/m and 20 kA/m, particularly preferably between 5 kA/m and 10 kA/m, or
   c) the second calculation step ("H controller", "fast H controller") is initiated after the first ("T selection") calculation step and possibly a third ("H selection") calculation step by a user input.

5. The method as claimed in one of claims 1 to 3, wherein

   a) the calculation result from the first calculation step ("T selection") is taken as a basis for automatically performing a third calculation step ("H selection") when the field strength value calculated in the first calculation step ("T selection") is greater than a prescribed maximum field strength value, particularly a maximum settable field strength value on the applicator, wherein a temperature distribution that is to be expected is calculated in the third calculation step ("H selection") for the prescribed maximum field strength value, or
   b) no temperature limit value is used in the calculations in the third calculation step ("H selection"), or

c) the calculation of the field strength value that is to be set in the first calculation step ("T selection") does not comprise an iteration in which temperature distributions are calculated from chosen field strength values so as to iteratively arrive at the sought field strength value.

6. The method as claimed in claim 1 or 2, wherein the first calculation step ("T selection") has the following steps:

- calculation of an average power absorption density ("SAR_aver") in the applicator magnetic field in the deposit volume, wherein a relative power absorption density ("$\Delta$SAR(x,y,z)") is calculated on the basis of a measured geometric distribution of the nanoparticles, a bioheat transfer equation describing the model is numerically solved precisely once in order to obtain a basal temperature distribution ("T0(x,y,z)") without power absorption, and the bioheat transfer equation is numerically solved precisely once in order to obtain a relative temperature increment distribution ("$\Delta$T(x,y,z)") on the basis of the relative power absorption density; and wherein the relative power absorption density ("$\Delta$SAR(x,y,z)") is scaled by a temperature-based scaling factor ("K") which is obtained on the basis of the at least one prescribed temperature limit value, the basal temperature distribution and the relative temperature increment distribution;
- calculation, on the basis of the calculated average power absorption density and the calculated mass of the nanoparticles, of a reference power absorption rate ("SAR_Fe") which indicates the specific power absorption rate of an undiluted magnetic fluid containing the nanoparticles, for example;
- calculation of a field strength value ("H") on the basis of the calculated reference power absorption rate ("SAR_Fe") and a prescribed characteristic curve which relates to a relationship between reference power absorption rate and applied field strength ("H"); and
- optional calculation of a respective temperature distribution ("T(x,y,z)") on the basis of the basal temperature distribution ("T0(x,y,z)"), the relative temperature increment distribution ("$\Delta$T(x,y,z)") and the temperature-based scaling factor ("K");

wherein the basal temperature distribution ("T0(x,y,z)") and/or the relative temperature increment distribution ("$\Delta$T(x,y,z)") are provided for at least one further use ("fast H controller") beyond the first calculation step.

7. The method as claimed in one of claims 2 to 6, wherein

a) in the second calculation step ("fast H controller"), regardless of the number of prescribed and/or user-defined field strength values, a provided (previously calculated in the first calculation step) basal temperature distribution ("T0(x,y,z)") and/or a provided (previously calculated in the first calculation step) relative temperature increment distribution ("$\Delta$T(x,y,z)") are used, or

b) in the second calculation step ("H controller"), regardless of the number of prescribed and/or user-defined field strength values, no more than two temperature distributions are calculated, namely a basal temperature distribution ("T0(x,y,z)") and/or a relative temperature increment distribution ("$\Delta$T(x,y,z)").

8. The method as claimed in one of claims 2 to 7, wherein in the second calculation step (H controller", "fast H controller"), the temperature distribution that is to be expected is calculated by means of power-absorption-based scaling ("K") of a calculated or provided relative temperature increment distribution ("$\Delta$T(x,y,z)").

9. The method as claimed in one of claims 7 and 8, wherein the second calculation step ("H controller", "fast H controller") has the following steps:

- calculation of a relative power absorption density distribution ("$\Delta$SAR(x,y,z)") and a relative average power absorption density ("$\Delta$SAR_aver") on the basis of a measured geometric distribution of the nanoparticles;
- provision of a basal temperature distribution ("T0(x,y,z)") on the basis of a numerical solution to a bioheat transfer equation describing the model without power absorption, and provision of a relative temperature increment distribution ("$\Delta$T(x,y,z)") on the basis of a numerical solution to the bioheat transfer equation with the calculated relative power absorption density distribution ("$\Delta$SAR(x,y,z)");
- performance of the following steps for each field strength value from the plurality of prescribed field strength values and/or the user-defined field strength value:

  ◦ calculation of a reference power absorption rate ("SAR_Fe") which indicates the specific power absorption rate of an undiluted magnetic fluid containing the nanoparticles, for example, wherein the calculation is based on the respective field strength value ("H") and a prescribed characteristic curve which relates to a relationship between reference power absorption rate ("SAR_Fe") and applied field strength ("H");

∘ calculation, on the basis of the reference power absorption rate ("SAR_Fe") and the calculated mass of the nanoparticles in the deposit volume, of an average power absorption density ("SAR_aver");
∘ calculation of a power-absorption-based scaling factor ("K") on the basis of the respective average power absorption density ("SAR_aver") and the relative average power absorption density ("ΔSAR aver");
∘ calculation of a respective temperature distribution ("T(x,y,z)") on the basis of the basal temperature distribution ("T0(x,y,z)"), the relative temperature increment distribution ("ΔT(x,y,z)") and the power-absorption-based scaling factor ("K").

10. The method as claimed in claim 1 ("T selection"), wherein the method has the following steps ("T selection"):

- calculation of an average power absorption density ("SAR_aver") in the applicator magnetic field in the deposit volume, wherein a relative power absorption density ("ΔSAR(x,y,z)") is calculated on the basis of a measured geometric distribution of the nanoparticles, a bioheat transfer equation describing the model is numerically solved precisely once in order to obtain a basal temperature distribution ("T0(x,y,z)") without power absorption, and the bioheat transfer equation is numerically solved precisely once in order to obtain a relative temperature increment distribution ("ΔT(x,y,z)") on the basis of the relative power absorption density; and wherein the relative power absorption density ("ΔSAR(x,y,z)") is scaled by a temperature-based scaling factor ("K") which is obtained on the basis of the at least one prescribed temperature limit value, the basal temperature distribution and the relative temperature increment distribution;
- calculation, on the basis of the calculated average power absorption density and the calculated mass of the nanoparticles, of a reference power absorption rate ("SAR_Fe") which indicates the specific power absorption rate of an undiluted magnetic fluid containing the nanoparticles, for example;
- calculation of a field strength value ("H") on the basis of the calculated reference power absorption rate ("SAR_Fe") and a prescribed characteristic curve which relates to a relationship between reference power absorption rate ("SAR_Fe") and applied field strength ("H");
- optional calculation of a respective temperature distribution ("T(x,y,z)") on the basis of the basal temperature distribution ("T0(x,y,z)"), the relative temperature increment distribution ("ΔT(x,y,z)") and the temperature-based scaling factor ("K");
- provision of the calculated field strength value ("H") in order to provide assistance for the user in planning the thermotherapy; and
- optional provision of the calculated temperature distribution ("T(x,y,z)") in order to provide assistance for the user in planning the thermotherapy.

11. A computer-aided simulation method ("H controller", "fast H controller") for providing assistance in thermotherapy planning,
wherein the thermotherapy comprises hyperthermia treatment of a tumor volume in a body volume of a human body,
wherein the hyperthermia treatment comprises the application of a magnetic field in a treatment volume by means of a magnetic field applicator,
wherein thermal energy can be introduced into at least one deposit volume by power absorption in the applied magnetic field by means of magnetic, paramagnetic and/or superparamagnetic nanoparticles deposited in the body,
wherein the method relates to the calculation, for each field strength value from a plurality of prescribed field strength values and/or a user-defined field strength value, of a temperature distribution that is to be expected for at least some of the body volume;
and wherein the temperature distribution that is to be expected is calculated by means of power-absorption-based scaling ("K") of a calculated or provided relative temperature increment distribution ("ΔT(x,y,z)"), preferably wherein

a) regardless of the number of prescribed and/or user-defined field strength values, a provided (previously calculated in the T selection step) basal temperature distribution ("T0(x,y,z)") and/or a provided (previously calculated in the T selection step) relative temperature increment distribution ("ΔT(x,y,z)") is/are used ("fast H controller"), or
b) regardless of the number of prescribed and/or user-defined field strength values, no more than two temperature distributions are calculated ("H controller"), namely a basal temperature distribution ("T0(x,y,z)") and/or a relative temperature increment distribution ("ΔT(x,y,z)").

12. The method as claimed in claim 11, wherein the method has the following steps ("H controller", "fast H controller"):

- calculation of a relative power absorption density distribution ("ΔSAR(x,y,z)") and a relative average power absorption density ("ΔSAR aver") on the basis of a measured geometric distribution of the nanoparticles;

- provision of a basal temperature distribution ("T0(x,y,z)") on the basis of a numerical solution to a bioheat transfer equation describing the model without power absorption, and provision of a relative temperature increment distribution ("ΔT(x,y,z)") on the basis of a numerical solution to the bioheat transfer equation with the calculated relative power absorption density ("ΔSAR(x,y,z)");
- performance of the following steps for each field strength value from the plurality of prescribed field strength values and/or the user-defined field strength value:

  ∘ calculation of a reference power absorption rate ("SAR_Fe") which indicates the specific power absorption rate of an undiluted magnetic fluid containing the nanoparticles, for example, wherein the calculation is based on the respective field strength value ("H") and a prescribed characteristic curve which relates to a relationship between reference power absorption rate ("SAR_Fe") and applied field strength ("H");
  ∘ calculation, on the basis of the reference power absorption rate ("SAR_Fe") and the calculated mass of the nanoparticles in the deposit volume, of an average power absorption density ("SAR_aver");
  ∘ calculation of a power-absorption-based scaling factor ("K") on the basis of the respective average power absorption density ("SAR_aver") and the relative power absorption density ("ΔSAR_aver");
  ∘ calculation of a respective temperature distribution ("T(x,y,z)") on the basis of the basal temperature distribution ("T0(x,y,z)"), the relative temperature increment distribution ("ΔT(x,y,z)") and the power-absorption-based scaling factor ("K");

- provision of the calculated temperature distributions in order to provide assistance for the user in planning the thermotherapy.

13. A computer program for carrying out the method as claimed in one of the preceding claims when the computer program is executed on a programmable computer device.

14. A data storage medium on which the computer program as claimed in claim 13 is recorded.

15. A computer device designed for a computer-aided simulation method as claimed in one claims 1 to 9, wherein the computer device comprises the following components:

    - a first calculation component ("sim t.exe", "mainsubroutine_sim_t_voxel win") designed to calculate ("T selection") a field strength value that needs to be set on the applicator on the basis of a geometric distribution of the nanoparticles and at least one prescribed temperature limit value which is not meant to be exceeded by the hyperthermia treatment;
    - a second calculation component ("sim_hr.exe", "mainsubroutine_sim_hr_voxel win"), designed to optionally calculate ("H controller, fast H controller") a temperature distribution that is to be expected for at least some of the body volume for

      o each field strength value from a plurality of prescribed field strength values, and/or
      ∘ a user-defined field strength value; and

    - a component for providing the calculated field strength value and optionally at least one calculated temperature distribution in order to provide assistance for the user in planning the thermotherapy.

16. A computer device designed for a computer-aided simulation method as claimed in claim 10 (T selection"), wherein the computer device has a component ("sim_t.exe", "mainsubroutine_sim_t_voxel win") which is designed to calculate a field strength that needs to be set on the applicator on the basis of a geometric distribution of the nanoparticles and at least one prescribed temperature limit value which is not meant to be exceeded by the hyperthermia treatment; and wherein the component ("sim_t.exe", "mainsubroutine_sim_t_voxel win") has a module for calculating the field strength value on the basis of a prescribed characteristic curve, wherein the characteristic curve indicates a relationship between power absorption rate and field strength, preferably wherein the computer device has the following module ("T selection"):

    - a module for calculating an average power absorption density in the applicator magnetic field in the deposit volume, wherein a relative power absorption density is calculated on the basis of a measured geometric distribution of the nanoparticles, a bioheat transfer equation describing the model is numerically solved precisely once in order to obtain a basal temperature distribution without power absorption, and the bioheat transfer equation is numerically solved precisely once in order to obtain a relative temperature increment distribution on the basis of the relative power absorption density; and wherein the relative power absorption density is scaled by a scaling factor which is obtained on the basis of the at least one prescribed temperature limit value, the basal temperature distribution and the relative temperature increment distribution ("temperature-based scaling factor");

- a module for calculating, on the basis of the calculated average power absorption density and the calculated mass of the nanoparticles, a reference power absorption rate which indicates the specific power absorption rate of an undiluted magnetic fluid containing the nanoparticles, for example;

- a module for calculating a field strength value on the basis of the calculated reference power absorption rate and a prescribed characteristic curve which relates to a relationship between reference power absorption rate and applied field strength;

- a module for providing the calculated field strength value in order to provide assistance for the user in planning the thermotherapy,

- optionally a module for calculating respective temperature distribution on the basis of the basal temperature distribution, the relative temperature increment distribution and the temperature-based scaling factor; and

- optionally a module for providing the calculated temperature distributions in order to provide assistance for the user in planning the thermotherapy.

**17.** A computer device designed for a computer-aided simulation method as claimed in claim 11 ("H controller", "fast H controller"), wherein the computer device has a component ("sim_hr.exe", "mainsubroutine_sim_hr_voxel_win") which is designed to calculate, for each field strength value from a plurality of prescribed field strength values and/or a user-defined field strength value, a temperature distribution that is to be expected for at least some of the body volume;

and wherein the component ("sim_hr.exe", "mainsubroutine_sim_hr_voxel_win") has a module for calculating the temperature distribution that is to be expected by means of power-absorption-based scaling ("K") of a calculated or provided temperature increment distribution ("$\Delta T(x,y,z)$"), preferably wherein

a) the component ("mainsubroutine_sim_hr_voxel_win") is designed to calculate the temperature distribution that is to be expected in order to use, regardless of the number of prescribed and/or user-defined field strength values, a provided (previously calculated in mainsub-routine_sim_t_voxel_win) basal temperature distribution ("T0(x,y,z)") and/or a provided (previously calculated in mainsubroutine_sim_t_voxel win) relative temperature increment distribution ("$\Delta T(x,y,z)$") ("fast H controller"), or

b) the component ("sim_hr.exe") for calculating the temperature distribution that is to be expected is designed to calculate, regardless of the number of prescribed and/or user-defined field strength values, no more than two temperature distributions ("H controller"), namely a basal temperature distribution ("T0(x,y,z)") and/or a relative temperature increment distribution ("$\Delta T(x,y,z)$").

**18.** The computer device as claimed in claim 17, wherein the computer device has the following modules ("H controller", "fast H controller"):

- a module for calculating a relative power absorption density distribution and a relative average power absorption density on the basis of a measured geometric distribution of the nanoparticles;

- a module for providing a basal temperature distribution on the basis of a numerical solution to a bioheat transfer equation describing the model without power absorption, and providing a relative temperature increment distribution on the basis of a numerical solution to the bioheat transfer equation with the calculated relative power absorption density distribution;

- a module for performing the following steps for each field strength value from the plurality of prescribed field strength values and/or the user-defined field strength value:

◦ calculation of a reference power absorption rate which indicates the specific power absorption rate of an undiluted magnetic fluid containing the nanoparticles, for example, wherein the calculation is based on the respective field strength value and a prescribed characteristic curve which relates to a relationship between reference power absorption rate and applied field strength;

◦ calculation, on the basis of the reference power absorption rate and the calculated mass of the nanoparticles in the deposit volume, of an average power absorption density;

◦ calculation of a power-absorption-based scaling factor on the basis of the respective average power absorption density and the relative power absorption density;

◦ calculation of a respective temperature distribution on the basis of the basal temperature distribution, the relative temperature increment distribution and the power-absorption-based scaling factor;

**EP 2 683 440 B1**

- a module for providing the calculated temperature distributions in order to provide assistance for the user in planning the thermotherapy.

**19.** A system, comprising a computer device as claimed in one of claims 15 to 18 and a magnetic field applicator.

**20.** A system, comprising a magnetic fluid containing magnetic nanoparticles and

a) a computer program as claimed in claim 13, or
b) a data storage medium as claimed in claim 14, or
c) a computer device as claimed in one of claims 15 to 18, or
d) a computer device as claimed in one of claims 15 to 18 and a magnetic field applicator.

**Revendications**

**1.** Procédé de simulation assisté par ordinateur, pour aider à la planification d'une thermothérapie,
dans lequel la thermothérapie comporte un traitement par hyperthermie d'un volume tumoral dans un volume corporel d'un corps humain,
dans lequel le traitement par hyperthermie comporte l'application d'un champ magnétique dans un volume de traitement au moyen d'un applicateur de champ magnétique,
dans lequel une énergie thermique peut être introduite par absorption de puissance dans le champ magnétique appliqué dans au moins un volume de dépôt, au moyen de nanoparticules magnétiques, paramagnétiques et/ou super-paramagnétiques, déposées dans le corps,
et dans lequel le procédé comporte les étapes suivantes :

- dans une première étape de calcul ("définition de T"), calcul d'une valeur d'intensité de champ à régler sur l'applicateur, sur la base d'une répartition géométrique des nanoparticules et d'au moins une valeur limite de température prédéfinie qu'il convient de ne pas dépasser dans le traitement par hyperthermie, la valeur d'intensité de champ dans la première étape de calcul ("définition de T") étant calculée sur la base d'une courbe caractéristique prédéfinie qui indique une relation entre le taux d'absorption de puissance (de référence) et l'intensité du champ ; et
- mise à disposition de la valeur d'intensité de champ calculée et, en option, d'au moins une distribution de température calculée pour aider l'utilisateur dans la planification de la thermothérapie.

**2.** Procédé selon la revendication 1, ledit procédé comportant, après la première étape de calcul, l'étape supplémentaire suivants :

- dans une deuxième étape de calcul ("régulateur H", "régulateur H rapide"), calcul d'une distribution de température prévisionnelle pour au moins une partie du volume corporel pour

• chaque valeur d'intensité de champ d'une pluralité de valeurs d'intensité de champ prédéfinies, et /ou
• une valeur d'intensité de champ définie par l'utilisateur.

**3.** Procédé selon la revendication 1 ou 2, dans lequel
la valeur limite de température ou l'une de plusieurs valeurs limites de température concerne une température maximale uniquement à l'intérieur du volume de traitement à chauffer, la valeur limite de température concernant de préférence un maximum de température prédéfini dans une plage de 60°C à 100°C, de préférence 70°C à 90°C, en particulier 80°C, dans le volume de traitement, et/ou
la valeur limite de température ou l'une des valeurs limites de température concerne une température maximale à l'extérieur du volume de traitement à chauffer, la valeur limite de température concernant de préférence un maximum de température prédéfini dans une plage de 40°C à 45°C, en particulier 43°C, à l'extérieur du volume de traitement.

**4.** Procédé selon l'une des revendications 2 ou 3, dans lequel

a) aucune valeur limite de température n'entre dans les calculs à la deuxième étape de calcul ("régulateur H", "régulateur H rapide"), ou
b) les calculs à la deuxième étape de calcul ("régulateur H") sont effectués pour une pluralité de valeurs d'intensité de champ prédéfinies, réglables sur l'applicateur, de préférence entre 3 kA/m et 20 kA/m, encore mieux entre 5 kA/m et 10 kA/m, ou

c) la deuxième étape de calcul ("régulateur H", "régulateur H rapide") est déclenchée par une entrée de l'utilisateur après la première étape de calcul ("définition de T") et, le cas échéant, une troisième étape de calcul ("définition de H").

**5.** Procédé selon l'une quelconque des revendications 1 à 3, dans lequel

a) en fonction du résultat du calcul de la première étape de calcul ("définition de T"), une troisième étape de calcul ("définition de H") est alors effectuée automatiquement lorsque la valeur d'intensité de champ calculée dans la première étape de calcul ("définition de T") est supérieure à une valeur d'intensité de champ maximale prédéfinie, en particulier une valeur d'intensité de champ réglable au maximum sur l'applicateur, dans lequel une distribution de température prévisionnelle est calculée dans la troisième étape de calcul ("définition de H") pour la valeur d'intensité de champ maximale prédéfinie, ou

b) aucune valeur limite de température n'entre dans les calculs à la troisième étape de calcul ("définition de H"), ou

c) le calcul de la valeur d'intensité de champ à régler, à la première étape de calcul ("définition de T"), ne comporte pas d'itération, dans laquelle des distributions de température sont calculées à partir des valeurs d'intensité de champ sélectionnées, afin de parvenir ainsi de manière itérative à la valeur d'intensité de champ cherchée.

**6.** Procédé selon la revendication 1 ou 2, dans lequel la première étape de calcul ("définition de T") comporte les étapes suivantes :

- calcul d'une densité d'absorption de puissance moyenne ("SAR_aver") dans le champ magnétique de l'applicateur à l'intérieur du volume de dépôt, dans lequel une densité d'absorption de puissance relative ("$\Delta$SAR(x,y,z)") est calculée sur la base d'une répartition géométrique mesurée des nanoparticules, une équation de transfert bio-thermique, définissant le modèle, étant résolue numériquement exactement une fois pour obtenir une distribution de la température basale ("T0(x,y,z)") sans absorption de puissance, et l'équation de transfert bio-thermique étant résolue numériquement exactement une fois pour obtenir une distribution incrémentale relative de la température ("$\Delta$T(x,y,z)") sur la base de la densité d'absorption de puissance relative ; et dans lequel la densité d'absorption de puissance relative ("$\Delta$SAR(x,y,z)") est mise à l'échelle au moyen d'un facteur d'échelle ("K") basé sur la température, lequel est obtenu sur la base de ladite au moins une valeur limite de température prédéfinie, de la distribution de la température basale et de la distribution incrémentale relative de la température ;

- calcul, basé sur la densité d'absorption de puissance moyenne calculée et sur une masse calculée des nanoparticules, d'un taux d'absorption de puissance de référence ("SAR_Fe") qui indique, par exemple, le taux d'absorption de puissance spécifique d'un liquide magnétique non dilué contenant les nanoparticules ;

- calcul d'une valeur d'intensité de champ ("H") sur la base du taux d'absorption de puissance de référence ("SAR_Fe") calculé et d'une courbe caractéristique prédéfinie qui concerne une relation entre le taux d'absorption de puissance de référence et l'intensité de champ ("H") appliquée ; et

- en option, calcul d'une distribution de température ("T(x,y,z)") respective sur la base de la distribution de la température basale ("T0(x,y,z)"), de la distribution incrémentale relative de la température ("$\Delta$T(x,y,z)") et du facteur d'échelle ("K") basé sur la température ;

dans lequel, de préférence, la distribution de la température basale ("T0(x,y,z)") et/ou la distribution incrémentale relative de la température ("$\Delta$T(x,y,z)") est mise à disposition pour au moins une autre utilisation ("régulateur H rapide") en plus de la première étape de calcul.

**7.** Procédé selon l'une quelconque des revendications 2 à 6, dans lequel

a) dans la deuxième étape de calcul ("régulateur H rapide"), indépendamment du nombre de valeurs d'intensité de champ prédéfinies et/ou définies par l'utilisateur, est utilisée une distribution de la température basale ("T0(x,y,z)") mise à disposition (calculée précédemment à la première étape de calcul) et/ou une distribution incrémentale relative de la température ("$\Delta$T(x,y,z)") mise à disposition (calculée précédemment à la première étape de calcul), ou

b) dans la deuxième étape de calcul ("régulateur H"), indépendamment du nombre de valeurs d'intensité de champ prédéfinies et/ou définies par l'utilisateur, sont calculées au maximum deux distributions de température, à savoir une distribution de la température basale ("T0(x,y,z)") et/ou une distribution incrémentale relative de la température ("ΔT(x,y,z)").

8. Procédé selon l'une quelconque des revendications 2 à 7, dans lequel dans la deuxième étape de calcul ("régulateur H", "régulateur H rapide"), la distribution de température prévisionnelle est calculée au moyen d'une mise à l'échelle ("K"), basée sur l'absorption de puissance, d'une distribution incrémentale relative de la température ("0T(x,y,z)") calculée ou mise à disposition.

9. Procédé selon l'une des revendications 7 et 8, dans lequel la deuxième étape de calcul ("régulateur H", "régulateur H rapide") comporte les étapes suivantes :

   - calcul d'une répartition relative de la densité d'absorption de puissance ("ΔSAR(x,y,z)") et d'une densité d'absorption de puissance moyenne relative ("ΔSAR_aver") sur la base d'une répartition géométrique mesurée des nanoparticules ;
   - mise à disposition d'une distribution de la température basale ("T0(x,y,z)") sur la base d'une résolution numérique d'une équation de transfert bio-thermique, définissant le modèle, sans absorption de puissance, et mise à disposition d'une distribution incrémentale relative de la température ("ΔT(x,y,z)") sur la base d'une résolution numérique de l'équation de transfert bio-thermique avec la répartition relative de la densité d'absorption de puissance ("ΔSAR(x,y,z)") calculée ;
   - mise en oeuvre des étapes suivantes pour chaque valeur d'intensité de champ de la pluralité de valeurs d'intensité de champ prédéfinies et/ou pour la valeur d'intensité de champ définie par l'utilisateur :

      • calcul d'un taux d'absorption de puissance de référence ("SAR_Fe") qui indique, par exemple, le taux d'absorption de puissance spécifique d'un liquide magnétique non dilué contenant les nanoparticules, le calcul étant basé sur la valeur d'intensité de champ ("H") respective et sur une courbe caractéristique prédéfinie, qui concerne une relation entre le taux d'absorption de puissance de référence ("SAR_Fe") et l'intensité de champ ("H") appliquée ;
      • calcul d'une densité d'absorption de puissance moyenne ("SAR_aver"), basé sur le taux d'absorption de puissance de référence ("SAR_Fe") et sur une masse calculée des nanoparticules dans le volume de dépôt ;
      • calcul d'un facteur d'échelle ("K") basé sur l'absorption de puissance, sur la base de la densité d'absorption de puissance moyenne ("SAR_aver") respective et la densité d'absorption de puissance moyenne relative ("ΔSAR_aver") ;
      • calcul d'une distribution de température ("T(x,y,z)") respective sur la base de la distribution de la température basale ("T0(x,y,z)"), de la distribution incrémentale relative de la température ("ΔT(x,y,z)") et du facteur d'échelle ("K") basé sur l'absorption de puissance.

10. Procédé selon la revendication 1 ("définition de T"), ledit procédé comportant, en outre, les étapes suivantes ("définition de T") :

   - calcul d'une densité d'absorption de puissance moyenne ("SAR_aver") dans le champ magnétique de l'applicateur à l'intérieur du volume de dépôt, une densité d'absorption de puissance relative ("ΔSAR(x,y,z)") étant calculée sur la base de la répartition géométrique mesurée des nanoparticules, une équation de transfert bio-thermique, définissant le modèle, étant résolue numériquement exactement une fois pour obtenir une distribution de la température basale ("T0(x,y,z)") sans absorption de puissance, et l'équation de transfert bio-thermique étant résolue numériquement exactement une fois pour obtenir une distribution incrémentale relative de la température ("ΔT(x,y,z)") sur la base de la densité d'absorption de puissance relative ; et la densité d'absorption de puissance relative ("ΔSAR(x,y,z)") étant mise à l'échelle au moyen d'un facteur d'échelle ("K") basé sur la température, lequel est obtenu sur la base de ladite au moins une valeur limite de température prédéfinie, de la distribution de la température basale et de la distribution incrémentale relative de la température ;
   - calcul, basé sur la densité d'absorption de puissance moyenne calculée et sur une masse calculée des nanoparticules, d'un taux d'absorption de puissance de référence ("SAR_Fe") qui indique, par exemple, le taux d'absorption de puissance spécifique d'un liquide magnétique non dilué contenant les nanoparticules ;
   - calcul d'une valeur d'intensité de champ ("H") sur la base du taux d'absorption de puissance de référence ("SAR_Fe") calculé et d'une courbe caractéristique prédéfinie qui concerne une relation entre le taux d'absorption de puissance de référence ("SAR_Fe") et l'intensité de champ ("H") appliquée ;
   - en option, calcul d'une distribution de température ("T(x,y,z)") respective sur la base de la distribution de la température basale ("T0(x,y,z)"), de la distribution incrémentale relative de la température ("ΔT(x,y,z)") et du facteur d'échelle ("K") basé sur la température ;
   - mise à disposition de la valeur d'intensité de champ ("H") calculée pour aider l'utilisateur dans la planification de la thermothérapie ; et

- en option, mise à disposition de la distribution de température ("T(x,y,z)") calculée pour aider l'utilisateur dans la planification de la thermothérapie.

**11.** Procédé de simulation assisté par ordinateur ("régulateur H", "régulateur H rapide"), pour aider à la planification d'une thermothérapie,

dans lequel la thermothérapie comporte un traitement par hyperthermie d'un volume tumoral dans un volume corporel d'un corps humain,

dans lequel le traitement par hyperthermie comporte l'application d'un champ magnétique dans un volume de traitement au moyen d'un applicateur de champ magnétique,

dans lequel une énergie thermique peut être introduite par absorption de puissance dans le champ magnétique appliqué dans au moins un volume de dépôt, au moyen de nanoparticules magnétiques, paramagnétiques et/ou super-paramagnétiques, déposées dans le corps,

le procédé concernant, pour chaque valeur d'intensité de champ d'une pluralité de valeurs d'intensité de champ et/ou pour une valeur d'intensité de champ définie par l'utilisateur, le calcul d'une distribution de température prévisionnelle pour au moins une partie du volume corporel ;

et la distribution de température prévisionnelle étant calculée au moyen d'un facteur d'échelle ("K"), basé sur l'absorption de puissance, d'une distribution incrémentale relative de la température ("ΔT(x,y,z)") calculée ou mise à disposition, dans lequel, de préférence,

a) indépendamment du nombre de valeurs d'intensité de champ prédéfinies'et/ou définies par l'utilisateur, est utilisée ("régulateur H rapide") une distribution de la température basale ("T0(x,y,z)") mise à disposition (calculée précédemment dans l'étape de la définition de T) et/ou une distribution incrémentale relative de la température ("ΔT(x,y,z)") mise à disposition (calculée précédemment dans l'étape de la définition de T), ou

b) indépendamment du nombre de valeurs d'intensité de champ prédéfinies et/ou définies par l'utilisateur, sont calculées ("régulateur H") au maximum deux distributions de température, à savoir une distribution de la température basale ("T0(x,y,z)") et/ou une distribution incrémentale relative de la température ("ΔT(x,y,z)").

**12.** Procédé selon la revendication 11, dans lequel le procédé comporte les étapes suivantes ("régulateur H", "régulateur H rapide") :

- calcul d'une répartition relative de densité d'absorption de puissance ("ΔSAR(x,y,z)") et d'une densité d'absorption de puissance moyenne relative ("ΔSAR_aver") sur la base d'une répartition géométrique mesurée des nanoparticules ;

- mise à disposition d'une distribution de la température basale ("T0(x,y,z)") sur la base d'une résolution numérique d'une équation de transfert bio-thermique, définissant le modèle, sans absorption de puissance, et mise à disposition d'une distribution incrémentale relative de la température ("ΔT(x,y,z)") sur la base d'une résolution numérique de l'équation de transfert bio-thermique avec la densité d'absorption de puissance relative ("ΔSAR(x,y,z)") calculée ;

- mise en oeuvre des étapes suivantes pour chaque valeur d'intensité de champ de la pluralité de valeurs d'intensité de champ prédéfinies et/ou pour la valeur d'intensité de champ définie par l'utilisateur :

• calcul d'un taux d'absorption de puissance de référence ("SAR_Fe") qui indique, par exemple, le taux d'absorption de puissance spécifique d'un liquide magnétique non dilué contenant les nanoparticules, le calcul étant basé sur la valeur d'intensité de champ ("H") respective et sur une courbe caractéristique prédéfinie, qui concerne une relation entre le taux d'absorption de puissance de référence ("SAR_Fe") et l'intensité de champ ("H") appliquée ;

• calcul d'une densité d'absorption de puissance moyenne ("SAR_aver"), sur la base du taux d'absorption de puissance de référence ("SAR_Fe") et d'une masse calculée des nanoparticules dans le volume de dépôt ;

• calcul d'un facteur d'échelle ("K") basé sur l'absorption de puissance, sur la base de la densité d'absorption de puissance moyenne ("SAR_aver") respective et de la densité d'absorption de puissance relative ("ΔSAR_aver") ;

• calcul d'une distribution de température ("T(x,y,z)") respective sur la base de la distribution de la température basale ("T0(x,y,z)"), de la distribution incrémentale relative de la température ("ΔT(x,y,z)") et du facteur d'échelle ("K") basé sur l'absorption de puissance ;

- mise à disposition des distributions de température calculées pour aider l'utilisateur dans la planification de la thermothérapie.

13. Programme informatique permettant la mise en oeuvre du procédé selon l'une des revendications précédentes, lorsque le programme informatique est exécuté sur une installation informatique programmable.

14. Support de données, sur lequel est enregistré le programme informatique selon la revendication 13.

15. Installation informatique configurée pour un procédé de simulation assisté par ordinateur selon l'une des revendications 1 à 9,
ladite installation informatique comportant les composants suivants :

- un premier composant de calcul ("sim_t.exe", "mainsubroutine_sim_t_voxel_win") configuré pour calculer ("définition de T") une valeur d'intensité de champ à régler sur l'applicateur, sur la base d'une répartition géométrique des nanoparticules et d'au moins une valeur limite de température prédéfinie qu'il convient de ne pas dépasser lors du traitement par hyperthermie ;
- un deuxième composant de calcul ("sim_hr.exe", "mainsubroutine_sim_hr_voxel_win") configuré pour calculer en option ("régulateur H", "régulateur H rapide") une distribution de température prévisionnelle pour au moins une partie du volume corporel pour

  • chaque valeur d'intensité de champ d'une pluralité de valeurs d'intensité de champ prédéfinies, et/ou
  • une valeur d'intensité de champ définie par l'utilisateur ; et

- un composant destiné à mettre à disposition la valeur d'intensité de champ calculée et, en option, au moins une distribution de température calculée pour aider l'utilisateur dans la planification de la thermothérapie.

16. Installation informatique configurée pour un procédé de simulation assisté par ordinateur selon la revendication 10 ("définition de T"), ladite installation informatique comportant un composant ("sim_t.exe", "mainsubroutine_sim_t_voxel_win") qui est configuré pour calculer une valeur d'intensité de champ à régler sur l'applicateur, sur la base d'une répartition géométrique des nanoparticules et d'au moins une valeur limite de température prédéfinie qu'il convient de ne pas dépasser lors du traitement par hyperthermie ;
et le composant ("sim_t.exe", "mainsubroutine_sim_t_voxel_win") comportant un module destiné à calculer la valeur d'intensité de champ sur la base d'une courbe caractéristique prédéfinie, ladite courbe caractéristique indiquant une relation entre le taux d'absorption de puissance et l'intensité de champ, l'installation informatique comportant de préférence les modules suivants ("définition de T") :

- un module pour calculer une densité d'absorption de puissance moyenne dans le champ magnétique de l'applicateur à l'intérieur du volume de dépôt, une densité d'absorption de puissance relative étant calculée sur la base d'une répartition géométrique mesurée des nanoparticules, une équation de transfert bio-thermique, définissant le modèle, étant résolue numériquement exactement une fois pour obtenir une distribution de la température basale sans absorption de puissance, et l'équation de transfert bio-thermique étant résolue numériquement exactement une fois sur la base de la densité d'absorption de puissance relative pour obtenir une distribution incrémentale relative de la température ; et la densité d'absorption de puissance relative étant mise à l'échelle au moyen d'un facteur d'échelle, qui est obtenu sur la base de ladite au moins une valeur limite de température prédéfinie, de la distribution de la température basale et de la distribution incrémentale relative de la température ("facteur d'échelle basé sur la température") ;
- un module pour calculer, sur la base de la densité d'absorption de puissance moyenne calculée et d'une masse calculée des nanoparticules, un taux d'absorption de puissance de référence qui indique, par exemple, le taux d'absorption de puissance spécifique d'un liquide magnétique non dilué contenant les nanoparticules ;
- un module pour calculer une valeur d'intensité de champ sur la base du taux d'absorption de puissance de référence calculé et d'une courbe caractéristique prédéfinie qui concerne une relation entre le taux d'absorption de puissance de référence et l'intensité de champ appliquée ;
- un module pour mettre à disposition la valeur d'intensité de champ calculée, afin d'aider l'utilisateur dans la planification de la thermothérapie,
- en option, un module pour calculer une distribution de température respective sur la base de la distribution de la température basale, de la distribution incrémentale relative de la température et du facteur d'échelle basé sur la température ; et
- en option, un module pour mettre à disposition les distributions de température calculées, afin d'aider l'utilisateur dans la planification de la thermothérapie.

17. Installation informatique configurée pour un procédé de simulation assisté par ordinateur selon la revendication 11 ("régulateur H", "régulateur H rapide"), ladite installation informatique comportant un composant ("sim_hr.exe", "mainsubroutine_sim_hr_voxel_win") qui est configuré pour calculer pour chaque valeur d'intensité de champ d'une pluralité de valeurs d'intensité de champ et/ou pour une valeur d'intensité de champ définie par l'utilisateur, une distribution de température prévisionnelle pour au moins une partie du volume corporel ;
et le composant ("sim_hr.exe", "mainsubroutine_sim_hr_voxel_win") comportant un module pour calculer la distribution de température prévisionnelle au moyen d'un facteur d'échelle ("K"), basé sur l'absorption de puissance, d'une distribution incrémentale de la température ("∆T(x,y,z)") calculée ou mise à disposition,
dans laquelle, de préférence,

a) le composant ("mainsubroutine_sim_hr_voxel_win") est configuré pour calculer la distribution de température à escompter, afin d'utiliser ("régulateur H rapide"), indépendamment du nombre de valeurs d'intensité de champ prédéfinies et/ou définies par l'utilisateur, une distribution de la température basale ("T0(x,y,z)") mise à disposition (calculée précédemment dans mainsubroutine_sim_t_voxel_win) et/ou une distribution incrémentale relative de la température ("∆T(x,y,z)") mise à disposition (calculée précédemment dans mainsubroutine_sim_t_voxel_win), ou
b) le composant ("sim_hr.exe") est configuré pour calculer la distribution de température à escompter, afin de calculer ("régulateur H"), indépendamment du nombre de valeurs d'intensité de champ prédéfinies et/ou définies par l'utilisateur, au maximum deux distributions de température, à savoir une distribution de la température basale ("T0(x,y,z)") et/ou une distribution incrémentale relative de la température ("∆T(x,y,z)").

18. Installation informatique selon la revendication 17, ladite installation informatique comportant les modules suivants ("régulateur H", "régulateur H rapide") :

- un module pour calculer une répartition relative de la densité d'absorption de puissance et une densité d'absorption de puissance moyenne relative sur la base d'une répartition géométrique mesurée des nanoparticules ;
- un module pour mettre à disposition une distribution de la température basale sur la base d'une résolution numérique d'une équation de transfert bio-thermique, définissant le modèle, sans absorption de puissance, et pour mettre à disposition une distribution incrémentale relative de la température sur la base d'une résolution numérique de l'équation de transfert bio-thermique avec la répartition relative de la densité d'absorption de puissance calculée ;
- un module pour la mise en oeuvre des étapes suivantes pour chaque valeur d'intensité de champ de la pluralité de valeurs d'intensité de champ prédéfinies et/ou pour la valeur d'intensité de champ définie par l'utilisateur :

• calcul d'un taux d'absorption de puissance de référence qui indique, par exemple, le taux d'absorption de puissance spécifique d'un liquide magnétique non dilué contenant les nanoparticules, le calcul étant basé sur la valeur d'intensité de champ respective et sur une courbe caractéristique prédéfinie, qui concerne une relation entre le taux d'absorption de puissance de référence et l'intensité de champ appliquée ;
• calcul d'une densité d'absorption de puissance moyenne, sur la base du taux d'absorption de puissance de référence et d'une masse calculée des nanoparticules dans le volume de dépôt ;
• calcul d'un facteur d'échelle basé sur l'absorption de puissance, sur la base de la densité d'absorption de puissance moyenne respective et la densité d'absorption de puissance relative ;
• calcul d'une distribution de température respective sur la base de la distribution de la température basale, de la distribution incrémentale relative de la température et du facteur d'échelle basé sur l'absorption de puissance ;

- un module pour mettre à disposition les distributions de température calculées, afin d'aider l'utilisateur dans la planification de la thermothérapie.

19. Système, comportant une installation informatique selon l'une des revendications 15 à 18 et un applicateur de champ magnétique.

20. Système, comportant un fluide magnétique contenant des nanoparticules magnétiques et

a) un programme informatique selon la revendication 13, ou
b) un support de données selon la revendication 14, ou

c) une installation informatique selon l'une des revendications 15 à 18, ou

d) une installation informatique selon l'une des revendications 15 à 18 et un applicateur de champ magnétique.

PATIENTENDATEN

↓

BILDFUSIONIERUNG

↓

SEGMENTIERUNG

1. Geometrisch-anatomische Regionen (Aussen/"Kopf"/Tumor)
2. Quellvolumina (Nanopartikel)
3. PTV und Out-of-PTV (thermische Grenzbedingung < 43°C)

↓

TEMPERATURSIMULATION

1. Simulator „T-Vorgabe" („sim_t.exe")
2. Simulator „H-Vorgabe" („sim_h.exe")
3. Simulator „H-Regler" („sim_hr.exe")

↓

THERAPIEPLAN

## Fig. 1

**USER**

**SEGMENTIERUNG**

1. *Geometrisch-anatomische Regionen (Aussen/"Kopf"/Tumor)*
2. *Quellvolumina (Nanopartikel)*
3. *PTV und Non-PTV (thermische Grenzbedingung < 43°C)*

T < 43°C Non-PTV
T < 80°C überall

Simulator „T-Vorgabe"
„sim_t.exe", MODUS=1

H=15 kA/m

**NEIN**

**H-FELDSTÄRKE:**
**H<15 kA/m?**

Simulator „H-Vorgabe"
„sim_h.exe", MODUS=2

**JA**

H=5,6,7,8,9,10,11,12,13,14 kA/m

**EMPFEHLUNG:**
H-Feldstärke+
3D-Temperaturverteilung

Simulator „H-Regler"
„sim_hr.exe", MODUS=3

10 Temperaturverteilungen

-H

**NEIN**

**NEIN**

**NEIN**

**USER:**

**FELDSTÄRKE UND**
**TEMPERATUR OK?**

H=USER-VORGABE (Beliebiger Wert)

Simulator „H-Vorgabe"
„sim_h.exe", MODUS=2

**JA**

**THERAPIEPLAN**

## Fig. 2

**Fig. 3**

EINLESEN:
CT, LV

VORGABE:
Grenztemperaturen
T_grenz(x,y,z)

Nanopartikel-Volumen,
Eisenkonzentration, Eisenmasse

Relative SAR-Verteilung $\Delta$SAR(x,y,z); $\Delta$SAR_aver

Absolute Basal-Temperaturverteilung T0(x,y,z)

Relative T-Inkrement-
Verteilung $\Delta$T(x,y,z)

Skalierungsfaktor K:
K=Min {T_grenz(x,y,z)-T0(x,y,z)} / $\Delta$T(x,y,z)

Absolute Temperaturverteilung
T(x,y,z)= T0(x,y,z)+K $\Delta$T(x,y,z)
→ AUSGABE

Absolute SAR-Verteilung
SAR_aver=K $\Delta$SAR_aver

SAR_Fe

H-Feldstärke
→ AUSGABE

# Fig. 4

**Fig. 5**

SEGMENTIERUNG

*1. Geometrisch-anatomische Regionen (Aussen/"Kopf"/Tumor)*
*2. Quellvolumina (Nanopartikel)*
*3. PTV und Non-PTV (thermische Grenzbedingung < 43°C)*

USER

T < 43°C Non-PTV
T < 80°C überall

Simulator „T-Vorgabe"
MODUS=1
(„automatischer" Lauf)

H=15 kA/m

NEIN

H-FELDSTÄRKE:
H<15 kA/m?

JA

Simulator „H-Vorgabe"
MODUS=2
(„automatischer" Lauf)

EMPFEHLUNG:
H-Feldstärke+
3D-Temperaturverteilung

Simulator „Schneller-H-Regler"
MODUS=3
(„manueller" Lauf)

USER → H

NEIN

NEIN

USER:
FELDSTÄRKE UND
TEMPERATUR OK?

JA

THERAPIEPLAN

Fig. 6

56

INPUT:
CT, LV

Planungsprogramm-Core &
Simulator-Hauptprogramm

INPUT:
H-Feldstärke

Patientenmodell:
Regionen, PTV, NP-Volumen

HU-Mittelwert im NP-Volumen
HU_aver

SAR_Fe

Mittlere Eisenmasse im NP-Volumen
m_fe

Mittelwert absolut
SAR_aver

Absolute SAR Verteilung
SAR(x,y,z)

Absolute Temperaturverteilung
T(x,y,z)

OUTPUT: T(x,y,z)

Planungsprogramm-Core &
Simulator-Hauptprogramm

**Fig. 7**

**Fig. 8**

**Fig. 9**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20080268061 A
- US 20110052609 A
- WO 2009100716 A
- WO 2011082796 A
- WO 0113949 A

**In der Beschreibung aufgeführte Nicht-Patentliteratur**

- **GNEVECKOW et al.** Description and characterization of the novel hyperthermia- and thermoablation-system MFH® 300F for clinical magnetic fluid hyperthermia. *Med. Phys.,* Juni 2004, vol. 31 (6), 1444 ff
- Specific Absorption Rate of Iron. *Watt pro Gramm*
- **WUST et al.** Magnetic nanoparticles for interstitial thermotherapy - feasibility, tolerance and achieved temperatures. *Int. J. Hyperthermia,* Dezember 2006, vol. 22 (8), 673-685
- **MAIER-HAUFF et al.** Efficacy and safety of intratumoral thermotherapy using magnetic iron-oxide nanoparticles combined with external beam radiotherapy on patients with recurrent glioblastoma multiforme. *J. Neurooncol.,* 16. September 2010
- **NADOBNY et al.** Evaluation of MR-Induced Hot Spots for Different Temporal SAR Modes Using a Time-Dependent Finite Difference Method With Explicit Temperature Gradient Treatment. *IEEE Transactions on Biomedical Engineering,* Oktober 2007, vol. 54 (10), 1837 ff
- **HILDEBRANDT, B. et al.** The cellular and molecular basis of hyperthermia. *Critical Reviews in Oncology Hematology,* 2002, vol. 43 (1), 33-56
- **JORDAN, A. et al.** The effect of thermotherapy using magnetic nanoparticles on rat malignant glioma. *J Neurooncol.,* 29. November 2005, vol. 78 (1), 7-14
- **JOHANNSEN, M. et al.** *Eur Urol.,* 17. November 2006, vol. 52 (6), 1653-61
- **JOHANNSEN, M. et al.** *Int J Hyperthermia,* 2010, vol. 26 (8), 790-5
- **WUST, P. et al.** *Int J Hyperthermia,* 2006, vol. 22 (8), 673-85
- **THIESEN, B. ; A. JORDAN.** *Int J Hyperthermia,* 2008, vol. 24 (6), 467-74
- **NADOBNY et al.** *iterive way,* 2007, 1841
- **NADOBNY et al.** *decomposition way,* 2007, 1841